# EUROPÄISCHE PATENTANMELDUNG

(11) **EP 4 365 297 A1**
(43) Veröffentlichungstag der Anmeldung: **08.05.2024**
(21) Anmeldenummer: 22205810.9
(22) Anmeldetag: 07.11.2022
(51) Int. Cl.: C12N 15/82, A01N 63/60

(54) **NUKLEINSÄUREWIRKSTOFFE GEGEN VERSCHIEDENE PFLANZENPATHOGENE**

(71) Anmelder: Martin-Luther-Universität Halle-Wittenberg, 06108 Halle (Saale) (DE)
(72) Erfinder: Ghasemzadeh, Aysan, Ardebil, 561577663 (IR); Knoblich, Marie, 06110 Halle (Saale) (DE); Behrens, Sven-Erik, 06120 Halle (Saale) (DE); Gago-Zachert, Selma Persida, 06108 Halle (Saale) (DE); Gursinsky, Torsten, 06110 Halle (Saale) (DE)
(74) Vertreter: Maikowski & Ninnemann Patentanwälte Partnerschaft mbB

(57) **Zusammenfassung**

Die Erfindung betrifft neu identifizierte Nukleinsäuren, Ribonukleinsäuren (RNAs) und Deoxyribonukleinsäuren (DNAs), speziell *e*siRNAs/*E*RNAs (effektiv wirksame *small interfering* RNAs) und davon abgeleitete RNAs, sowie eASO (effektiv wirksame *antisense* DNA *oligonucleotides*), in der Summe als eNAs (effective Nucleic *A*cids) bezeichnet, die in RNA *silencing*/RNAi- bzw. *Antisense*-Verfahren als Wirkstoffe gegen verschiedene variable Pflanzenpathogene eingesetzt werden können.

Die Erfindung betrifft ferner die Konstruktion von doppelsträngigen Ribonukleinsäuren, edsRNAs (effektiv wirksame doppelsträngige RNAs), die Nukleotidsequenzen identifizierter *e*siRNAs/*E*RNAs oder davon abgeleiteter verwandter RNAs enthalten und die im RNA *silencing*/RNAi-Verfahren als Wirkstoffe gegen ebendiese variablen Pflanzenpathogene eingesetzt werden können.

## Beschreibung

### Gebiet der Erfindung

Die Erfindung betrifft neu identifizierte Nukleinsäuren, Ribonukleinsäuren (RNAs) und Deoxyribonukleinsäuren (DNAs), speziell *e*siRNAs/*E*RNAs (*e*ffektiv wirksame *small interfering* RNAs) und davon abgeleitete RNAs, sowie *e*ASO (*e*ffektiv wirksame *antisense* DNA *oligonucleotides*)*,* in der Summe als *e*NAs (*e*ffective *N*ucleic *A*cids) bezeichnet, die in RNA *silencing*/RNAi- bzw. *Antisense-*Verfahren als Wirkstoffe gegen verschiedene variable Pflanzenpathogene^{∗} eingesetzt werden können.

Die Erfindung betrifft ferner die Konstruktion von doppelsträngigen Ribonukleinsäuren, edsRNAs (*e*ffektiv wirksame doppelsträngige RNAs), die Nukleotidsequenzen identifizierter *e*siRNAs/*E*RNAs oder davon abgeleiteter verwandter RNAs enthalten und die im RNA *silencing*/RNAi-Verfahren als Wirkstoffe gegen ebendiese variablen Pflanzenpathogene^{∗} eingesetzt werden können.

^{∗}Unter dem Begriff "Pflanzenpathogene" werden hier zusammenfassend pflanzeninfizierende Viren ebenso wie pflanzenbefallende Organismen wie Nematoden und Pilze verstanden, die eine schädigende Wirkung auf Pflanzen ausüben.

### Hintergrund der Erfindung

RNA *silencing* oder RNA *interference* (RNAi) ist ein Mechanismus, der am besten in höheren Eukaryonten charakterisiert, jedoch in Zellen fast aller Organismen aktiv ist. Der RNAi-Mechanismus dient dem Abschalten (*silencing*) oder der Modulation der Genexpression und kann, wie nachfolgend noch erläutert wird, in diesen Funktionen auch gezielt eingesetzt werden (siehe u.a. Shabalina und Koonin 2008; Carthew und Sontheimer 2009). Zelluläre Faktoren, die im weiteren Text im Detail beschrieben werden, inaktivieren dabei über kleine Ribonukleinsäuremoleküle *small* RNAs, im folgenden "*s*RNAs" genannt, Ziel-Ribonukleinsäuremoleküle (im Folgenden "*target*-RNAs" genannt) oder modulieren die Funktion, wie z.B. die Translation, dieser *target*-RNAs. Unter den Begriff sRNAs fallen z.B. *small interfering* RNAs, siRNAs, sowie z.B. *micro* RNAs, miRNAs. Die *target*-RNAs stammen dabei aus Pathogenen, die Fremdzellen infizieren bzw. direkt aus einer Zelle, die Teil eines Organismus, auch eines als Pathogen agierenden Organismus ist, in dem RNAi wirksam ist.

RNAi ist ursprünglich wahrscheinlich ein evolutionär konservierter Verteidigungsmechanismus (*defense mechanism*) der Zelle. So ist RNAi bei Insekten, Nematoden, Oomyzeten, Pilzen und Pflanzen eine Hauptkomponente der Immunantwort gegen Pathogene (siehe u.a. Ding 2010; Zvereva und Pooggin 2012; Gammon und Mello 2015; Guo et al. 2019). Der RNAi-Prozess wird im Folgenden für die Pflanzenzelle und bezogen auf dessen Funktion als *defense mechanism* weiter beschrieben, da er hier mit am besten untersucht ist, insbesondere in Bezug auf dessen antivirale Wirksamkeit. RNAi ist, mit Modifikationen, in ähnlicher Form auch in Nematoden-, Oomyzeten-, Pilz- und Insektenzellen aktiv.

Ausgelöst wird RNAi durch Ribonukleinsäuren, die doppelsträngige (ds) Bereiche enthalten, d.h. zwei über komplementäre Basenpaarungen gepaarte (hybridisierte) Nukleotidstränge verschiedener RNA-Moleküle oder des gleichen RNA-Moleküls. Bei RNA-Molekülen, deren Nukleotidbausteine über weite Bereiche, unter Umständen hunderte oder tausende Nukleotide, Doppelstränge ausbilden können, spricht man auch von "dsRNAs". Gut untersucht ist die Induktion von RNAi bei viralen Infektionen: Auslöser können strukturierte, doppelsträngige Regionen viraler *messenger-RNAs* (mRNAs) oder viraler RNA-Genome sein. Besonders effektiv wird RNAi durch dsRNA-Replikationsintermediate ausgelöst. Diese entstehen bei der Replikation von RNA-Viren und bestehen aus komplementären RNA-Doppelsträngen, die hunderte oder tausende von Basenpaaren umfassen. Doppelsträngige Bereiche von RNAs können in der Zelle als sog. *pathogen-associated molecular pattern* (PAMP) und damit als fremd wahrgenommen werden. Detektoren sind dabei u.a. zelluläre Enzyme, die zur Familie der Typ III-Ribonukleasen gehören und als *Dicer* oder *Dicer-like proteins* (DCLs) bezeichnet werden (Fukudome und Fukuhara 2017; Song und Rossi 2017). Bei der antiviralen RNAi-lmmunantwort der Pflanze haben hierbei die erstmals in der Modellpflanze *Arabidopsis thaliana* (*A. thaliana*) charakterisierten Proteine DCL2 und DCL4 eine zentrale Rolle (Deleris et al. 2006; Parent et al. 2015). DCL2 und DCL4 binden dsRNAs und prozessieren diese durch endonukleolytische Hydrolyse (Spaltung) zu siRNAs. siRNAs sind kurze, 21-25 Nukleotide (nt) lange RNA-Moleküle, die aus zwei komplementären RNA-Einzelstrang-Komponenten bestehen. Am 5'-Ende sind diese sog. "RNA-Duplexe" phosphoryliert und am 3'-Ende weisen sie einen 2 nt langen, einzelsträngigen Überhang (*overhang*) und 3'-Hydroxylgruppen auf (Elbashir et al. 2001a; Elbashir et al. 2001b). Antiviral wirken insbesondere siRNAs, die Längen von 21 oder 22 nt haben (Deleris et al. 2006). Die siRNAs werden nach der Generierung durch die DCLs in sog. *RNA-induced silencing complexes* (RISC) aktiv. Dabei assoziieren sog. Argonaute (AGO)-Proteine, die ebenfalls endonukleolytische Aktivität haben können und Hauptkomponenten der RISC sind, einen Strang, den sog. *guide strand* (gs) des siRNA-Duplex, während der andere Strang des Duplex (*passenger strand,* ps) entfernt und abgebaut wird (Meister 2013; Kobayashi und Tomari 2016). In A. *thaliana* sind 10 verschiedene AGO-Proteine (AGO1-10) kodiert; sie haben unterschiedliche Funktionen, die z.T. noch nicht vollständig verstanden sind. Für AGO1 und AGO2 wurde fundiert gezeigt, dass diese Proteine zentral und essenziell in die antivirale Immunantwort der Pflanze involviert sind (Carbonell und Carrington 2015). Die AGO-Proteine haben dabei verschiedene Bindungsprioritäten für *siRNA-guide strands.* So bindet z.B. AGO1 mit hoher Priorität RNAs, die ein 5'-terminales U-Nukleotid enthalten, während AGO2 priorisiert RNAs mit 5'-terminalem A-Nukleotid binden (Mi et al. 2008; Takeda et al. 2008; Schuck et al. 2013). Nach Bindung des siRNA-*guide strands* durch AGO erfolgt die Assoziation der entsprechenden RISC an die *target-*RNA. Eine wichtige Rolle spielt dabei die Sequenzkomplementarität des *guide strand* mit der Sequenz in der *target-RNA,* an die dieser hybridisieren kann, hier als *target-site* bezeichnet (Liu et al. 2014). Die Sequenzkomplementarität ist naturgemäß am höchsten zu der *target-RNA,* aus der die siRNAs ursprünglich stammen. Demzufolge handelt es sich bei den *target-RNAs* unter natürlichen Umständen dann auch um diejenigen RNA-Moleküle ("kognaten RNAs"), die ursprünglich zu siRNAs prozessiert wurden. Bei viralen Infektionen sind dies entsprechend virale mRNAs, virale Genome oder virale Replikationsprodukte. Nach Assoziation des AGO/RISC an die zum *siRNA-guide strand* komplementären Regionen der kognaten *target-RNA,* dies können kodierende oder nicht kodierende (nicht translatierte) Regionen sein, kann eine durch das AGO-Protein katalysierte endonukleolytische Hydrolyse (Spaltung, *"slicing"*) der *target-RNA* erfolgen; diese ereignet sich zwischen den Nukleotiden der *target-RNA,* die gegenüber den Nukleotiden 10 und 11 des *siRNA-guide strands* liegen (Elbashir et al. 2001a; Elbashir et al. 2001c; Wang et al. 2008). Alternativ gibt es Hinweise darauf, dass an *target-*RNAs assoziierte siRNA-haltige AGO/RISC die Translation dieser RNAs inhibieren (Brodersen et al. 2008; Iwakawa und Tomari 2013). Ergebnis der Aktivität der siRNAs im RNAi-Prozess ist somit in jedem Fall eine temporäre Hemmung der Genexpression: Die *target-RNAs* werden gespalten und abgebaut oder die Synthese der durch diese RNAs kodierten Proteine wird gehemmt. Resultat des gegen Pathogene gerichteten RNAi ist somit, über die Hemmung der Genexpression, eine Inhibition der Vermehrung des Pathogens (Zvereva und Pooggin 2012). RNAi wird entsprechend bereits gezielt zum Schutz von Pflanzen gegen Pathogene wie Viren genutzt (Khalid et al. 2017; Pooggin 2017). DsRNAs oder sRNAs, die auf viralen Sequenzen beruhen, werden entsprechend als antivirale Wirkstoffe eingesetzt. Darüber hinaus können sRNA-Wirkstoffe, die das RNAi-Immunsystem von Pflanzen, Nematoden, Pilzen und Insekten gegen die eigenen RNAs wie z.B. mRNAs dieser Organismen aktivieren, entsprechend als Herbizide, Nematizide, Fungizide oder Insektizide eingesetzt werden. Diese sRNA-Wirkstoffe werden dann in der Regel gegen mRNAs dieser Organismen gerichtet (siehe u.a. Tomilov et al. 2008; Banerjee et al. 2017; Majumdar et al. 2017; Price und Gatehouse 2008). Bei eingesetzten sRNAs können dies siRNAs aber auch andere, verwandte RNAs wie z.B. miRNAs sein. Wie folgend noch detaillierter erläutert wird, agieren miRNAs nach dem gleichen Wirkprinzip wie siRNAs: Grundsätzlich wird in analoger Form eine Einzelstrang-Komponente einer miRNA in RISC eingebaut und im RNA *silencing*/RNAi-Prozess gegen eine *target-RNA* durch *slicing* oder durch Inhibition der Translation aktiv.

Wie bereits ausgeführt, wird RNAi für den künstlichen *"knock-down"* der Genexpression so praktiziert, dass siRNAs oder andere verwandte oder davon abgeleitete sRNAs wie z.B. miRNAs als Wirkstoffe in Zellen eingebracht werden, um *target-RNAs* gezielt in ihrer Funktion zu beeinflussen. So kann, wie geschildert, die zelluläre Genexpression, aber auch die Genexpression von Pathogenen wie Viren auf der Ebene von mRNAs unterdrückt oder moduliert werden. Darüber hinaus kann die Vermehrung (Replikation) von Viren, deren Genome RNA-Moleküle sind, über das *silencing* dieser Genome unterbunden werden.

Zum Zweck einer künstlichen Induktion von RNAi kann ein Organismus RNA-Wirkstoffe auch selbst exprimieren. Bei Pflanzen wird dies bereits praktiziert. In diesem Fall spricht man von *"host-induced gene silencing"*(*HIGS*): Pflanzen exprimieren dsRNAs, siRNAs oder andere sRNAs wie z.B. miRNAs in Form eines Transgens (Herrera-Estrella et al. 2005; Dong und Ronald 2019; Koch und Wassenegger 2021) um einen Schutz gegen Pathogene wie Viren, aber auch Nematoden, Pilze, Insekten, Oomyzeten oder andere, parasitäre Pflanzen zu erreichen. *"Transgenic applications"* sind jedoch aufwendig, für viele Anwendungen ungeeignet (Jan et al. 2000; Savenkov und Valkonen 2001; Simón-Mateo und García 2006; Tabashnik et al. 2013; Kung et al. 2015) und in vielen Ländern verboten, da gentechnisch veränderte Organismen (GVO) eingesetzt werden (Lucht 2015).

Alternativ können RNA-Wirkstoffe in sog. oberflächlich/transienten Anwendungen (*topical*/*transient applications*) eingesetzt werden. Dabei wird gelöste RNA, z.B. im einfachsten Fall in Form eines Sprays, auf ein Zielorgan oder Zielzellen eines Organismus aufgebracht. Dort wird die RNA über bisher noch unvollständig geklärte Mechanismen teilweise aufgenommen. Die Verbesserung der Aufnahmemechanismen der negativ geladenen RNA-Moleküle in Zielzellen, die ineffizient sind, ist entsprechend Thema intensiver Forschung. Das sog. *"spray-induced silencing"* (SIGS), bei dem RNA in Form einer Lösung (z.B. als Gießwasser oder Spray) auf und in Pflanzen eingesetzt wird, ist, auch aufgrund der biologischen Abbaubarkeit von RNAs, ökologisch wesentlich unkritischer als HIGS und für die Verabreichung von RNA-Wirkstoffen entsprechend attraktiv (Dalakouras et al. 2020). Zudem wurden in den letzten etwa 5 Jahren Verfahren entwickelt, die eine Herstellung von dsRNA im Gramm- oder sogar Kilogramm-Maßstab erlauben (Robinson et al. 2014; Kaur et al. 2018). So wurde der Preis von einem Gramm dsRNA, das etwa für den behandelnden Einsatz auf einem kleinen Feld von Kulturpflanzen benötigt wird, von über 100.000 US$ auf nunmehr unter 2 US$ gesenkt (Le Page 2017).

Wie schon oben angesprochen, ist im Falle des Pflanzenschutzes das Ziel von *topical*/*transient applications* von RNAs entweder, dass Pflanzen die RNA-Wirkstoffe internalisieren, um z.B. virale Replikation zu inhibieren, oder aber, dass attackierende Pathogene wie Pilze, Nematoden, Insekten oder parasitäre Pflanzen RNA-Wirkstoffe über die Oberfläche der zu schützenden Pflanze aufnehmen und diese dann im RNAi-System der jeweiligen Organismen gegen essenzielle *target-RNAs* der Pathogene aktiv werden (siehe auch oben: Tomilov et al. 2008; Banerjee et al. 2017; Majumdar et al. 2017; Price und Gatehouse 2008).

Der Einsatz von siRNA-Wirkstoffen in *topical*/*transient applications* ist jedoch aus verschiedenen Gründen bisher nicht ausgereift. Ein Grund ist die bereits erwähnte ineffiziente Aufnahme von RNA in Zielzellen. Deshalb wird intensiv an Verfahren gearbeitet, RNA-Wirkstoffe durch chemische Modifikationen der Nukleotidbausteine zu stabilisieren und/oder durch physikalische bzw. biochemische Verfahren an den Wirkungsort im Zytoplasma der Zielzellen zu bringen (Dowdy 2017; Setten et al. 2019; Dalakouras et al. 2020). Hier gibt es jedoch von Anwendung zu Anwendung und von Organismus zu Organismus große Unterschiede. Ein weiterer Grund betrifft die Tatsache, dass sowohl natürliche, als auch künstlich induzierte RNA *silencing*-Prozesse meist ineffizient sind. Dies liegt insbesondere an der mittlerweile durch viele Daten gut fundierten Tatsache, dass durch die Dicer/DCI-Aktivitäten aus *target-RNAs* zwar eine enorme Vielzahl (ein *"pool*") von siRNAs entsteht, dass aus einem solchen *pool* final aber nur einige wenige siRNAs tatsächlich auf der *target-RNA* wirksam sind. Ursächlich hierfür ist insbesondere, dass die *target-RNAs* in der Regel hoch strukturiert sind und nur wenige Angriffsbereiche (hier ***accessible sites,*** "**a-Sites"** genannt) für eine effektive Assoziation von *s*RNA/AGO/RISC an die komplementären *target-sites* und damit für ein *silencing* zugänglich sind. Dies trifft verstärkt auf *target-RNAs* aus Pathogenen zu: Diese sind in ihrer Strukturierung durch Ko-Evolution mit dem Zielorganismus oft so angepasst (attenuiert), dass sie dem RNA-*silencing* bestmöglich entgehen.

a-Sites sind hier entsprechend als Bereiche einer *target-RNA* definiert, die zugänglich für Nukleinsäurewirkstoffe sind. Eine a-Site kann einer "*target-site*" entsprechen. Eine *target-site* ist hier als die Sequenz einer *target-RNA* definiert, an die eine einzelsträngige Nukleinsäure wie ein siRNA *guide-strand* oder auch ein *antisense* Oligonukleotid (siehe unten) über komplementäre Basenpaarung hybridisieren kann. Eine a-Site kann aber auch größere Bereiche einer *target-RNA* umfassen, also z.B. RNA-Strukturmotive, die besonders gut zugänglich sind und eine oder mehrere *target-sites* von einzelsträngigen Nukleinsäuren enthalten.

Bei SIGS werden bisher vor allem doppelsträngige Versionen von *target*-RNAs, also z.B. dsRNA-Versionen von mRNAs oder viraler Genome, für das RNAi eingesetzt (Robinson et al. 2014). Diese dsRNAs bestehen z.B. aus den *target-RNAs* (die meist mehrere hundert Nukleotide umfassen) und einem zweiten RNA-Strang mit komplett komplementärer Sequenz. Alternativ befinden sich beide komplementäre Bereiche auf einem RNA-Molekül und bilden einen sog. *"hairpin".* In einem *hairpin* werden zwei RNA-Stränge komplementärer Sequenz durch eine weitgehend unstrukturierte, einzelsträngige Sequenz beliebiger Nukleotidkomposition und Länge, einem *"spacer",* verbunden. Die Mindestlänge eines solchen *spacers* sind dabei vier Nukleotide, die Maximallänge ist unbegrenzt. Nach Eintritt in die Pflanzenzelle werden dsRNA bzw. dsRNA-hairpins entsprechend analog zu den normalen *target-RNAs* durch die DCLs in einen siRNA-*pool* prozessiert. Dieser unterliegt jedoch wiederum dem oben genannten Problem: Nur sehr wenige der aus diesen "konventionellen dsRNAs" generierten siRNAs sind wirksam. Die übrigen siRNAs des *pools,* d.h. die überwältigende Mehrzahl, kann dagegen zelluläre AGO/RISC unspezifisch absättigen (sie wirken damit als "Lockvogel" oder *"decoy"* für die AGO/RISC), was dazu führen kann, dass das RNA *silencing* durch die Verwendung konventioneller dsRNAs oder *dsRNA-hairpins* sogar inhibiert wird. Zudem besteht die Gefahr, dass siRNAs eines *pools* ein *silencing* auf nicht anvisierten RNAs ("*off-target effects*") auslösen, z.B. über nicht komplett komplementäre Basenpaarung von *guide strands,* die aber dennoch ausreicht, um funktionelle RISC zu bilden (Jackson und Linsley 2004; Jackson et al. 2006; Senthil-Kumar und Mysore 2011; Casacuberta et al. 2015; Kamola et al. 2015). In der Summe ist somit die Verwendung von dsRNAs, bei deren Einsatz besonders viele, nicht näher charakterisierte und nicht auf der *target-RNA* aktive siRNAs generiert werden, problematisch (Qu et al. 2012; Dalakouras et al. 2016). Das gleiche gilt für dsRNAs, aus denen andere sRNAs generiert werden.

Bis vor kurzem konnten die wenigen, effektiv im RNAi wirksamen siRNAs, hier **"*e*siRNAs"** oder **"*E*RNAs"** und im Folgenden kombiniert **"*e*siRNAs/*E*RNAs"** genannt, aus siRNA-*pools* nicht zuverlässig identifiziert und eingesetzt werden. Viele siRNA- bzw. sRNA-Wirkstoffe wurden entsprechend so "designt", dass sie entweder gegen Bereiche einer *target*-mRNA gerichtet wurden, die für konservierte Bereiche eines Proteins kodieren, oder sie wurden anhand von unzuverlässigen *in silico*-Vorhersagen von a-Sites in den *target-RNAs* ermittelt und dann bezüglich ihrer Wirkung in sehr aufwändigen empirischen Untersuchungen ("*trial and error"*) getestet (Birmingham et al. 2007; Cerritelli und Crouch 2009; Miozzi et al. 2013; Fakhr et al. 2016; Carbonell et al. 2018; Eastman et al. 2018; Han et al. 2018; Qureshi et al. 2018; Setten et al. 2019).

Erst in den letzten Jahren ist es vor allem durch Arbeiten aus unserem Labor gelungen, experimentelle Methoden zu etablieren, mit denen es in einem kurzen Zeitrahmen möglich ist, in unterschiedlichsten *target-RNAs* a-Sites zuverlässig zu detektieren (Schuck et al. 2013; Gago-Zachert et al. 2019; WO2019001602 A1; PCT/EP2022/057596). Über die Kenntnis der a-Sites können entsprechend auch *e*siRNAs/*E*RNAs identifiziert werden, die zuverlässig und effizient im RNA *silencing*-Prozess gegen die jeweiligen *target-RNAs* wirksam sind.

Mit der Möglichkeit, ausschließlich *e*siRNAs/*E*RNAs gegen *target-RNAs* einsetzen zu können, wird die Effizienz von *RNA silencing*-Prozessen signifikant erhöht. Zudem erhöht die Verwendung von *e*siRNAs/*E*RNAs die Spezifität von RNAi: Die Wahrscheinlichkeit von *off-target-effects* auf nicht adressierten RNA-Molekülen und damit ungünstiger und unerwünschter Nebenwirkungen von RNA-Wirkstoffen wird deutlich verringert.

Wie bereits ausgeführt, können mit der Identifizierung von a-sites in *target-*RNAs *e*siRNAs/*E*RNAs definiert werden. Mit anderen Worten, in den a-sites sind Sequenzen, *target-sites,* zugänglich, über die *e*siRNA/*E*RNA *guide strands* an die *target-RNA* binden (über komplementäre Basenpaarung hybridisieren) können, und durch die Aktivität des RISC wird die *target-RNA* dann in der beschriebenen Weise (d.h. über endonukleolytische Spaltung oder Inhibition der Translation inaktiviert (s.o.)). Ein analoger Effekt kann auch durch verwandte sRNAs, wie z.B. miRNAs, erreicht werden, deren Sequenzen aus den Sequenzen von *e*siRNAs/*E*RNAs abgeleitet werden können und die dann in einem analogen RNA *silencing*/RNAi-Prozess wie *e*siRNAs/*E*RNAs eingesetzt werden können.

**Erst kürzlich wurde demonstriert, dass die Identifizierung von a-Sites und die Definition von *e*siRNAs/ERNAs, deren *guide strands* an *target-sites* in diesen a-Sites binden können, simultan auch die Definition von *antisense*-Deoxyribonukleinsäure (DNA)-Oligonukleotiden (ASO) erlaubt, die ebenfalls an diese *target-sites* binden können (**PCT/EP2022/057596**).** In analoger Terminologie werden hier von *e*siRNAs/*E*RNAs abgeleitete ASO als ***e*ASO** bezeichnet. ***e*ASO haben zu Einzelsträngen von *e*siRNAs/ERNAs homologe DNA-Sequenzen (d.h. Deoxynukleotide statt Ribonukleotide; Thymidin statt Uridin) und können entsprechend auch an die jeweiligen *target-sites* in den a-Sites der *target*-RNAs hybridisieren und aktiv werden.** ASO können ebenso wie sRNAs transient eingesetzt werden und wirken in sog. "*Antisense*-Verfahren" über unterschiedliche Mechanismen. Im gegebenen Kontext sind zwei dieser Mechanismen wichtig: Zum einen kann die Bildung eines DNA:RNA Heteroduplex zwischen ASO und *target-RNA* den Translationsprozess der RNA im Zytoplasma hemmen. Zum anderen können durch die Bildung von DNA:RNA Heteroduplexen RNase H Endonukleasen (in eukaryontischen Zellen RNAase H1 und/oder RNAase H2) im Zellkern oder im Zytoplasma aktiviert werden, die dann, ähnlich wie AGO/RISC, geleitet durch die Bindung des ASO an die komplementäre RNA, den Abbau dieser *target-RNA* katalysieren (Shen und Corey 2018; Bennett 2019; PCT/EP2022/057596; Wdowikowska und Janicka, 2021, Crooke et al., 2021). **Optimal aktive ASO haben eine Länge von 12-20 Nukleotiden (Crooke et al., 2021). Entsprechend können *e*ASO gut von 21, 22, 23 oder 24 nt langen *e*siRNAs/*E*RNAs abgeleitet werden.**

Der nachfolgende Text beschränkt sich im Wesentlichen auf die Beschreibung der Aktivität von *e*siRNAs/*E*RNAs. **Analoge Feststellungen gelten auch für andere, verwandte sRNAs wie z.B. miRNAs** sowie *e*ASO, deren RNA bzw. DNA-Sequenzen aus den Sequenzen identifizierter esiRNAs/ERNAs abgeleitet werden können: Aufgrund der übereinstimmenden Sequenz können die Einzelstränge solcher sRNAs bzw. die *e*ASO an die gleichen *target-sites* in den entsprechenden *target*-RNAs binden und somit ebenfalls in RNA *silencing*/*RNAi* bzw. *Antisense*-Verfahren zur Pathogenbekämpfung eingesetzt werden (PCT/EP2022/057596). In der Summe werden hier identifizierte esiRNAs/ERNAs und die davon abgeleiteten verwandten sRNAs und *e*ASO im Folgenden als eNAs (*e*ffective *Nu*cleic Acids) bezeichnet.

Es soll hier auch angemerkt werden, dass RNA- wie auch DNA-Wirkstoffe in chemisch modifizierter Form eingesetzt werden können: Chemische Modifikationen, Konjugate oder der Aufbau, u.a. als sog. *"gapmers"* oder *"mixmers",* können das Potenzial siRNA- oder ASO-basierter Wirkstoffe in RNAi- bzw. *Antisense*-Verfahren *in vivo* erheblich verbessern (Setten et al. 2019; Wdowikowska und Janicka, 2022; Crooke et al., 2021; siehe auch claims).

Die Kenntnis einer großen Zahl von a-Sites (und damit *target-sites*) einer *target-RNA* ist besonders wichtig bei Verfahren, die das Ziel haben, Pathogene zu bekämpfen, die variabel sind. Speziell Viren mit einem RNA-Genom und einer RNA-abhängigen RNA-Polymerase (*RNA-dependent RNA polymerase,* RdRp) als Hauptenzym der viralen Replikation weisen eine hohe Plastizität (Variabilität) auf und damit die Möglichkeit schneller Resistenzbildung gegen antivirale Wirkstoffe. Virale RdRps haben keine oder nur eine ineffziente *editing*-Funktion, die Fehler bei der Inkorporation von Nukleotiden in neu synthetisierten Tochter-Nukleinsäurestränge korrigieren kann. Entsprechend hoch ist die Mutations- und Evolutionsrate von RNA-Viren während der Replikation: man spricht hier von *"antigenic drift".* Besonders hoch ist die Mutations- und Evolutionsrate von Viren, deren Genom auf mehrere Segmente aufgeteilt vorliegt. Bei Ko-Infektion einer Zelle durch verschiedene Viren können sich diese Segmente zu völlig neuen Kombinationen sortieren - man spricht hier von "reassortieren". Dies führt zu *"antigenic shifts",* erheblichen genetischen Veränderungen, die zur Generierung von Viren mit völlig neuen Eigenschaften führen können und eine große Gefahr für den jeweiligen Wirt darstellen, weil gegen diese Viren unter Umständen keine wirksame Immunantwort vorliegt ("*viral escape*")*.*

Eine hohe Variabilität ist aber auch bei anderen Organismen zu finden, speziell solchen, die unter starkem Selektionsdruck stehen. Das ist bei Pflanzenpathogenen der Fall, die routinemäßig mit antipathogen wirksamen Substanzen behandelt werden. Diese mutieren "unter diesen Substanzen" und bilden restistente Formen aus. Dies trifft z.B. für Nematoden und Pilze zu, die in Landwirtschaft und Gartenbau konventionell mit Nematiziden bzw. Fungiziden behandelt werden.

Durch die Anwendung eines breiten Spektrums von eNAs in HIGS oder SIGS sollte sich jedoch die Möglichkeit der Entwicklung von Pathogenformen, die einem antipathogen wirkenden Verfahren gegenüber resistent werden (*escape*), in den verschiedenen Organismen drastisch einschränken lassen. Breites Spektrum bedeutet, dass zwei oder mehr eNAs gegen eine *target-RNA* eingesetzt werden oder dass zwei oder mehr eNAs eingesetzt werden, die gegen verschiedene *target-RNAs* des Zielorganismus gerichtet sind. Mit den gleichen Ansätzen ließe sich auch eine Breitbandwirkung gegen Pathogenvarianten erzielen (die z.B. im Zuge einer Virusepidemie auftreten können). Schließlich kann durch den Einsatz verschiedener eNAs gegen verschiedene *target*-RNAs verschiedener Pathogene ein Breitbandschutz gegen unterschiedliche Pathogene erreicht werden.

Durch den Einsatz von effektiv wirksamen eNAs ergeben sich somit folgende Anwendungsvorteile:
(i) Durch die Kombinierbarkeit verschiedener *e*siRNAs/*E*RNAs kann die Spezifität und Effizienz von RNA *silencing*/RNAi-Verfahren maximal gesteigert werden. Das gleiche gilt für andere sRNAs, deren Sequenz von *e*siRNAs/*E*RNAs abgeleitet wird. Das gleiche gilt für *e*ASO, deren Sequenz von *e*siRNAs/*E*RNAs abgeleitet wird und die in *Antisense*-Verfahren eingesetzt werden.
(ii) Auf Pathogene, die durch *antigenic drifts* oder *antigenic shifts* starken Veränderungen unterliegen bzw. in komplett neuer Form entstehen können, kann eine RNAi-Immunantwort durch eine Neukombination von anti-pathogenen *e*siRNAs/*E*RNAs schnell und spezifisch angepasst werden. Das gleiche gilt für *e*siRNA/ERNA abgeleitete andere sRNAs. Das gleiche gilt für *e*siRNA/ERNA abgeleitete *e*ASO, die in *Antisense*-Verfahren eingesetzt werden.
(iii) Durch eine ökonomische Produktion von Nukleinsäure-basierten Wirkstoffen sollte sich die Akzeptanz des Einsatzes von RNAi oder *Antisense-*Verfahren, z.B. in der Pflanzenproduktion, deutlich erhöhen lassen. SIGS-Ansätze können effizienter durchgeführt werden und der Einsatz transgener Ansätze deutlich vermindert werden.
(iv) Um den gleichzeitigen Einsatz von *e*siRNAs/*E*RNAs optimal zu ermöglichen und dabei auch simultan die Einsatzfähigkeit von RNA in HIGS- und SIGS-Verfahren zu verbessern, sollten diese bevorzugt in Form von dsRNAs verwendet werden. Solche hier so genannten **"***e*dsRNAs" sollten die Nukleotidsequenzen mehrerer sRNAs (*e*siRNAs/*E*RNAs oder davon abgeleiteter anderer sRNAs) enthalten. Als generellen Vorteil haben dsRNAs gegenüber siRNAs, die an den 3'-Enden, wie beschrieben, einzelsträngige Sequenzüberhänge haben, eine deutlich verminderte Degradationsrate (erhöhte Halbwertszeit) in HIGS und SIGS-Verfahren (Bachman et al. 2020) und daher deutliche Anwendungsvorteile.

### Problem/Aufgabenstellung

Im Pflanzenschutz stellt sich bei der Bekämpfung stark veränderlicher Pathogene die Aufgabe, *target*-RNAs dieser Pathogene mit adaptierbaren Nukleinsäurewirkstoffen zu inaktivieren und so deren Vermehrung zu unterbinden. Die objektiv-technische Aufgabe der Erfindung besteht somit darin, Pflanzen gegen verschiedene, variable Pathogene mit nachhaltigen Nukleinsäure-basierten Methoden zu schützen.

Zur Lösung dieser Aufgabe stellt die Erfindung verschiedene Nukleinsäure-basierte Wirkstoffe, Verfahren zur Herstellung und Verfahren zu deren Anwendung bereit.

In einem Aspekt betrifft die Erfindung RNAi-Verfahren mit der Anwendung einer oder mehrerer *e*siRNAs/ERNAs bzw. verwandter *s*RNAs oder *Antisense*-Verfahren mit der Anwendung einer oder mehrerer *e*ASO, deren Sequenzen von esiRNAs/ERNAs abgeleitet wurden. Aktive *e*siRNAs/*E*RNAs bzw. davon abgeleitete andere sRNAs bzw. *e*ASO (in der Summe *e*NAs genannt) sollen eine verlässlich hohe, effektive antipathogene Wirksamkeit haben und in eingesetzten Kombinationen gegen verschiedene *target*-RNAs oder verschiedene Bereiche (*target-sites*) von *target*-RNAs gerichtet sein, um auch stark veränderliche (variable) Pathogene verlässlich bekämpfen zu können.

In einem weiteren Aspekt betrifft die Erfindung RNAi-Verfahren mit edsRNAs, die die Nukleotidsequenzen von *e*siRNAs/ERNAs oder davon abgeleiteten sRNAs enthalten und die im Verlauf des RNA silencing-Prozesses in diese *e*siRNAs/*E*RNAs bzw. sRNAs prozessiert werden.

Die Nukleinsäure-basierten Wirkstoffe der Erfindung können gegen Stellvertreter verschiedener Klassen variabler bzw. hochvariabler Pathogene generiert bzw. angewendet werden, die den Wirtsorganismus Pflanze infizieren. Wie ausgeführt, gehören zu den wichtigsten variablen Pflanzenpathogenen Viren, Nematoden und Pilze. Die Erfindung ist besonders vorteilhaft, da die erfindungsgemäßen Nukleinsäure-basierten Wirkstoffe gegen ökonomisch besonders wichtige Vertreter dieser Pathogene generiert bzw. angewendet werden können. Ein Vertreter eines ökonomisch bedeutsamen, hochvariablen Viruspathogens in Pflanze ist das Cucumber mosaic virus (CMV). Ein Vertreter eines ökonomisch bedeutsamen, variablen Nematodenpathogens in Pflanze ist *Meloidogyne incognita* (*M. incognita*). Ein Vertreter eines ökonomisch bedeutsamen, variablen Pilzpathogens in Pflanze ist *Botrytis cinerea* (*B. cinerea*)*.*

Durch die Lösung der Aufgabe der Erfindung wird eine breite Palette wirksamer eNAs bereitgestellt, die als Wirkstoffe im Pflanzenschutz individuell oder in Kombination gegen diese wichtigen Pathogene eingesetzt werden können.

### Verwendetes Verfahren

Zur Identifizierung von eNAs wurde ein publiziertes bzw. zum Patent angemeldetes Verfahren angewendet (PCT/DE2018/000194; PCT/EP2022/057596):
Es erfolgt in drei Schritten.
(i) Eine *target*-RNA, dies kann eine genomische RNA, eine mRNA oder eine dsRNA sein, wird einem zytoplasmatischen Extrakt aus Pflanzenzellen (*Nicotiana tabacum* BY-2 Zellen), dem sog. "BYL", ausgesetzt (L steht dabei für "Lysat"). Die im Extrakt präsenten (endogenen) DCLs erzeugen aus der *target*-RNA einen siRNA-*pool*, der als Hauptprodukte u.a. 21, 22, 23 und 24 nt siRNAs enthält. Der siRNA-*pool* wird in seiner Gesamtheit durch RNA-Sequenzierung der nächsten Generation (NGS, *RNA-Seq*) erfasst.
(ii) In den Extrakten werden RISC mit dem in (i) entstandenen siRNA-Pool und mit einem AGO-Protein nach Wahl rekonstituiert. Das AGO-Protein wird dazu in dem Extrakt von einer zugesetzten mRNA *in vitro* translatiert. Die gebildeten AGO/RISC werden immunpräzipitiert, und die durch Bindung an AGO angereicherten siRNA-Stränge werden wiederum durch *RNA-Seq* identifiziert. Die *RNA-Seq*-Daten aus Schritt (i) werden dabei zum Abgleich herangezogen, um eine Anreicherung in dem betreffenden AGO/RISC zu definieren.
(iii) Aus den in (ii) erfassten siRNAs werden schließlich mit weiteren *in vitro* Assays, in denen mit den jeweils verwendeten AGO/RISC auf endonukleoytische Spaltung (*slicing*) getestet wird (im Folgenden und in den Figuren **"*Slicer-Assay"*** genannt), diejenigen zuvor in den betreffenden AGO/RISC angereicherten siRNAs identifiziert, die ein effizientes *slicing* der *target-RNA* induzieren. Diese werden so in einem ersten Ansatz als *e*siRNAs/*E*RNAs validiert. Weitere Validierungsschritte testen dann die Wirksamkeit der jeweiligen *e*siRNAs/*E*RNAs *in vivo.* Hier kommen an die jeweiligen adressierten *target-*Pathogene angepasste, unterschiedliche Methoden zum Einsatz. Von derart identifizierten *e*siRNAs/*E*RNAs werden von der Nukleotidsequenzabfolge analog aufgebaute andere sRNAs und *e*ASO abgeleitet. Diese können ebenfalls in Slicer-Assays in BYL getestet werden. Bei *e*ASO sind die aktiven Komponenten dabei RNAase H Enzyme (PCT/EP2022/057596).

### Adressierte target-Organismen

### Das pflanzenpathogene Cucumber mosaic virus (CMV)

CMV, das Typ-bestimmende Virus der Gattung *Cucumovirus* (Familie *Bromoviridoe*), ist ein Pflanzenpathogen mit großer ökonomischer Bedeutung (Scholthof et al., 2011; Rybicki, 2015; Gallitelli, 2000). CMV hat ein tripartit-segmentiertes, einzelsträngiges (ss) positiv (+)-Strang RNA-Genom. Die drei genomischen RNAs 1 (3,3 kb), 2 (3,0 kb) und 3 (2,2 kb), die in drei verschiedenen Viruskapsiden verpackt werden und nur gemeinsam eine Infektion auslösen, weisen am 5'-Ende ein *cap* und am 3'-Ende eine tRNA-ähnliche Struktur auf. Aufgrund der Segmentierung des Genoms ist das CMV ein reassortierendes Virus mit der damit verbundenen hohen Mutationsrate: d.h. neben *antigenic drift,* bedingt durch die virale RdRP, zeigt das Virus *antigenic shifts.* Nach dem Eintritt in die Wirtszelle agieren die drei genomischen RNAs aufgrund ihrer (+)-Orientierung als mRNAs ((+) heißt *sense-*Orientierung wie eine mRNA). RNA 1 und RNA 2 kodieren jeweils für die Proteine "1a" (111 kDa) und "2a" (97 kDa). Das 2a-Protein ist die RdRp; zusammen mit 1a bildet sie die virale Komponente der Replikase, welche die Genomreplikation und die Transkription subgenomischer (sg) RNAs katalysiert. Während des (hier nicht detailliert beschriebenen) RNA-Replikationsprozesses werden durch die Replikase komplementäre (-)RNA-Kopien der viralen RNAs transkribiert. Diese dienen dann sowohl als *template* zur Synthese neuer (+)RNA-Moleküle, als auch als *template* für die Synthese von subgenomischen sgRNAs. Eine sgRNA, sgRNA 4 (1,1 kb), wird von der bei der Replikation entstehenden (-)RNA-Kopie der RNA 3 transkribiert und wird auch mit dieser zusammen in ein Kapsid verpackt. RNA 3 kodiert selbst für das 30 kDa *"movement protein* 3a"; die sgRNA 4 für das 24 kDa große "Kapsidprotein CP". 3a und CP sind während des Infektionsprozesses sowohl für die Zell-zu-Zell- als auch für die systemische Bewegung des Virus durch die Pflanze essenziell. Von der (-)RNA-Kopie der RNA 2 wird eine weitere subgenomische RNA, sgRNA 4A (0,7 kb), transkribiert. Diese kodiert für einen viralen Suppressor des RNA-*silencing* (VSR), "2b" (15 kDa). Das 2b interferiert mit dem RNA-*silencing-*Prozess, u.a. durch Sequestrierung (hochaffine Bindung) dabei generierter siRNAs. Alle fünf Genprodukte beeinflussen in einer wirtsspezifischen Weise die Ausbreitung des Virus in der Pflanze und somit auch die Virulenz. Bei Infektionen können im Zuge der viralen RNA-Replikation zusätzlich Satelliten-RNAs entstehen, deren Präsenz die Pathogenese maßgeblich beeinflussen kann (Gallitelli, 2000; Garcia-Arenal et al., 2008; Jaquemond, 2012; Roossinck et al., 2001; Roossinck et al., 2002, Palukaitis, 2016; Nouri et al., 2014; Mochizuki und Ohki 2012).

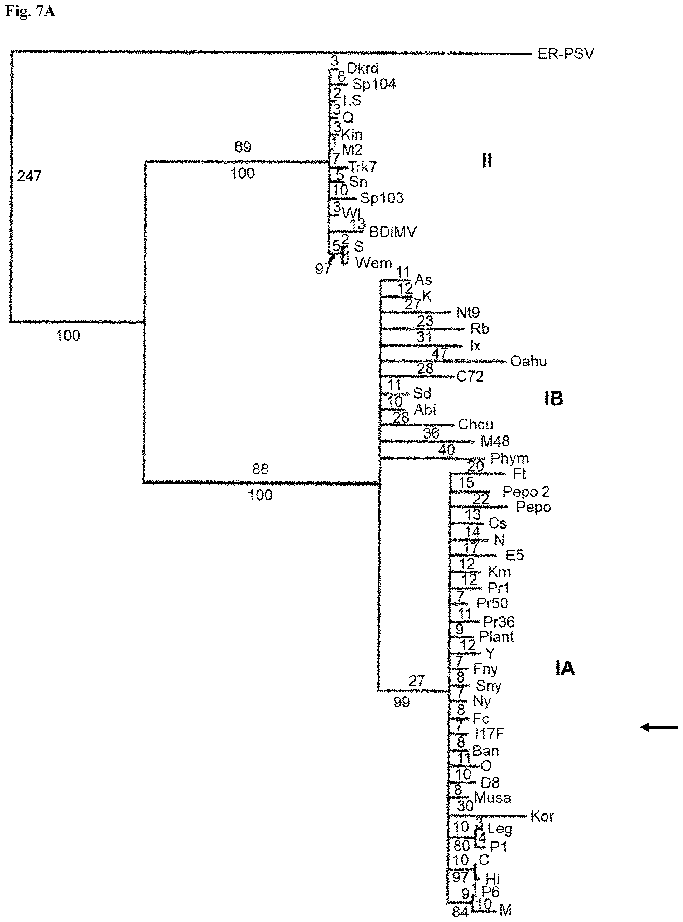

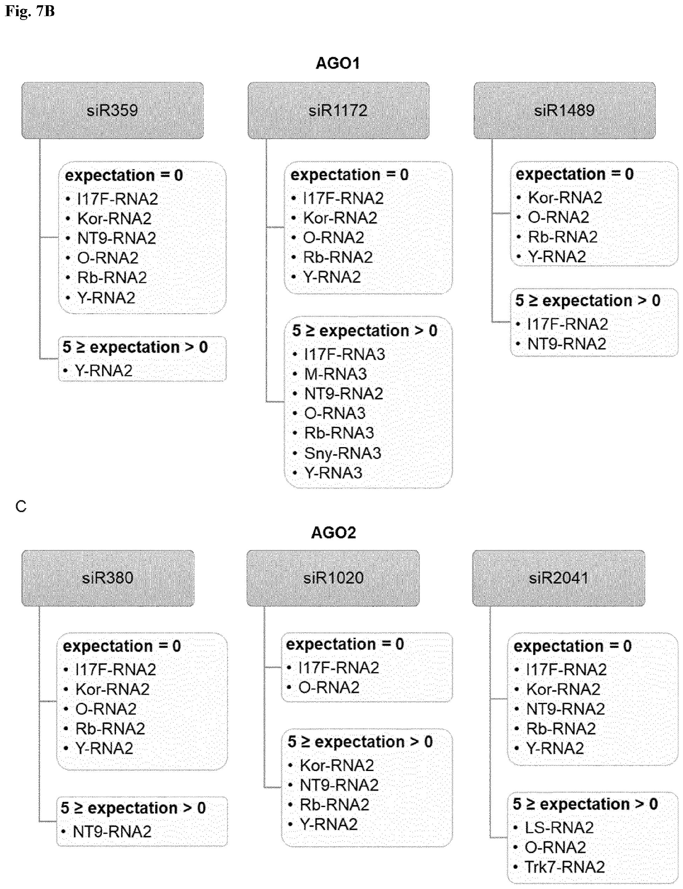

CMV-Stämme werden grob in zwei Untergruppen, 1(I) und 2 (II), unterschieden, wobei die Stämme der Untergruppe I nochmals in zwei (A und B) Untergruppen unterteilt werden (siehe auch unten und Figur 7). Während innerhalb einer Untergruppe die Sequenzähnlichkeiten groß sind (Untergruppe I: > 88 %; Untergruppe II: > 96 %), betragen sie zwischen den Untergruppen nur 70-75%. CMV-Stämme der Untergruppe IA und II sind weltweit verbreitet; die der Untergruppe IB kommen hauptsächlich in Ostasien vor (Garcia-Arenal et al., 2008; Jaquemond, 2012; Nouri et al., 2014; Mochizuki und Ohki 2012). Der serologische Differenzierungsindex (SDI) zwischen den CMV-Untergruppen liegt bei etwa 1 - 2. Diese unterscheiden sich mit je einem SDI von 6 - 7 von den anderen Cucumoviren PSV (*Peanut stunt virus*) und TAV (*Tomato aspermy virus*). Wie andere segmentierte RNA-Viren hat CMV eine hohe Variabilität. Diese wird bedingt durch die Akkumulation von Mutationen durch *antigenic drift* und *shift-*Prozesse (Scholthof et al., 2011; Rybicki, 2015; Gallitelli, 2000).

Im Gegensatz zu anderen Mitgliedern der Familie *Bromoviridoe* haben CMV-Stämme ein sehr breites, kollektives Wirtsspektrum und infizieren mehr als 1200 Pflanzenarten in über 100 Familien von Monoals auch Eu-/Dikotyledonen. Darunter sind wichtige Obst-, Gemüse- und Zierpflanzen wie *Fabaceae, Cucurbitaceae, Convolvulaceae* und *Solanaceae.* CMV ist damit das Pflanzenvirus, welches das größte Wirtsspektrum hat und wichtige Kulturpflanzen wie Bohne, Rübe, Karotte, Sellerie, Salat, Paprika, Melone, Kürbis, Tomate und Spinat gleichsam befällt (Scholthof et al., 2011; Garcia-Arenal et al., 2008; Jaquemond, 2012; Mochizuki und Ohki 2012). CMV-Infektionen verursachen u.a. schwere systemische Mosaiksymptome, Blattdeformation, systemische Nekrose, Chlorose, Zwergwuchs und Fruchtläsionen (Garcia-Arenal et al., 2008; Jaquemond, 2012; Holeva et al., 2021). Das CMV kann dabei in Nachtschattengewächsen synergistisch mit Potyviren, Tobamoviren und Potato virus X (PVX) interagieren sowie mit Potyviren in Cucurbit-Wirten (Scholthof et al., 2011; Gallitelli, 2000; Jaquemond, 2012).

CMV-Partikel werden von mehr als 80 Blattlausarten (Aphiden) in 33 Gattungen auf nicht persistente *"stylet-borne"*-Weise übertragen. Zudem können, je nach Pflanzenspezies, Übertragungen durch infizierte Samen erfolgen (Jaquemond, 2012; Ali und Kobayashi, 2010; O'Keefe et al., 2007). Weiterhin gibt es Indikationen, dass das Virus die Wintermonate in Saatgut überdauern kann, das dann eine bedeutende Quelle für Primärinfektionen zu Beginn der Vegetationsperiode ist.

Aufgrund des großen Wirtspflanzenkreises, der weltweiten Verbreitung und der nicht persistenten Übertragbarkeit durch zahlreiche Aphiden wird CMV zu den Pflanzenviren mit größter ökonomischer Bedeutung bzw. zu den wichtigsten Viren einjähriger Kulturen weltweit gezählt (Scholthof et al., 2011; Rybicki, 2015). Die Ernteverluste variieren von Jahr zu Jahr an verschiedenen Standorten und sind, insbesondere bei Mischinfektionen, schwer zu quantifizieren. Einen Einblick geben Schätzungen aus den 1990er und 2000er Jahren, die z.B. in China bei einer Tomatenernte von 25 %-50 %, oder in Spanien bei einer Melonenernte oder einer Paprikaernte von 60 % bzw. 80 % ausgehen. Bei Auftreten einer nekrogenen Satelliten-RNA mit bestimmten CMV-Stämmen wurden in Spanien und Italien in 70 % der Anbaugebiete Verluste von 80%-100 % der Tomatenpflanzen verzeichnet (Gallitelli, 2000).

Bemerkenswerterweise werden jedes Jahr weitere Wirte von CMV und neue CMV-induzierte Pflanzenkrankheiten beschrieben. So ist abzusehen, dass aufgrund des Klimawandels eine erhöhte Aphidenaktivität in den nördlichen gemäßigten Regionen zu weiteren Epidemien führen wird (Scholthof et al., 2011; Nicaise, 2014). CMV gewinnt aber auch in tropischen und subtropischen Regionen zunehmend an Bedeutung, insbesondere dort, wo Mischkulturen angebaut werden.

Bekämpfungsmaßnahmen, die allein auf Pestizideinsatz gegen Aphiden beruhen, sind nicht sehr wirksam (Gallitelli, 2000). Aber auch Ansätze, in denen transgene Pflanzen oder RNA-basierte Ansätze zur Pflanzenprotektion eingesetzt wurden, waren bisher, meist aufgrund der hohen Veränderlichkeit des Virus, nicht sehr erfolgreich (Nicaise, 2014).

### Zusammenfassung der Erfindung

Zur Lösung der Aufgabe konnten im Rahmen der Erfindung *e*siRNAs/ERNAs gegen verschiedene genomische CMV-RNAs charakterisiert werden, die zuverlässig eine hohe antivirale Wirksamkeit haben. Neben einer generell hohen antiviralen Wirksamkeit gegen die kognaten *target-RNAs* sollten diese *e*siRNAs/ERNAs bzw. Varianten dieser *e*siRNAs/ERNAs auch antiviral wirksam gegen verschiedene CMV Varianten sein. Darüber hinaus wurden für HIGS- und/oder SIGS-Verfahren bevorzugt eingesetzte dsRNAs, sog. "*e*dsRNAs" generiert, aus denen im Zuge des RNA *silencing-*Prozesses signifikante Quantitäten *e*siRNAs/*E*RNAs erzeugt werden und die dementsprechend ebenfalls verlässlich und effizient antiviral wirken. Von den so charakterisierten *e*siRNAs/*E*RNAs konnten auch andere sRNAs bzw. *e*ASO abgeleitet werden, die ebenso in RNA *silencing*/*RNAi-*Verfahren bzw. in *Antisense*-Verfahren gegen CMV einsetzbar sind.

### Der pflanzenpathogene Nematode Meloidogyne incognita

Nematoden (Fadenwürmer) sind evolutionär die ältesten vielzelligen Würmer. In den zahlreichen, feuchten Lebensräumen, in denen sie vertreten sind, stellen sie oft, sowohl hinsichtlich der Individuenanzahl als auch der Artenvielfalt, die größte Gruppe in der Metazoenfauna (Wikipedia). Sie sind sehr einfach strukturiert: Ihr Körper ist ohne Gliedmaßen, zylindrisch langgestreckt und glatt und von einer elastischen Kutikula umgeben. Die Kutikula wird von einer Epidermiszellschicht abgesondert und bildet das Exoskelett des Nematoden (Bird und Bird, 1991a). Die Kutikula ist durchlässig für Ionen und Wasser und reguliert den hydrostatischen Druck des Nematodenkörpers. Die meisten Nematoden durchlaufen während ihrer Entwicklung vier Häutungen vom juvenilen Stadium (Stadien J1 bis J4) bis zum Erreichen des adulten Stadiums. Dabei wird die Kutikula entweder vollständig abgestoßen oder, wie im Falle von *Meloidogyne,* teilweise absorbiert (Perry und Moens, 2011). Unter der Epidermis sind Muskeln in Längsrichtung entlang der Innenseite des Körpers ausgerichtet, die durch zwei ebenfalls längsseitig verlaufende und durch Nervenringe verbundene Nerven auf Rücken- und Bauchseite aktiviert werden (Bird und Bird, 1991b). Der Kopf des Wurms hat einige Sinnesorgane und einen "Mund", der in einen muskulösen Pharynx (Schlund) mündet. Letzterer dient als Pumpe, um Nahrung in den anschließenden Darm zu ziehen. Der Darm mündet in eine lange, einfache Darmhöhle ohne Muskeln und schließlich einen Anus nahe der Körperspitze. Es gibt kein Gefäßsystem für die Verteilung der verdauten Nahrung und kein Atmungssystem für die Aufnahme oder Verteilung von Sauerstoff. Stattdessen werden Nährstoffe und Abfallstoffe in der pseudo-celomischen Körperhöhle verteilt, deren Inhalt durch einen Ausscheidungskanal entlang jeder Seite des Körpers reguliert wird (Bird und Bird, 1991c).

Eine Nematodenuntergruppe sind pflanzenparasitäre Nematoden (PPN). PPN infizieren viele Pflanzenarten und verursachen weltweit enorme Ernteeinbußen (Blok et al., 2008). Trotz sehr unterschiedlicher Lebensweisen und Ernährungsstrategien haben alle PPN einen hohlen, vorstehenden Stachel, der dazu dient, die Wand von Pflanzenzellen zu durchstoßen und um Sekrete und/oder enzymatisch aktive Proteine zu injizieren, die die Infektion und die Aufnahme von Nährstoffen erleichtern. Produziert werden die Sekrete bzw. Proteine durch drei Ösophagus- "Speicheldrüsen", die Kutikula und chemosensorische Organe (Perry, 1996; Semblat et al., 2001; Curtis, 2007).

Von den PPN verursachen die sog. "sesshaften Endoparasiten" die größten wirtschaftlichen Schäden. Zu diesen gehören die Wurzelknotennematoden (RKNs), von denen die *Meloidogyne-*Arten und hier *Meloidogyne incognita* die bedeutendsten Pflanzenpathogene (Trudgill und Blok, 2001) sind, mit geschätzten verursachten Ernteverlusten von ca. 10 Milliarden Euro pro Jahr weltweit. RKN sind insbesondere in den gemäßigten und tropischen Regionen der Welt zu finden (Blok et al., 2008; Abad und Williamson, 2010). Sie infizieren tausende von Pflanzenarten, darunter praktisch alle Kulturpflanzen, und sie verursachen typische Wurzelverformungen (Gallen), die zu schwachen und ertragsarmen Pflanzen führen.

Der Lebenszyklus von RKNs erstreckt sich über 3-10 Wochen, je nach Nematodenart und Umweltbedingungen. Die wurmförmigen Jungtiere des zweiten Stadiums (J2) schlüpfen aus den Eiern in den Boden, um die Wurzeln des Wirts zu infizieren. In der präparasitären Phase dringen die J2 meist hinter den Wurzelspitzen in das Wurzelgewebe ein, indem sie mit ihrem Stilett die Wurzelzellen physisch durchstechen. Gleichzeitig setzen sie zellwandverändernde Enzyme frei, die eine weitere Wanderung in das Wurzelgewebe und zwischen den Zellen zur Wurzelspitze ermöglichen. Von dort wandern sie in den Gefäßzylinder der Pflanze (Perry und Moens, 2011) und induzieren die Bildung spezialisierter Fraßstellen in Form sogenannter Riesenzellen (GCs). GCs sind hypertrophiert und vielkernig. Sie entstehen durch wiederholte Kernteilungen und Zellwachstum in Abwesenheit von Zellteilung (Jones und Payne, 1978; Caillaud et al., 2008) und sind die einzige Nährstoffquelle des Nematoden. Dieser durchsticht die Zellen, um Zugang zum Zytoplasma zu erhalten, gleichzeitig wird durch Sekrete der hydrostatische Druck der Zellen so verändert, dass eine einfache Aufnahme von Nährstoffen möglich ist (Abad und Williamson, 2010). Die Teilungen der den Nematoden umgebenden Gefäßzellen und der Fresszellen führen zur Bildung einer typischen Galle. Nach Einrichtung der Fraßstellen werden die J2 sesshaft und wachsen dann durch drei weitere Häutungen (parasitische J3 und J4) zu adulten Weibchen oder Männchen heran. Die Weibchen sind sesshaft, während die Männchen wieder mobil werden und aus der Wurzel in den Boden wandern. Mitotische Parthenogenese ist der Fortpflanzungsmodus der RKN (Castagnone-Sereno et al., 2013); das Geschlecht wird durch die Umweltbedingungen bestimmt; bei schlechten Ernährungsbedingungen nimmt die Zahl der Männchen zu (Papadopoulou und Triantaphyllou, 1982). Am Ende ihrer Entwicklung werden die weiblichen RKN birnenförmig. Sie produzieren in einer schützenden, gallertartigen Matrix hunderte bis tausende von Eiern an der Außenfläche der Wurzel, die direkt in die Rhizosphäre abgegeben werden. Im Ei häuten sich die Jungtiere des ersten Stadiums nach der Embryogenese zu J2, die dann schlüpfen, um den Lebenszyklus unter günstigen Bedingungen in einem geeigneten Wirt fortzusetzen (Hussey und Mims, 1991; Chitwood und Perry, 2009; Curtis et al., 2009).

Lange Zeit wurden verschiedenste Chemikalien wie Methylbromide und Carbamate als Nematizide eingesetzt. Die meisten sind inzwischen jedoch aufgrund der mit ihrer Verwendung verbundenen potenziellen Umweltrisiken und Gesundheitsrisiken für Konsumenten verboten. Eine alternative Methode zur PPN-Bekämpfung ist die Fruchtfolge, bei der Nematoden-resistente Pflanzen in verschiedenen Saisons nacheinander angebaut werden. Diese Praxis hat jedoch Grenzen: So haben einige Nematoden wie *Meloidogyne* ein derart breites Wirtsspektrum, dass es schwierig ist, geeignete Kulturen auszuwählen, um den Befallszyklus zu unterbrechen. Bei der biologischen Bekämpfung von PPN werden ein oder mehrere Organismen wie nematophage Pilz- oder Bakterienarten eingesetzt. Diese werden in den Boden eingebracht, wo sie die Nematoden angreifen, ohne das Pflanzenwachstum zu beeinträchtigen (Evans et al., 1993). Derartige "Nematodenprädatoren" sind jedoch schwer in großem Maßstab zu handhaben. Zudem beeinflussen Umweltfaktoren wie Bodenbeschaffenheit, Feuchtigkeit, Temperatur und pH-Wert das Überleben von Biokontrollmitteln signifikant (Chen und Dickson, 2004; Stirling, 2014), was ihren Einsatz nicht erleichtert. Für Nematoden wie *Caenorhabditis elegans* (*C*. *elegans*), aber auch *Meloidogyne incognita* (*M. incognita*), ist beschrieben, dass sie siRNAs und auch dsRNAs über den oben beschriebenen Aufnahmemechanismus von Nährstoffen aufnehmen, und dass siRNAs dann auch im Körper der Tiere durch Aufnahme in Zellen im RNA-*silencing-*System des Wurmes aktiv werden können, z.B. in nematizider Weise durch Inaktivierung von mRNAs essenzieller Proteine (Arguel et al., 2012; Bakhetia et al., 2005; Banakar et al., 2020; Chaudhary et al., 2019; Dalzell et al., 2010a; Dalzell et al., 2010b; Danchin et al., 2013; Dong et al., 2014; Dong et al., 2016; Dutta et al., 2015; Huang et al., 2006; Iqbal et al., 2020; Niu et al., 2012; Papolu et al., 2013; Shivakumara et al., 2016).

### Zusammenfassung der Erfindung

Analog wie oben für CMV beschrieben wurden esiRNAs/ERNAs und edsRNAs charakterisiert, die zuverlässig eine hohe nematizide Wirksamkeit gegen verschiedene Varianten von *M*. *incognita* haben. Von den so charakterisierten *e*siRNAs/*E*RNAs konnten auch andere *s*RNAs bzw. *e*ASO abgeleitet werden, die ebenso in RNA *silencing*/RNAi-Verfahren bzw. in *Antisense*-Verfahren gegen *M. incognita* einsetzbar sind.

### Der pflanzenpathogene Pilz Botrytis cinerea

*Botrytis cinerea* (Teleomorph: *Botryotinia fuckeliana*), der Hauptverursacher der Grauschimmelkrankheit, ist ein aggressiver nekrotropher Pilzerreger, der eine große Anzahl von Pflanzenarten (mehr als 200 Arten) infizieren kann (Elad, 1997; van Kan, 2006; Choquer et al., 2007; Williamson et al, 2007; Nakajima & Akutsu, 2014). *B. cinera* sekretiert dabei unspezifische Phytotoxine, die Zellen eines breiten Spektrums von Pflanzen abtöten (Pinedo et al., 2008). Die verursachten wirtschaftlichen Schäden, weltweit 10 - 100 Milliarden US-Dollar per annum, mit bis zu 40% Verlusten bei Gewächshaus- und Feldkulturen, wenn keine chemischen Bekämpfungsmittel eingesetzt werden, machen *B. cinerea* zu einem der 10 wichtigsten pflanzlichen Pilzpathogene (Dean et al., 2012; Pedras et al., 2011; Villa Rojas et al., 2012). Der Einsatz chemischer Fungizide führt zu erheblichen Umweltbelastungen (Malhat et al., 2015; Oliveira et al., 2015; Tomenson und Matthews, 2009) und wird durch die ernährungsphysiologische Vielseitigkeit von *B. cinerea* limitiert. Zudem wurden mehrere Fälle von Fungizidresistenzen bei *B. cinerea* gemeldet (Leroux, 2007).

Taxonomisch gehört *B. cinerea* zum Phylum Ascomycota in der Klasse der Leotiomycetes und der Familie der *Sclerotiniaceae* (Garfinkel, 2021). Zwei Gruppen, I und II, wurden als phylogenetische Arten vorgeschlagen. Die Stämme der Gruppe I (auch *"Botrytis pseudocinerea"* genannt) und der Gruppe II ("*B. cinerea sensu stricto")* unterscheiden sich in ihrer Ökologie und ihrem Resistenzmuster gegenüber Fungiziden (Fournier et al., 2003, 2005). Das Genom zweier Stämme (B05.10 und T4) wurde komplett sequenziert (Choquer et al., 2007).

Im Gegensatz zu vielen anderen Pflanzenpathogenen tritt *B. cinerea* das ganze Jahr über und unter unterschiedlichsten Umweltbedingungen auf (Nair et al., 1995). Der Infektions-/Ernährungsprozess von *B. cinerea* wird in der Regel durch die folgenden Phasen beschrieben: Konidien, die auf Sklerotien infizierter Pflanzen oder Pflanzenresten produziert werden, heften auf der Oberfläche eines neuen Wirtes und bilden einen Keimschlauch. Dieser entwickelt sich zu einem Appressorium, das das Eindringen in die Wirtsoberfläche erleichtert. Um die Kutikula-Barriere des Wirtes zu überwinden, sondert *B. cinerea* zudem zellwandabbauende Enzyme (CWDEs) ab. Vor Eindringen der Infektionshyphen sterben Epidermis- und Mesophyllzellen ab. Eine Reihe von Metaboliten und Proteinen, die vom Pilz abgesondert werden, induzieren Symptome des programmierten Zelltods (PCD) bzw. verursachen nachweislich den Zelltod (Choquer et al., 2007; Nakajima und Akutsu, 2014).

Zur Kontrolle von *B. cinerea* wurde bereits HIGS eingesetzt. Basis für Hinweise auf Pathogenitäts- bzw. Virulenzgene wurden durch *knock-out*-Mutanten erhalten (Nakajima und Akutsu, 2014). So konnten u.a. auch potenzielle *target-Gene* ermittelt werden, die zum Teil im weiter unten beschriebenen Anwendungsbeispiel verwendet werden (ten Have et al., 1998; Li et al., 2019; Liu et al., 2018; Nerva et al., 2020; Qiao et al., 2021; Schumacher et al., 2008; Segmüller et al., 2008; Soulie' et al., 2006; Ren et al., 2018; Yang et al., 2013; Zheng et al., 2000). Neben HIGS wurden auch bereits *topical*/*transient applications* von dsRNA als alternativer Ansatz in Betracht gezogen (Wang et al., 2016; Weiberg et al., 2013), und einige Studien zeigten auch eine gewisse Wirkung bei verschiedenen Pilze (McLoughlin et al., 2018; Gebremichael et al., 2021). Derzeitiger Stand der Wissenschaft ist, dass die Erfolgsrate dieser Methode stark vom Potenzial des Pilzes abhängt externe RNA aufzunehmen (Qiao et al., 2021).

### Zusammenfassung der Erfindung

Analog wie oben für CMV und *M*. *incognita* beschrieben wurden *e*siRNAs/*E*RNAs und *e*dsRNAs charakterisiert, die zuverlässig eine hohe fungizide Wirksamkeit gegen verschiedene Varianten von *B. cinerea* haben. Von den so charakterisierten *e*siRNAs/*E*RNAs konnten auch andere *s*RNAs bzw. *e*ASO abgeleitet werden, die ebenso in RNA *silencing*/RNAi-Verfahren bzw. in *Antisense*-Verfahren gegen *M*. *incognita* einsetzbar sind.

### Detaillierte Beschreibung der Erfindung

Die Aufgabe der Erfindung wird gelöst durch eine Nukleinsäure gemäß Anspruch 1. Vorteilhafte Ausgestaltungen der Erfindung sind in den von Anspruch 1 abhängigen Ansprüchen beschrieben.

Die Aufgabe der Erfindung wird insbesondere gelöst durch eine Nukleinsäure zum Schutz von Pflanzen vor Pflanzenpathogenen, dadurch gekennzeichnet, dass
a. die Nukleinsäure eine Einzelstrang-RNA ist und aus 21, 22, 23 oder 24 Nukleotiden besteht und eine Nukleinsäure enthält, die aus der Gruppe bestehend aus den Nukleinsäuren mit den SEQ ID NO: 1 bis 180 ausgewählt ist; oder
b. die Nukleinsäure eine Einzelstrang-RNA gemäß Gruppe a. ist und Veränderungen an 1 bis 10 Positionen der Nukleotidsequenz aufweist; oder
c. die Nukleinsäure eine *small* RNA (sRNA, wie z.B. eine *small interfering* RNA, siRNA, oder wie z.B. eine micro RNA, miRNA) ist, deren RNA-Doppelstrang aus komplett komplementären oder teilweise komplementären Nukleinsäuren der Gruppe a. und/oder Gruppe b. besteht; oder
d. die Nukleinsäure eine Doppelstrang-RNA ist, die Nukleotidsequenzen von mindestens zwei sRNAs der Gruppe c. enthält; oder
e. die Nukleinsäure eine Einzelstrang-DNA ist, die eine Sequenz von 12 oder mehr Nukleotiden enthält, die homolog ist (Deoxyribonukleotide statt Ribonukleotide) zu einer der Nukleotidsequenzen der Einzelstrang-RNAs der Gruppen a. oder b..

Gemäß Gruppe a. ist die Nukleinsäure eine Einzelstrang-RNA und besteht aus 21, 22, 23 oder 24 Nukleotiden. Diese Einzelstrang-Nukleinsäure enthält eine Nukleinsäure, die aus der Gruppe bestehend aus den Nukleinsäuren mit den SEQ ID NO: 1 bis 180 ausgewählt ist. Die Einzelstrang-Nukleinsäuren mit den SEQ ID NO: 1 bis 180 sind Komponenten (Einzelstrang-Komponenten wie z.B. *guide strands*) der im Rahmen der Erfindung neu identifizierten effektiv wirksamen Nukleinsäuren, die in Form von *small* RNAs, sRNAs, in RNA *silencing*/RNAi-Verfahren im Pflanzenschutz als Wirkstoffe gegen verschiedene, variable Pflanzenpathogene eingesetzt werden können.

Gemäß Gruppe b. ist die Nukleinsäure der Erfindung eine Einzelstrang-RNA gemäß Gruppe a., die Veränderungen an 1 bis 10 Positionen der Nukleotidsequenz aufweist. Die Nukleinsäuren der Gruppe b. stellen somit Varianten der Nukleinsäuren der Gruppe a. dar. "Veränderung" im Sinne der Erfindung bedeutet "Basenaustausch", d.h. dass eine Base, die ausgewählt ist aus Adenin (A), Uracil (U), Guanin (G) und Cytosin (C)) durch irgendeine der anderen Basen ausgetauscht sein kann. Solche Basenaustausche können an 1 bis 10 Positionen der Nukleotidsequenz einer Nukleinsäure der Gruppe a. erfolgen. Die Basenaustausche sind vorzugsweise an jeder Position unabhängig voneinander, d.h. jede der 1 bis 10 Positionen der Nukleotidsequenz einer Nukleinsäure der Gruppe a. kann unabhängig voneinander durch irgendeine der anderen Basen ausgetauscht sein.

Gemäß Gruppe c. ist die Nukleinsäure der Erfindung eine *small* RNA (sRNA, wie z.B. eine *small interfering* RNA, siRNA, oder wie z.B. eine micro RNA, miRNA), deren RNA-Doppelstrang aus komplett komplementären oder teilweise komplementären Nukleinsäuren der Gruppe a. und/oder Gruppe b. besteht.

*Small interfering* RNAs, abgekürzt siRNAs, sind kurze, doppelsträngige, nicht-kodierende Ribonukleinsäure-Moleküle von 20 bis 25 Basenpaaren Länge, die am 5'-Ende phosphoryliert sind und am 3'-Ende einen 2 Nukleotide (nt) langen, einzelsträngigen Überhang (*overhang*) aufweisen. Wie oben beschrieben, werden siRNAs unter natürlichen Bedingungen in einer Zelle aus doppelsträngigen Bereichen von RNA-Molekülen, in der Regel nicht zelleigener Fremd-RNA, generiert; sie können aber, in synthetischer Form, auch gezielt in RNA *silencing*/RNAi-Verfahren eingesetzt werden. Bis auf den erwähnten 3'-Überhang sind die beiden konstituierenden Einzelstränge einer siRNA vollständig (heisst über die gesamte Länge der RNA) über komplementäre Basenpaarung (in der Regel Adenin (A) mit Uracil (U) und Guanin (G) mit Cytosin (C)) basengepaart und bilden einen RNA-Duplex. Während des RNA *silencing*/RNAi-Prozesses assoziiert einer der beiden Einzelstrang-Komponenten der siRNA an komplementäre einzelsträngige Bereiche (*target-sites*) anderer Ribonukleinsäure-Moleküle, hier *target-RNAs* genannt. Dies sind überwiegend die kognaten RNAs, aus denen die siRNAs urprünglich generiert wurden. Die Funktion der *target-RNAs* kann so auf verschiedene Weise unterbunden oder moduliert werden. So kann in Eukaryoten mit siRNAs die Replikation von Pathogenen wie Viren oder die Expression zellulärere Gene auf der post-transkriptionellen Ebene unterdrückt werden.

MicroRNAs, abgekürzt miRNAs, sind kurze, nicht-kodierende Ribonukleinsäuren, die in Form von Vorläufermolekülen durch das zelluläre Genom kodiert sind. Nach Transkription und Prozessierung bilden komplementäre Bereiche der aus in der Regel 21 bis 23 nt langen RNA-Strängen bestehenden miRNA über Basenpaarung Doppelstränge aus. Im Gegensatz zu siRNAs können diese doppelsträngigen Bereiche bei miRNAs durch einzelsträngige Bereiche in Form sog. *"mismatches"* (betreffend einzelne Nukleotide) und/oder *"loops"* und/oder *"bulges"* (betreffend mehrere Nukleotide) unterbrochen sein. Wie siRNAs inaktivieren oder modulieren miRNAs über Bindung (Hybridisierung) an komplett oder nicht komplett komplementäre Bereiche einer *target-RNA* deren Funktion über den RNA *silencing*/RNAi-Prozess. miRNAs haben entsprechend eine zentrale Funktion in der post-transkriptionellen Regulation der zellulären Genexpression und können, wie siRNAs, auch zur künstlichen Modulation der zellulären Genexpression eingesetzt werden.

Wie oben bereits ausgeführt, werden zwei Nukleinsäurestränge als "komplementär" bezeichnet, wenn deren Nukleotide miteinander basenpaaren können. Ursprünglich definiert wurde die Paarungsregel bei DNA nach der klassischen Paarungsregel der Nukleotidbasen nach Watson und Crick, wo über Wasserstoffbrückenbindungen Adenin (A) aus einem Nukleinsäurestrang mit Thymin (T) aus dem anderen Nukleinsäurestrang und Guanin (G) aus einem Nukleinsäurestrang mit Cytosin (C) aus dem anderen Nukleinsäurestrang interagieren (basenpaaren) können. Dadurch bildet DNA einen Doppelstrang, wobei jeder Strang sozusagen die Negativversion des anderen Stranges ist.

RNA-Moleküle können nach einem ähnlichen Muster, d.h. über Wasserstoffbrückenbindungen von Adenin (A) mit Uracil (U) (RNA-Moleküle enthalten kein Thymin sondern Uracil) und von Guanin (G) mit Cytosin (C), aber auch über andere, hier nicht ausgeführte Basenpaarungen, Doppelstränge ausbilden. Diese Doppelstränge können intermolekular, also zwischen verschiedenen RNA-Molekülen oder intramolekular, also innerhalb eines RNA-Moleküls ausgebildet werden. Wie oben ausgeführt, können doppelsträngige Bereiche einer RNA viele Nukleotidbausteine (u.U. hunderte oder tausende) und damit entsprechend viele Basenpaare umfassen. In diesem Fall wird, wie oben ausgeführt, der Begriff "dsRNA" verwendet. Intramolekular ausgebildetet Doppelstränge sind eine wesentliche Determinante der Ausbildung komplexer Strukturen innerhalb einer RNA. Ebenso ist es möglich, dass komplementäre RNA- und DNA-Moleküle Doppelstränge, sog. DNA:RNA-Hybride oder DNA:RNA-Heteroduplexe, ausbilden.

"Komplett komplementär" definiert in der Regel zwei Nukleinsäurestränge, die über deren gesamte Länge und über deren gesamte Zahl an Nukleotidbausteinen Basenpaarungen und damit einen Doppelstrang ausbilden können. Wie ausgeführt, weisen funktionelle siRNAs und andere sRNAs Überhänge einzelner oder mehrerer Nukleotide an den Termini auf. Ebenso können dsRNAs Überhänge einzelner oder mehrerer Nukleotide an den Termini oder *spacer* (siehe Definition oben) aufweisen. Der Begriff "komplett komplementär" im Sinne der Erfindung bezieht sich hier entsprechend auf die Nukleotidsequenzen der Nukleinsäuren der Gruppen a., b., c. und d. **exklusive** dieser Überhänge oder *spacer.*

"Teilweise komplementär" im Sinne der Erfindung definiert entsprechend Nukleinsäurestränge, die nicht komplett komplementär sind und entsprechend nicht über die gesamte Länge und gesamt Zahl an Nukleotidbausteinen Basenpaarungen ausbilden können. Doppelsträngige Bereiche dieser Nukleinsäuren werden durch einzelsträngige Bereiche wie z.B. *mismatches, loops* und/oder *bulges* unterbrochen.

Besonders bevorzugt in der Gruppe c. ist es, wenn die Nukleinsäure eine siRNA ist, deren RNA-Doppelstrang (Duplex) aus komplett komplementären Nukleinsäuren der Gruppe a. und/oder Gruppe b. besteht. Hierbei handelt es sich um im Rahmen der Erfindung neu identifizierte *e*siRNAs/*E*RNAs (effektiv wirksame siRNAs), die in RNA *silencing*/RNAi-Verfahren im Pflanzenschutz als Wirkstoffe gegen variable Pflanzenpathogene eingesetzt werden können.

Bevorzugt in der Gruppe c. ist auch, wenn die Nukleinsäure eine sRNA, wie z.B. eine miRNA, ist, deren RNA-Doppelstrang aus teilweise komplementären Nukleinsäuren der Gruppe a. und/oder Gruppe b. besteht. Hierbei handelt es sich um sRNAs, deren Nukleotidsequenzen von den oben genannten *e*siRNAs/*E*RNAs abgeleitet werden können und die ebenfalls in RNA *silencing*/RNAi-Verfahren im Pflanzenschutz als Wirkstoffe gegen variable Pflanzenpathogene eingesetzt werden können.

Gemäß Gruppe d. ist die Nukleinsäure der Erfindung eine Doppelstrang-RNA, die Nukleotidsequenzen von mindestens zwei sRNAs der Gruppe c. enthält. Hierbei handelt es sich um im Rahmen der Erfindung neuartig konstruierte doppelsträngige Ribonukleinsäuren (edsRNAs; effektiv wirksame doppelsträngige RNAs), die die Nukleotidsequenzen von identifizierten *e*siRNAs/*E*RNAs oder davon abgeleiteten anderen sRNAs, wie z.B. miRNAs, enthalten und die in RNA *silencing*/RNAi-Verfahren im Pflanzenschutz als Wirkstoffe gegen variable Pflanzenpathogene eingesetzt werden können.

Im Sinne der Erfindung bedeutet dies, dass der RNA-Doppelstrang dieser Nukleinsäuren Nukleotidsequenzen von mindestens zwei Nukleinsäuren der Gruppe c. enthält.

In einer bevorzugten Ausführungsform enthält eine Nukleinsäure der Gruppe d. die Nukleotidsequenzen von mindestens zwei *small* RNAs (sRNAs, wie z.B. eine *small interfering* RNA, siRNA, oder z.B. einer micro RNA, miRNA), deren RNA-Doppelstrang aus komplett oder teilweise komplementären Nukleinsäuren der Gruppe a. und/oder Gruppe b. besteht. In einer weiteren bevorzugten Ausführungsform weist eine Nukleinsäure der Gruppe d. die Nukleotidsequenzen von mindestens zwei siRNAs auf, deren RNA-Doppelstrang aus komplett komplementären Nukleinsäuren der Gruppe a. und/oder Gruppe b. besteht. In einer weiteren bevorzugten Ausführungsform enthält eine Nukleinsäure der Gruppe d. die Nukleotidsequenzen von mindestens zwei sRNAs, wie z.B. miRNAs, auf, deren RNA-Doppelstrang aus teilweise komplementären Nukleinsäuren der Gruppe a. und/oder Gruppe b. besteht.

In einer weiteren bevorzugten Ausführungsform enthält eine Nukleinsäure der Gruppe d. die Nukleotidsequenzen von zwei, drei, vier, fünf...bis unendlich vielen sRNAs der Gruppe c.. Vorzugsweise enthält eine Nukleinsäure der Gruppe d. die Nukleotidsequenzen von 2 bis 100 sRNAs der Gruppe c.. Besonders bevorzugt ist es, wenn die Nukleinsäure der Gruppe d. die Nukleotidsequenzen von 2 bis 90, 2 bis 80, 2 bis 70, 2 bis 60, 2 bis 50, 2 bis 40 oder 2 bis 30 sRNAs der Gruppe c. enthält. Insbesondere bevorzugt ist es, wenn die Nukleinsäure der Gruppe d. die Nukleotidsequenzen von 2 bis 20 oder 2 bis 10 sRNAs der Gruppe c. enthält. In einer anderen Ausführungsform der Erfindung ist es bevorzugt, wenn die Nukleinsäure der Gruppe d. die Nukleotidsequenzen von mehr als 2 sRNAs der Gruppe c. enthält, also mindestens 3, 4, 5, 6, 7, 8, 9, 10 oder mehr (bis zu 100) sRNAs der Gruppe c..

Gemäß Gruppe e. ist die Nukleinsäure eine Einzelstrang-DNA, die eine Sequenz von 12 oder mehr Nukleotiden enthält, die homolog ist (Deoxyribonukleotide statt Ribonukleotide) zu einer der Nukleotidsequenzen der Einzelstrang-RNAs der Gruppen a. oder b..

Unter Sequenzhomologie versteht man die Ähnlichkeit von Nukleotid- oder Aminosäuresequenzen aufgrund identischer chemischer Bausteine in mehr oder weniger großen Teilbereichen der Molekülketten von Peptiden, RNA oder DNA. Bei völlig identischen Molekülketten liegt eine 100%ige Sequenzhomologie vor. Im vorliegenden Fall bedeutet Homologie eine 100%ige Sequenzhomologie über 12 oder mehr Nukleotide, die in der Einzelstrang-DNA enthalten sind, zu einer der Einzelstrang-RNAs der Gruppen a. oder b., wobei die DNA aus Deoxyribonukleotiden und die RNA aus Ribonukleotiden besteht.

Gruppe e. umfasst die mittels der Erfindung bereitgestellten *antisense*-Deoxyribonukleinsäure (DNA)-Oligonukleotide (ASO), deren Sequenz von den im Rahmen der Erfindung identifizierten *e*siRNAs/*E*RNAs abgeleitet werden kann. In analoger Terminologie zum Begriff *e*siRNAs/*E*RNAs werden hier die von den Sequenzen der Einzelstrang-RNA-Komponenten der *e*siRNAs/*E*RNAs der Gruppen a. und b. abgeleiteten ASO entsprechend als *e*ASO bezeichnet. *e*ASO enthalten zu den Einzelstrang-RNA-Komponenten der *e*siRNAs/*E*RNAs der Gruppen a. und b. homologe DNA-Sequenzen (d.h. Deoxynukleotide statt Ribonukleotide; Thymidin statt Uridin) und können somit auch über entsprechende Basenpaarung an die jeweiligen *target*-sites in den a-Sites der *target-RNAs* hybridisieren und aktiv werden. Die Funktionsweise von ASO ist ähnlich aber nicht identisch zu der von sRNAs. Wie oben detailliert ausgeführt, binden ASO über Basenpaarung (Hybridisierung) an komplett oder teilweise komplementäre Bereiche *(target-sites)* einer *target-RNA.* Die inaktivierende Wirkweise von ASO auf einer *target-RNA* erfolgt aber nicht wie bei sRNAs über RNA *silencing*/RNAi sondern über Antisense-Prozesse, d.h. z.B. Inhibition der Translation oder endonukleolytischen Abbau durch RNasen vom Typus RNase H (siehe oben).

Gemäß einem Aspekt der Erfindung ist die Nukleinsäure eine Nukleinsäure zum Schutz von Pflanzen vor dem Pflanzenpathogen Cucumber mosaic virus (CMV).

Vorzugsweise ist die Nukleinsäure zum Schutz vor CMV eine Nukleinsäure, die gegen eine *target-RNA* des CMV gerichtet ist. Besonders bevorzugt ist es, wenn die Nukleinsäure zum Schutz vor CMV eine Nukleinsäure ist, die gegen eine *target-RNA* des CMV gerichtet ist, wobei die *target-RNA* des CMV ausgewählt ist aus den *target*-RNAs mit den SEQ ID NO: 189 und 190.

Eine Nukleinsäure, die gegen eine *target-RNA* des CMV ausgewählt aus den SEQ ID NO: 189 und 190 gerichtet ist, ist vorzugsweise eine Ribonuklein- oder Deoxyribonukleinsäure, die mindestens eine Nukleinsäure enthält oder daraus besteht, die aus der Gruppe bestehend aus den Nukleinsäuren mit den SEQ ID NO: 1 bis 92 ausgewählt ist.

Die *target-RNA* des CMV mit der SEQ ID NO: 189 wird hierin im Weiteren auch mit "CMV-RNA 2" bezeichnet. Die *target-RNA* des CMV mit der SEQ ID NO: 190 wird hierin im Weiteren auch mit "CMV-RNA 3" bezeichnet.

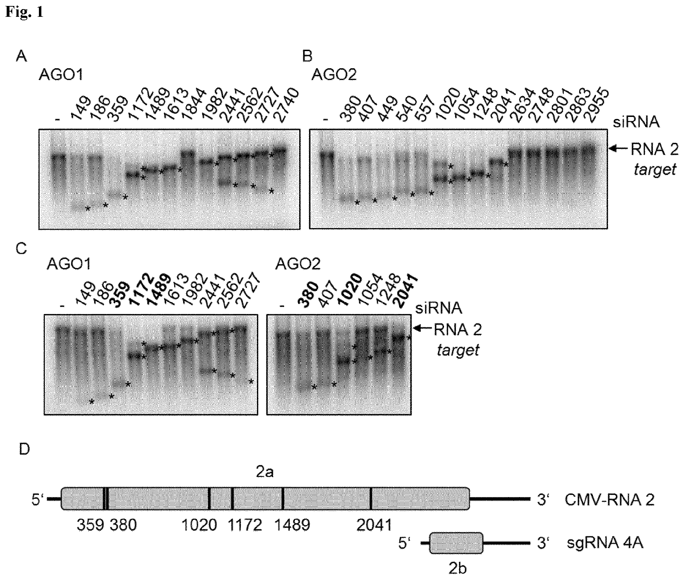

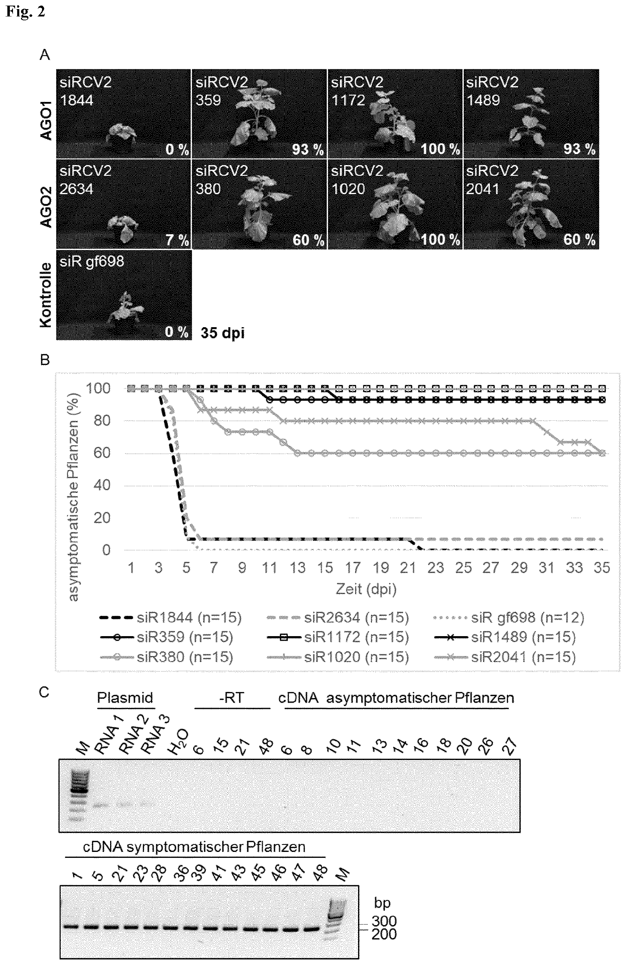

Wie in Anwendungsbeispiel 1 beschrieben, wurden gegen CMV-RNA 2 (SEQ ID NO: 189) Nukleinsäuren identifiziert, von denen ein erheblicher Anteil im Slicer-Assay (Spalt-Assay) eine effiziente Hydrolyse der *target-RNA* (CMV-RNA 2) induziert und Pflanzen gegen CMV-Infektionen schützt (siehe auch **Figur 1** A und B sowie **Figur 2** A, B und C). Das Screening wurde sowohl mit AGO1 (L-Version; Gursinsky et al., 2015) als auch mit AGO2 aus *Nicotiana benthamiana* (Nb) durchgeführt.

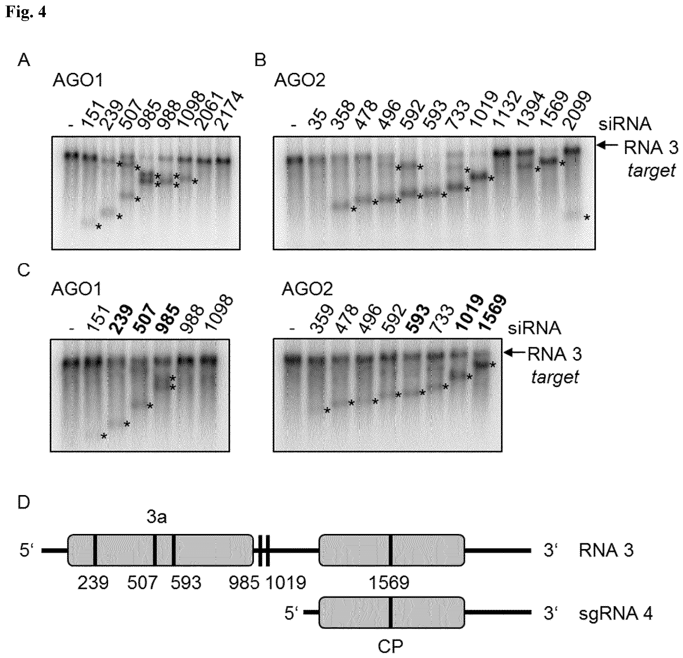

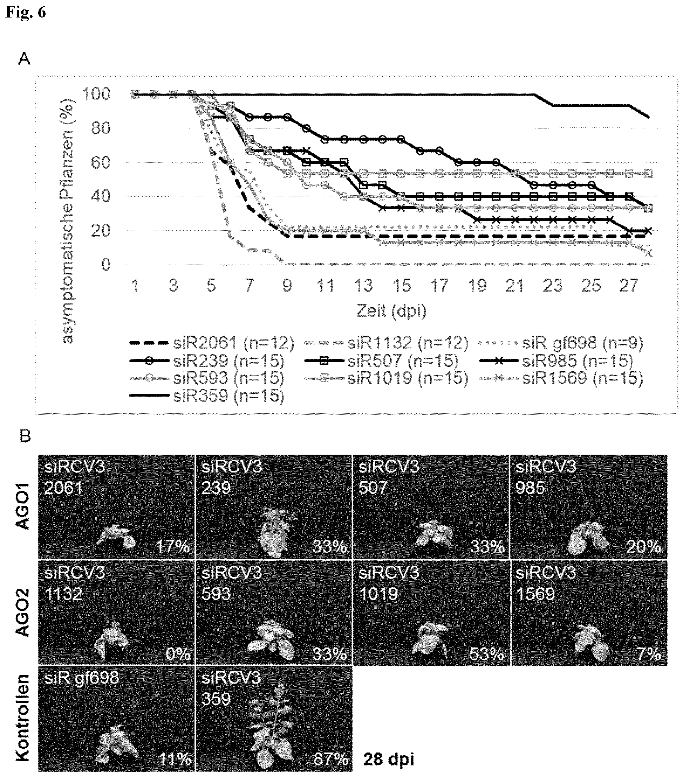

Wie in Anwendungsbeispiel 1 beschrieben, wurden gegen CMV-RNA 3 (SEQ ID NO: 190) Nukleinsäuren identifiziert, von denen ein erheblicher Anteil im Slicer-Assay (Spalt-Assay) eine effiziente Hydrolyse der *target-RNA* (CMV-RNA 3) induziert und Pflanzen gegen CMV-Infektionen schützt (siehe auch **Figur 4** A, B und C sowie **Figur 6** A und B). Das Screening wurde sowohl mit AGO1 (L-Version; Gursinsky et al., 2015) als auch mit AGO2 aus *Nicotiana benthamiana* (Nb) durchgeführt.

Eine Nukleinsäure, die gegen eine *target*-RNA CMV-RNA 2 des CMV mit der SEQ ID NO: 189 gerichtet ist, ist vorzugsweise eine Ribonuklein- oder Deoxyribonukleinsäure, die mindestens eine Nukleinsäure enthält oder daraus besteht, die aus der Gruppe bestehend aus den Nukleinsäuren mit den SEQ ID NO: 1 bis 52 ausgewählt ist.

Eine Nukleinsäure, die gegen eine *target*-RNA CMV-RNA 2 des CMV mit der SEQ ID NO: 189 gerichtet ist und im Screening mit AGO1 identifiziert wurde, ist vorzugsweise eine Ribonuklein- oder Deoxyribonukleinsäure, die mindestens eine Nukleinsäure enthält oder daraus besteht, die aus der Gruppe bestehend aus den Nukleinsäuren mit den SEQ ID NO: SEQ ID NO: 1 bis 12 und SEQ ID NO: 27 bis 38, vorzugsweise mit den SEQ ID NO: 1 bis 4, 6 bis 11, 27 bis 30 und 32 bis 37 ausgewählt ist.

Eine Nukleinsäure, die gegen eine *target*-RNA CMV-RNA 2 des CMV mit der SEQ ID NO: 189 gerichtet ist und im Screening mit AGO2 identifiziert wurde, ist vorzugsweise eine Ribonuklein- oder Deoxyribonukleinsäure, die mindestens eine Nukleinsäure enthält oder daraus besteht, die aus der Gruppe bestehend aus den Nukleinsäuren mit den SEQ ID NO: SEQ ID NO: 13 bis 26 und SEQ ID NO: 39 bis 52, vorzugsweise mit den SEQ ID NO: 14 bis 17, 21 bis 25, 40 bis 43 und 47 bis 51 ausgewählt ist.

Eine Nukleinsäure, die gegen eine *target*-RNA CMV-RNA 3 des CMV mit der SEQ ID NO: 190 gerichtet ist, ist vorzugsweise eine Ribonuklein- oder Deoxyribonukleinsäure, die mindestens eine Nukleinsäure enthält oder daraus besteht, die aus der Gruppe bestehend aus den Nukleinsäuren mit den SEQ ID NO: 53 bis 92 ausgewählt ist.

Eine Nukleinsäure, die gegen eine *target*-RNA CMV-RNA 3 des CMV mit der SEQ ID NO: 190 gerichtet ist und im Screening mit AGO1 identifiziert wurde, ist vorzugsweise eine Ribonuklein- oder Deoxyribonukleinsäure, die mindestens eine Nukleinsäure enthält oder daraus besteht, die aus der Gruppe bestehend aus den Nukleinsäuren mit den SEQ ID NO: SEQ ID NO: 53 bis 55, 59, 63, 64, 66, 67, 70-75, 79, 83, 84, 86, 87 und 90 bis 92, vorzugsweise mit den SEQ ID NO: 54, 55, 59, 63, 64, 66, 74, 75, 79, 83, 84, und 86 ausgewählt ist.

Eine Nukleinsäure, die gegen eine *target-RNA* CMV-RNA 3 des CMV mit der SEQ ID NO: 190 gerichtet ist und im Screening mit AGO2 identifiziert wurde, ist vorzugsweise eine Ribonuklein- oder Deoxyribonukleinsäure, die mindestens eine Nukleinsäure enthält oder daraus besteht, die aus der Gruppe bestehend aus den Nukleinsäuren mit den SEQ ID NO: 56 bis 58, 60 bis 62, 65, 68, 69, 76 bis 78, 80 bis 82, 85, 88 und 89 ausgewählt ist.

Gemäß einem weiteren Aspekt der Erfindung ist die Nukleinsäure eine Nukleinsäure zum Schutz von Pflanzen vor dem Pflanzenpathogen *Meloidogyne incognita.*

Vorzugsweise ist die Nukleinsäure zum Schutz vor *Meloidogyne incognita* eine Nukleinsäure, die gegen eine *target-RNA* von *Meloidogyne incognita* gerichtet ist. Besonders bevorzugt ist es, wenn die Nukleinsäure zum Schutz vor *Meloidogyne incognita* eine Nukleinsäure ist, die gegen eine *target-RNA* von *Meloidogyne incognita* gerichtet ist, wobei die *target-RNA* von *Meloidogyne incognita* ausgewählt ist aus den *target*-RNAs mit den SEQ ID NO: 191, 192 und 193.

Die *target-RNA* von *Meloidogyne **incognita*** mit der SEQ ID NO: 191 wird hierin im Weiteren auch mit "SPF" bezeichnet. Die *target-RNA* von *Meloidogyne incognita* mit der SEQ ID NO: 192 wird hierin im Weiteren auch mit "INT" bezeichnet. Die *target-RNA* von *Meloidogyne incognita* mit der SEQ ID NO: 193 wird hierin im Weiteren auch mit "ACT" bezeichnet.

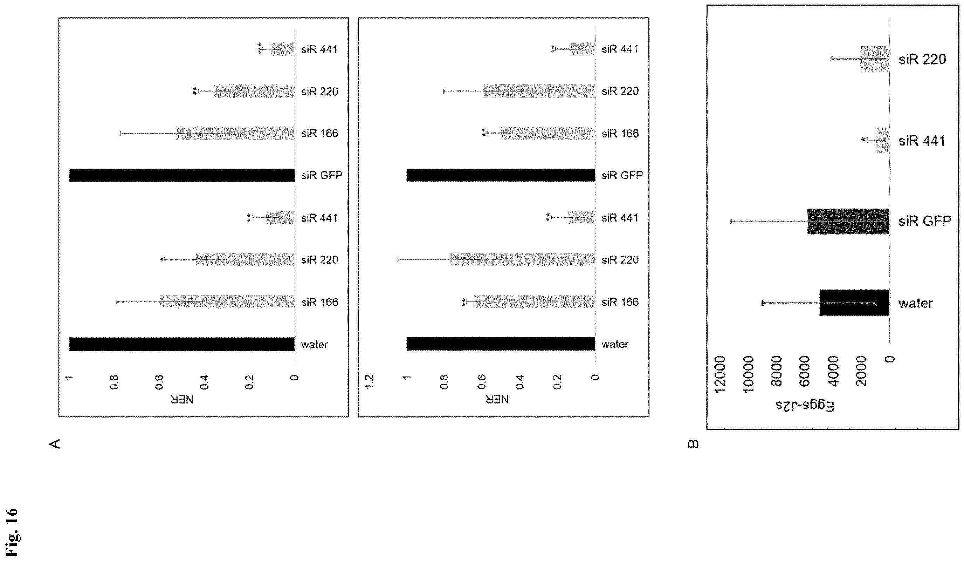

Wie in Anwendungsbeispiel 3 beschrieben, wurden gegen SPF (SEQ ID NO: 191) Nukleinsäuren identifiziert, von denen ein erheblicher Anteil im Slicer-Assay (Spalt-Assay) und *in vivo* nach Aufnahme in den Nematoden eine effiziente Hydrolyse der *target-RNA* (SPF) induziert und Pflanzen gegen *Meloidogyne incognita*-Infektionen schützt (siehe auch **Figur 16** A und B). Das Screening wurde mit dem AGO2 Protein von *Nicotiana benthamiana* (Nb) durchgeführt.

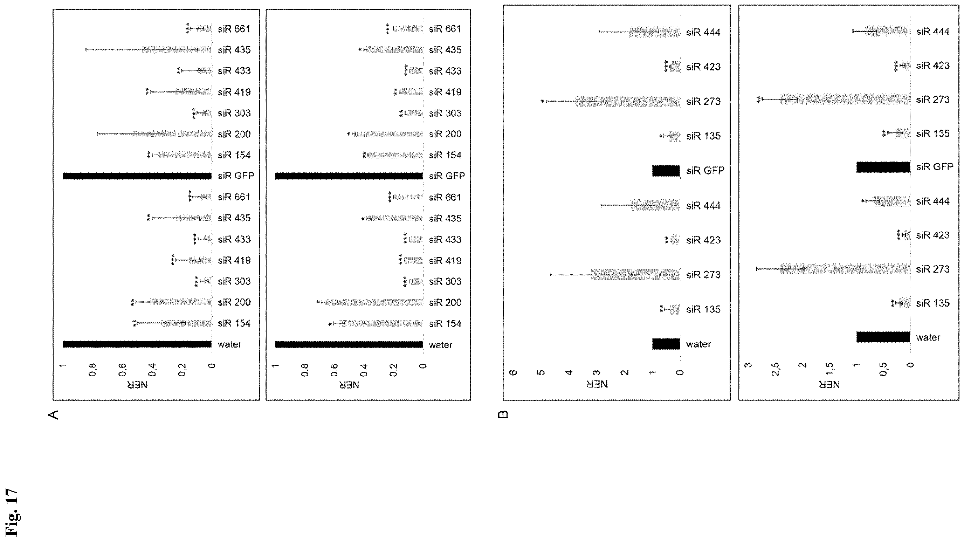

Wie in Anwendungsbeispiel 3 beschrieben, wurden gegen INT (SEQ ID NO: 192) Nukleinsäuren identifiziert, von denen ein erheblicher Anteil im Slicer-Assay (Spalt-Assay) und *in vivo* nach Aufnahme in den Nematoden eine effiziente Hydrolyse der *target-RNA* (SPF) induziert und Pflanzen gegen *Meloidogyne incognita*-Infektionen schützt (siehe auch **Figur 17** A und B). Das Screening wurde mit dem AGO2 Protein von *Nicotiana benthamiana* (Nb) durchgeführt.

Wie in Anwendungsbeispiel 3 beschrieben, wurden gegen ACT (SEQ ID NO: 193) Nukleinsäuren identifiziert, von denen ein erheblicher Anteil im Slicer-Assay (Spalt-Assay) und *in vivo* nach Aufnahme in den Nematoden eine effiziente Hydrolyse der *target-RNA* (SPF) induziert und Pflanzen gegen *Meloidogyne incognita*-Infektionen schützt (siehe auch **Figur 17** A und B). Das Screening wurde mit dem AGO2 Protein von *Nicotiana benthamiana* (Nb) durchgeführt.

Eine Nukleinsäure, die gegen eine *target-RNA* von *Meloidogyne incognita* ausgewählt aus den SEQ ID NO: 191, 192 und 193 gerichtet ist, ist vorzugsweise eine Ribonuklein- oder Deoxyribonukleinsäure, die mindestens eine Nukleinsäure enthält oder daraus besteht, die aus der Gruppe bestehend aus den Nukleinsäuren mit den SEQ ID NO: 93 bis 120 ausgewählt ist.

Eine Nukleinsäure, die gegen eine *target-RNA* von *Meloidogyne incognita* SPF mit der SEQ ID NO: 191 gerichtet ist, ist vorzugsweise eine Ribonuklein- oder Deoxyribonukleinsäure, die mindestens eine Nukleinsäure enthält oder daraus besteht, die aus der Gruppe bestehend aus den Nukleinsäuren mit den SEQ ID NO: 93 bis 95 sowie 107 bis 109 ausgewählt ist.

Eine Nukleinsäure, die gegen eine *target-RNA* von *Meloidogyne incognita* INT mit der SEQ ID NO: 192 gerichtet ist, ist vorzugsweise eine Ribonuklein- oder Deoxyribonukleinsäure, die mindestens eine Nukleinsäure enthält oder daraus besteht, die aus der Gruppe bestehend aus den Nukleinsäuren mit den SEQ ID NO: 96 bis 99, 110 bis 113 ausgewählt ist.

Eine Nukleinsäure, die gegen eine *target-RNA* von *Meloidogyne incognita* ACT mit der SEQ ID NO: 193 gerichtet ist, ist vorzugsweise eine Ribonuklein- oder Deoxyribonukleinsäure, die mindestens eine Nukleinsäure enthält oder daraus besteht, die aus der Gruppe bestehend aus den Nukleinsäuren mit den SEQ ID NO: 100 bis 106, 114 bis 120 ausgewählt ist.

Gemäß einem weiteren Aspekt der Erfindung ist die Nukleinsäure eine Nukleinsäure zum Schutz von Pflanzen vor dem Pflanzenpathogen *Botrytis cinerea.*

Vorzugsweise ist die Nukleinsäure zum Schutz vor *Botrytis cinerea* eine Nukleinsäure, die gegen eine *target-RNA* von *Botrytis cinerea* gerichtet ist. Besonders bevorzugt ist es, wenn die Nukleinsäure zum Schutz vor *Botrytis cinerea* eine Nukleinsäure ist, die gegen eine *target-RNA* von *Botrytis cinerea* gerichtet ist, wobei die target-RNA von *Botrytis cinerea* ausgewählt ist aus den *target-RNAs* mit den SEQ ID NO: 194, 195, 196, 197, 198 und 199.

Die *target-RNA* von *Botrytis cinerea* mit der SEQ ID NO: 194 wird hierin im Weiteren auch mit "VDS" bezeichnet. Die *target-RNA* von *Botrytis cinerea* mit der SEQ ID NO: 195 wird hierin im Weiteren auch mit "DCTN" bezeichnet. Die *target-RNA* von *Botrytis cinerea* mit der SEQ ID NO: 196 wird hierin im Weiteren auch mit "SAC" bezeichnet. Die *target-RNA* von *Botrytis cinerea* mit der SEQ ID NO: 197 wird hierin im Weiteren auch mit "ERG" bezeichnet. Die *target-RNA* von *Botrytis cinerea* mit der SEQ ID NO: 198 wird hierin im Weiteren auch mit "EF" bezeichnet. Die *target-RNA* von *Botrytis cinerea* mit der SEQ ID NO: 199 wird hierin im Weiteren auch mit "CHS" bezeichnet.

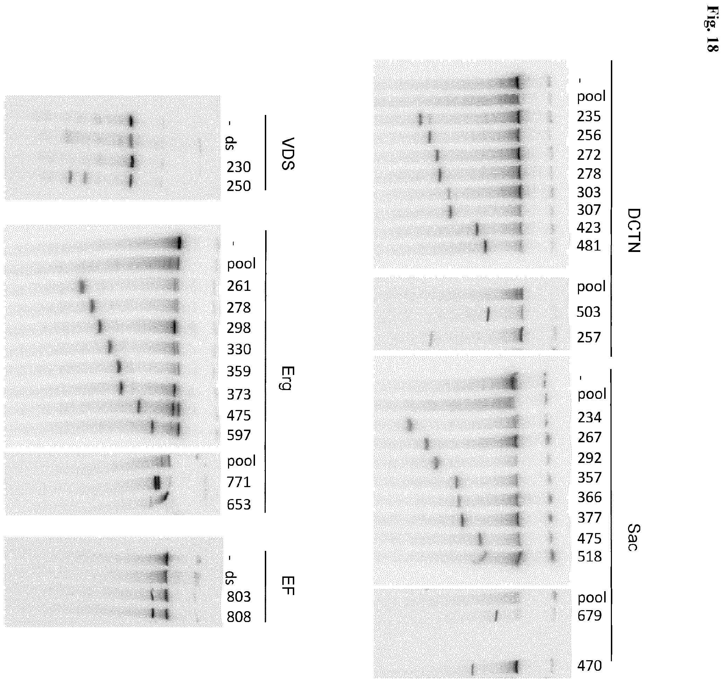

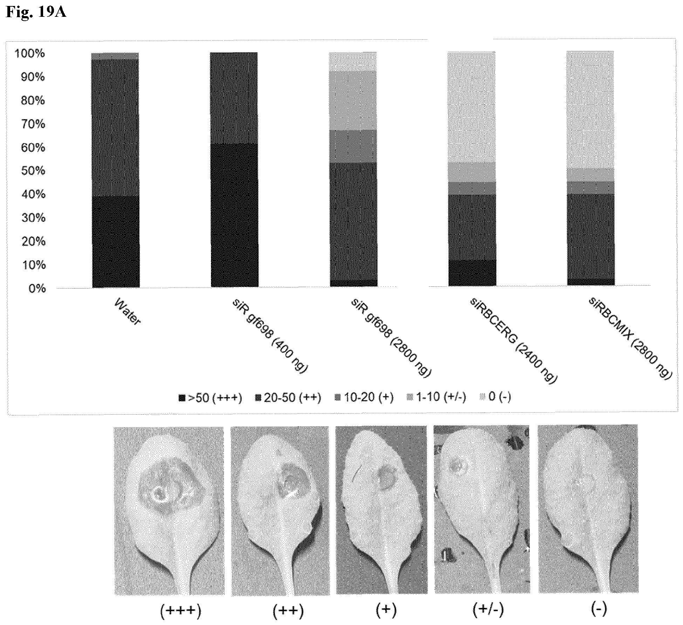

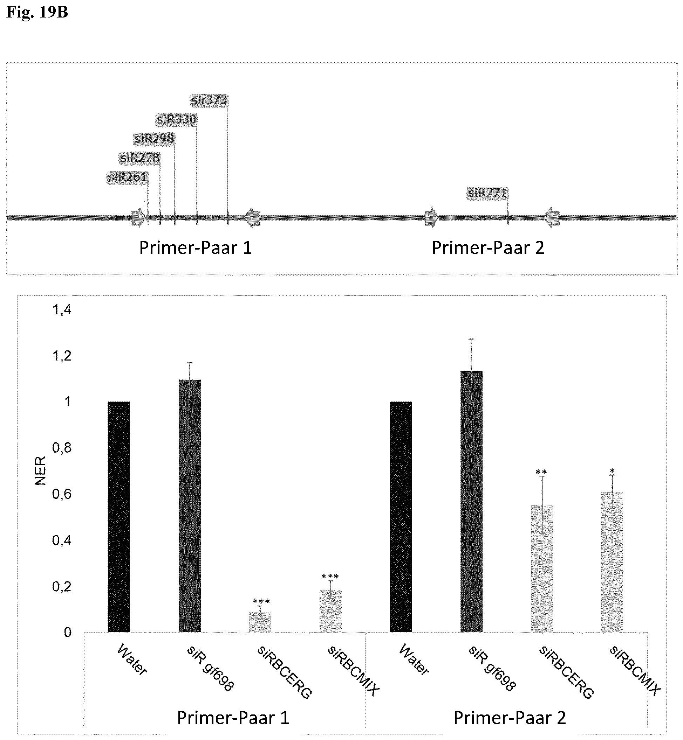

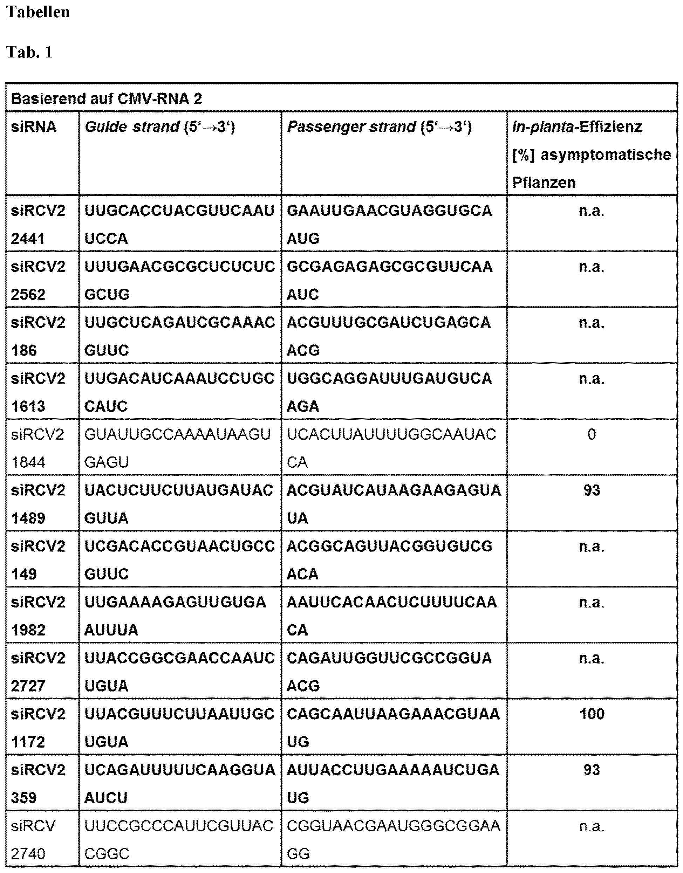

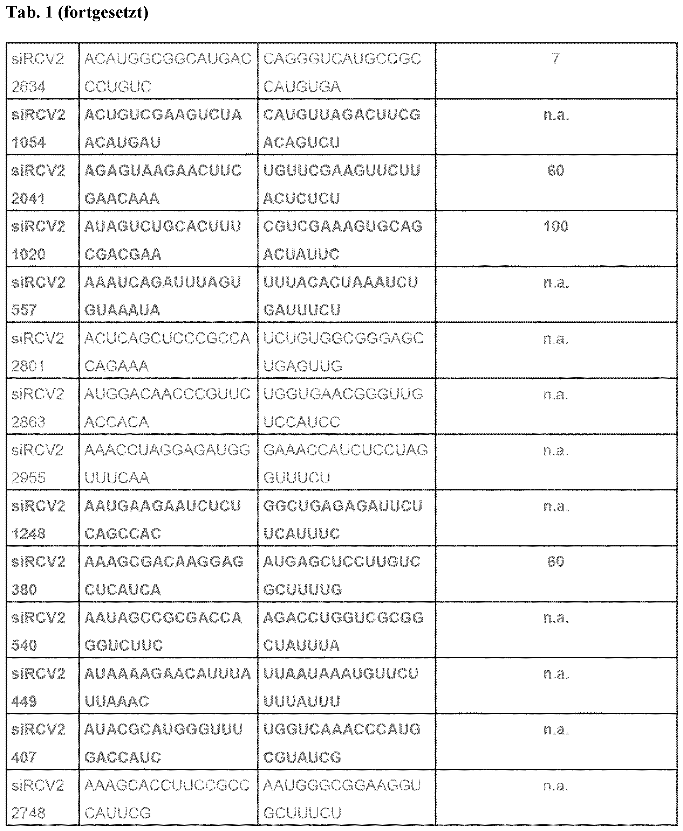

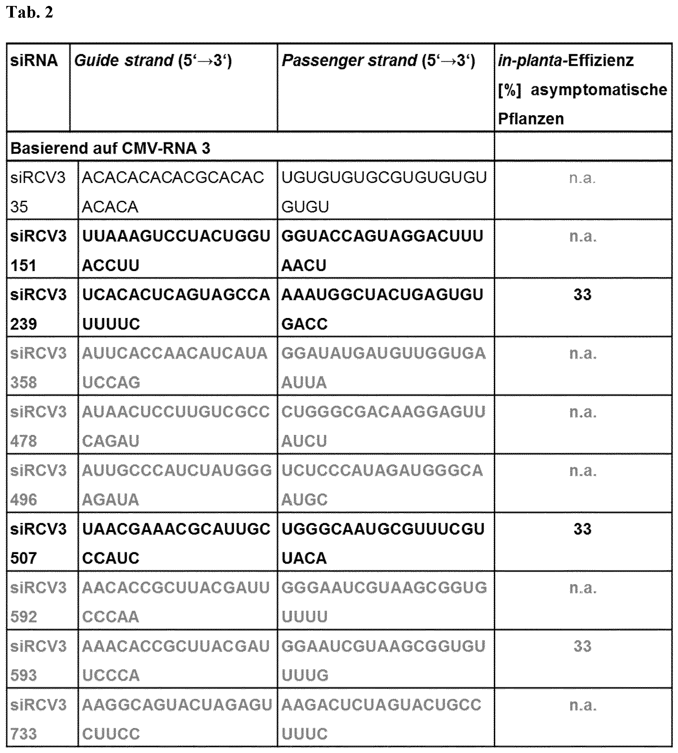

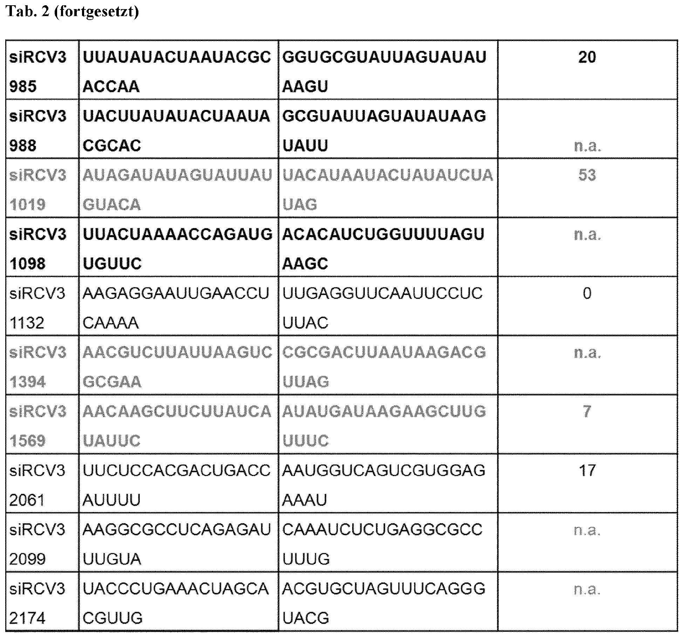

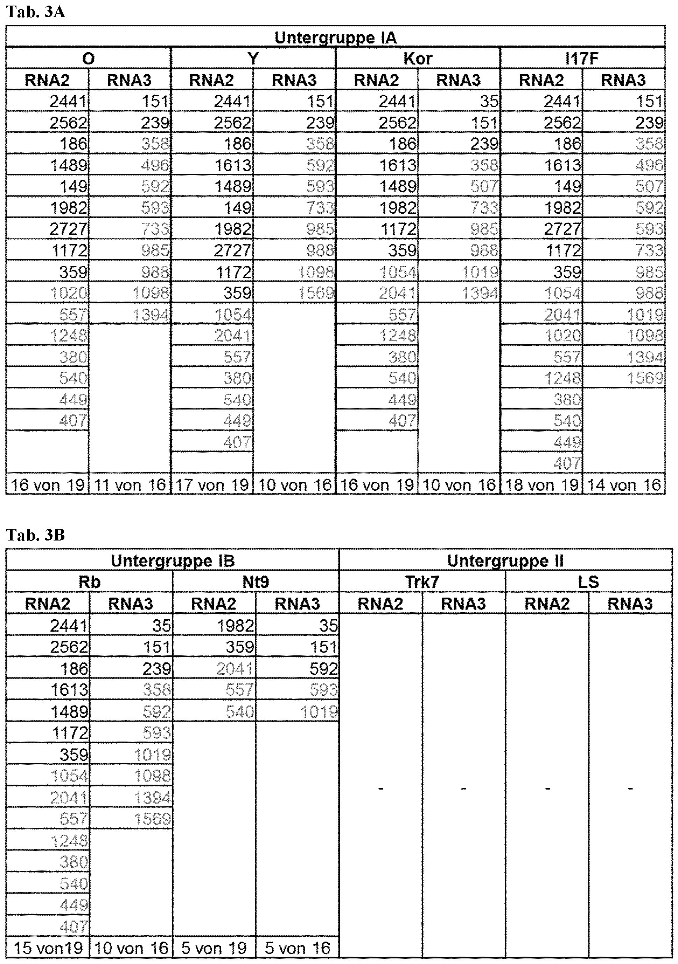

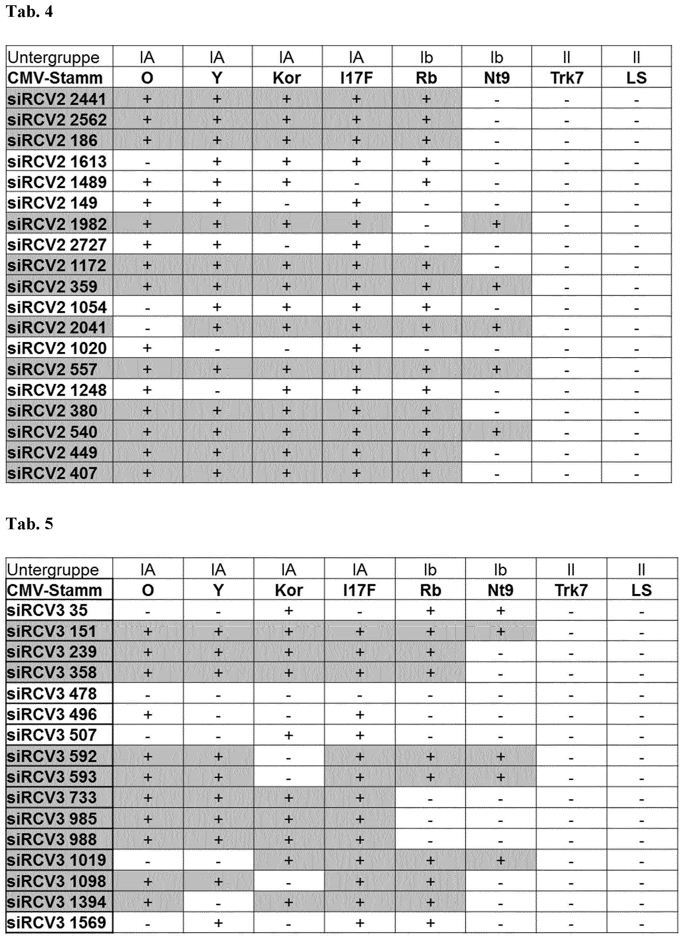

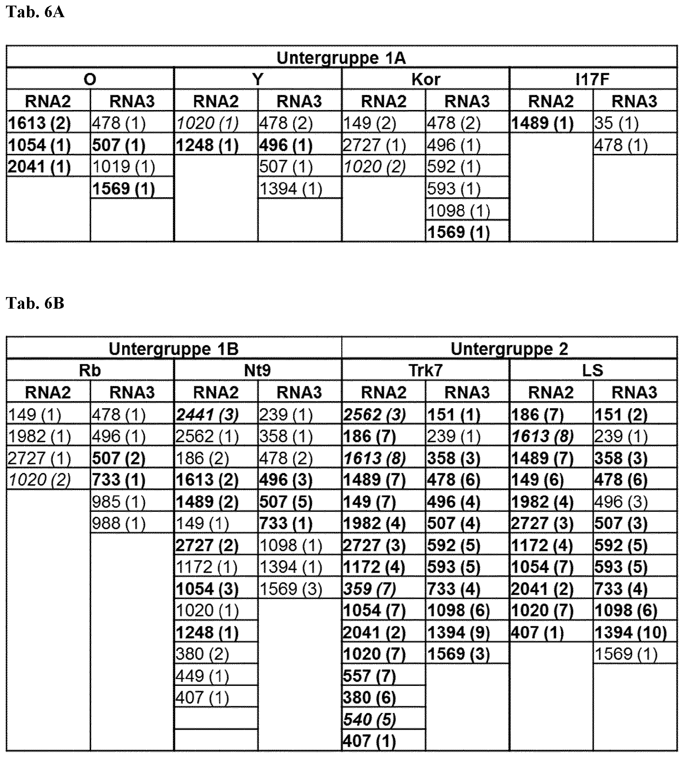

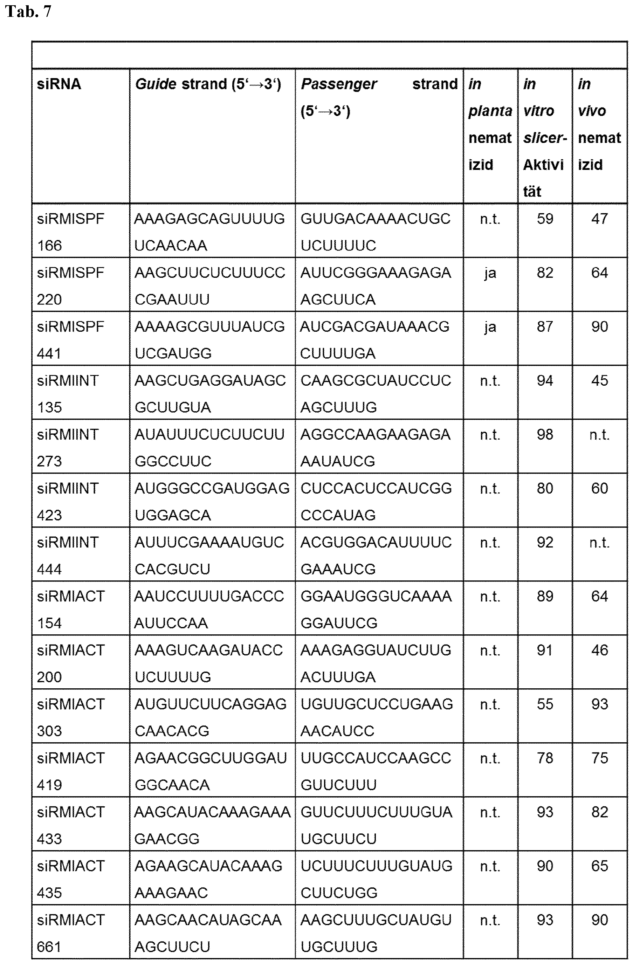

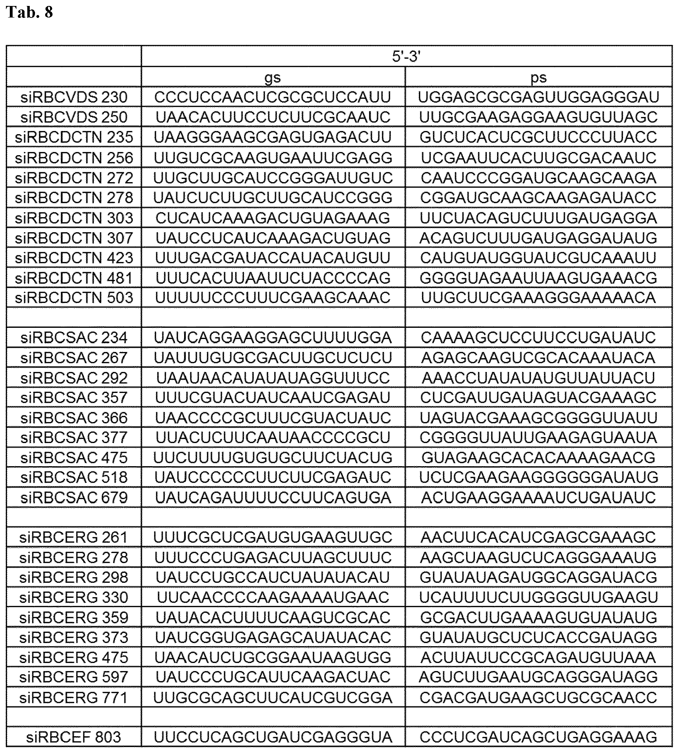

Wie in Anwendungsbeispiel 4 beschrieben, wurden gegen VDS (SEQ ID NO: 194) Nukleinsäuren identifiziert, von denen ein erheblicher Anteil im Slicer-Assay (Spalt-Assay) eine effiziente Hydrolyse der *target-RNA* (VDS) induziert und Pflanzen gegen *Botrytis cinerea*-Infektionen schützt (siehe auch **Figuren 18** und **19** A und B). Das Screening wurde mit dem AGO1 Protein von *Colletotrichum graminicula* (Cg) durchgeführt.

Wie in Anwendungsbeispiel 4 beschrieben, wurden gegen DCTN (SEQ ID NO: 195) Nukleinsäuren identifiziert, von denen ein erheblicher Anteil im Slicer-Assay (Spalt-Assay) eine effiziente Hydrolyse der *target-RNA* (DCTN) induziert und Pflanzen gegen *Botrytis cinerea*-Infektionen schützt (siehe auch **Figuren 18** und **19** A und B). Das Screening wurde mit dem AGO1 Protein von *Colletotrichum graminicula* (Cg) durchgeführt.

Wie in Anwendungsbeispiel 4 beschrieben, wurden gegen SAC (SEQ ID NO: 196) Nukleinsäuren identifiziert, von denen ein erheblicher Anteil im Slicer-Assay (Spalt-Assay) eine effiziente Hydrolyse der *target-RNA* (SAC) induziert und Pflanzen gegen *Botrytis cinerea*-Infektionen schützt (siehe auch **Figuren 18** und **19** A und B). Das Screening wurde mit dem AGO1 Protein von *Colletotrichum graminicula* (Cg) durchgeführt.

Wie in Anwendungsbeispiel 4 beschrieben, wurden gegen ERG (SEQ ID NO: 197) Nukleinsäuren identifiziert, von denen ein erheblicher Anteil im Slicer-Assay (Spalt-Assay) eine effiziente Hydrolyse der *target-RNA* (ERG) induziert und Pflanzen gegen *Botrytis cinerea*-Infektionen schützt (siehe auch **Figuren 18** und **19** A und B). Das Screening wurde mit dem AGO1 Protein von *Colletotrichum graminicula* (Cg) durchgeführt.

Wie in Anwendungsbeispiel 4 beschrieben, wurden gegen EF (SEQ ID NO: 198) Nukleinsäuren identifiziert, von denen ein erheblicher Anteil im Slicer-Assay (Spalt-Assay) eine effiziente Hydrolyse der *target-RNA* (ERG) induziert und Pflanzen gegen *Botrytis cinerea*-Infektionen schützt (siehe auch **Figuren 18** und **19** A und B). Das Screening wurde mit dem AGO1 Protein von *Colletotrichum graminicula* (Cg) durchgeführt.

Wie in Anwendungsbeispiel 4 beschrieben, wurden gegen CHS (SEQ ID NO: 199) Nukleinsäuren identifiziert, von denen ein erheblicher Anteil im Slicer-Assay (Spalt-Assay) eine effiziente Hydrolyse der *target-RNA* (CHS) induziert und Pflanzen gegen *Botrytis cinerea*-Infektionen schützt (siehe auch **Figuren 18** **und** **19** **A und B).** Das Screening wurde mit dem AGO1 Protein von *Colletotrichum graminicula* (Cg) durchgeführt.

Eine Nukleinsäure, die gegen eine target-RNA von *Botrytis cinerea* ausgewählt aus den SEQ ID NO: 194, 195, 196, 197, 198 und 199 gerichtet ist, ist vorzugsweise eine Ribonuklein- oder Deoxyribonukleinsäure, die mindestens eine Nukleinsäure enthält oder daraus besteht, die aus der Gruppe bestehend aus den Nukleinsäuren mit den SEQ ID NO: 121 bis 180 ausgewählt ist.

Eine Nukleinsäure, die gegen eine *target-RNA* von *Botrytis cinerea* VDS mit der SEQ ID NO: 194 gerichtet ist, ist vorzugsweise eine Ribonuklein- oder Deoxyribonukleinsäure, die mindestens eine Nukleinsäure enthält oder daraus besteht, die aus der Gruppe bestehend aus den Nukleinsäuren mit den SEQ ID NO: 121, 122, 151, 152 ausgewählt ist.

Eine Nukleinsäure, die gegen eine *target-RNA* von *Botrytis cinerea* DCTN mit der SEQ ID NO: 195 gerichtet ist, ist vorzugsweise eine Ribonuklein- oder Deoxyribonukleinsäure, die mindestens eine Nukleinsäure enthält oder daraus besteht, die aus der Gruppe bestehend aus den Nukleinsäuren mit den SEQ ID NO: 123 bis 131, 153 bis 161 ausgewählt ist.

Eine Nukleinsäure, die gegen eine *target-RNA* von *Botrytis cinerea* SAC mit der SEQ ID NO: 196 gerichtet ist, ist vorzugsweise eine Ribonuklein- oder Deoxyribonukleinsäure, die mindestens eine Nukleinsäure enthält oder daraus besteht, die aus der Gruppe bestehend aus den Nukleinsäuren mit den SEQ ID NO: 132 bis 140, 162 bis 170 ausgewählt ist.

Eine Nukleinsäure, die gegen eine *target-RNA* von *Botrytis cinerea* ERG mit der SEQ ID NO: 197 gerichtet ist, ist vorzugsweise eine Ribonuklein- oder Deoxyribonukleinsäure, die mindestens eine Nukleinsäure enthält oder daraus besteht, die aus der Gruppe bestehend aus den Nukleinsäuren mit den SEQ ID NO: 141 bis 149, 171 bis 179 ausgewählt ist.

Eine Nukleinsäure, die gegen eine *target-RNA* von *Botrytis cinerea* EF mit der SEQ ID NO: 198 gerichtet ist, ist vorzugsweise eine Ribonuklein- oder Deoxyribonukleinsäure, die mindestens eine Nukleinsäure enthält oder daraus besteht, die aus der Gruppe bestehend aus den Nukleinsäuren mit den SEQ ID NO: 150 und 180 ausgewählt ist.

In einem weiteren Aspekt der Erfindung ist die Nukleinsäure eine Doppelstrang-RNA, wobei diese Doppelstrang-RNA vorzugsweise Nukleotidsequenzen enthält, die aus pseudo-siRNA-Sequenzen und Sequenzen von minimal zwei und maximal einer unendlichen Zahl von sRNAs gemäß Gruppe c. bestehen. Vorzugsweise enthält die Doppelstrang-RNA Nukleotidsequenzen, die aus pseudo-siRNA-Sequenzen und Sequenzen von 2 bis 10.000 sRNAs gemäß Gruppe c. bestehen. Besonders bevorzugt ist es, wenn die Doppelstrang-RNA Nukleotidsequenzen enthält, die aus pseudo-siRNA-Sequenzen und Sequenzen von 2 bis 5.000 oder 2 bis 2.500 sRNAs gemäß Gruppe c. bestehen. Ganz besonders bevorzugt ist es, wenn die Doppelstrang-RNA Nukleotidsequenzen enthält, die aus pseudo-siRNA-Sequenzen und Sequenzen von 2 bis 1.000, 2 bis 500, 2 bis 250, 2 bis 100 oder 2 bis 50 sRNAs gemäß Gruppe c. bestehen Hierbei handelt es sich um die Nukleinsäuren der Gruppe d., "*e*dsRNAs", wie oben und in Anwendungsbeispiel 2 beschrieben.

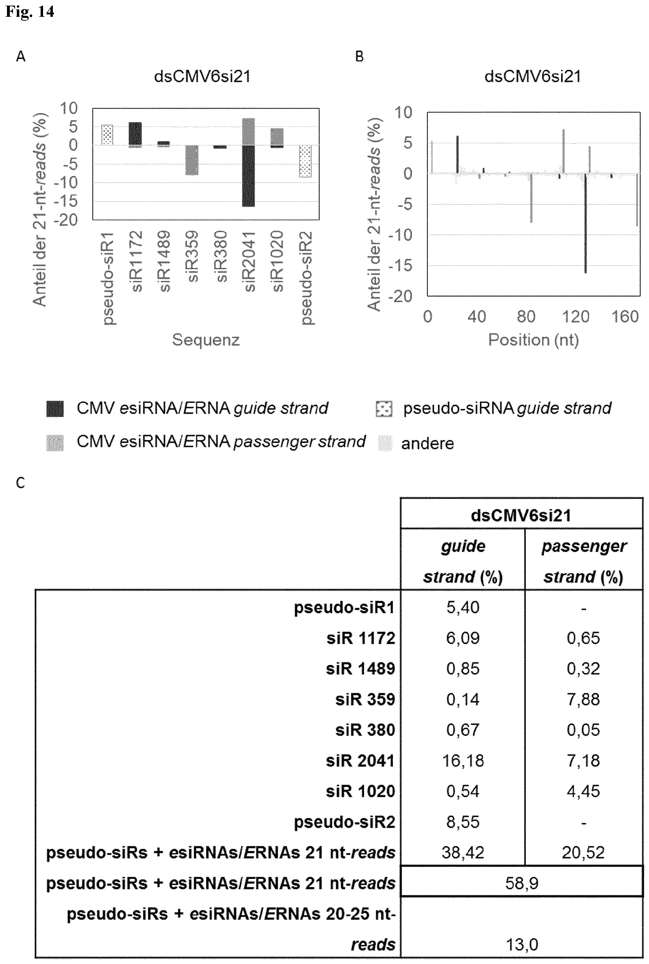

Eine "pseudo-siRNA-Sequenz" oder "pseudo-siRNA" im Sinne der Erfindung ist eine doppelsträngige Ribonukleotidsequenz beliebiger Komposition, die, entsprechend der geplanten Prozessierung der jeweiligen edsRNA durch Dicer-Enzyme oder DCLs, 21, 22, 23 oder 24 nt lang ist (siehe auch Anwendungsbeispiel 2). Pseudo-siRNA-Sequenzen befinden sich an den Termini der doppelsträngigen RNA, und sie zwingen durch ihre Präsenz die auf dieser RNA aktiven Dicer oder DCLs in ein "getaktetes *processing".* Wie in Ausführungsbeispiel 2 detailliert beschrieben, bedeutet getaktetes *processing,* dass entsprechend Position und Länge der pseudo-siRNA-Sequenzen und entsprechend der Aktivität der beteiligten Dicer/DCLs die endonukleolytischen Spaltungen der doppelsträngigen RNA so erfolgen, dass die dabei präferenziell generierten sRNAs die gleiche Länge wie die pseudo-siRNAs aufweisen. Dass dies so erfolgt wurde erfindungsgemäß demonstriert **(****Figur 14****).**

Pseudo-siRNA-Sequenzen können darüber hinaus Elemente enthalten, die im Sinne der Erfindung, für eine erfolgreiche Transkription und Prozessierung der jeweiligen RNA wichtig sind: Dies können Bereiche eines Transkriptionspromotors oder -terminators sein; es können aber auch Transportsignale oder Teile eines Ribozyms (*ribozymes*) sein, welches das korrekte 5'- bzw. 3'-Ende der jeweiligen RNAs durch *self-splicing* (eigenkatalysierte Spaltung) generiert.

Unter Transkriptionspromotoren versteht man Signalsequenzen in einem DNA-Doppelstrang, dessen einer Strang (*template*-Strang), für ein RNA-Molekül kodiert. Der Transkriptionspromotor wird von einem DNA-abhängigen RNA-Polymerasekomplex erkannt und bedingt durch die Assoziation an diese Promotorsequenz kann der RNA-Polymerasekomplex die Transkription (Synthese) eines RNA-Moleküls initiieren, dessen Nukleotidsequenz komplementär zum DNA *template*-Strang ist. Transkriptionsaktivatoren, die an den RNA-Polymerasekomplex und/oder andere DNA-Sequenzen binden, können die Transkription spezifisch induzieren. Beispiele für Transkriptionspromotoren sind virale Promotoren wie z.B. der Promotor des T7-Phagen (T7-Promotor). Beispiele für zelluläre Promotoren sind z.B. der Pol I und der Pol II-Promotor. Andere Beispiele gebräuchlicher viraler Promotoren sind: T3-Promotor (Promotor des T3-Phagen), SP6-Promotor (Promotor des SP6-Phagen), CMV-Promotor (Promotor des humanen Cytomegalievirus). Andere Beispiele gebräuchlicher zellulärer Promotoren sind: GAL-Promotoren, LAC4-Promotoren, Actin-Promotor, Pol III-Promotor. Andere geeignete Transkriptionspromotoren sind dem Fachmann bekannt.

Unter Transkriptionsterminatoren versteht man Nukleotidsequenzen, die, wie Transkriptionspromotoren von einer RNA-kodierenden DNA kodiert werden. Werden diese Sequenzen vom RNA-Polymerasekomplex transkribiert, kommt es zur Bildung von Protein-RNA-Komplexen, die den RNA-Polymerasekomplex zur Termination der Transkription veranlassen. Beispiel eines Transkriptionsterminators ist z.B. der Transkriptionsterminator des Vesicular stomatitis virus (VSV). Andere geeignete Transkriptionsterminatoren sind dem Fachmann bekannt.

Transportsignale in RNA-Moleküle bedingen die Bindung von Proteinen, die den gezielten Transport dieser RNA-Moleküle in oder aus bestimmten Zellkompartimenten ermöglichen. Ein Beispiel sind nukleare RNA-Exportsignale, die den Export von RNA-Molekülen aus dem Zellkern in das Zellzytoplasma ermöglichen. Andere Transportsignale sind dem Fachmann bekannt.

Ribozyme (*ribozymes*) sind katalytisch aktive RNA-Moleküle, die wie Enzyme chemische Reaktionen katalysieren. Beispiele sind z.B. Hammerhead (*hammerhead;* HH)-Ribozyme (Meyer und Masquida 2014) oder das Hepatitis Delta Virus (HDV)-Ribozym (Avis et al. 2012). Diese Ribozyme können selbständig eine endonukleolytische Spaltung des RNA-Moleküls katalysieren, dessen Bestandteil sie sind (*self-cleavage* oder *self-splicing*)*.*

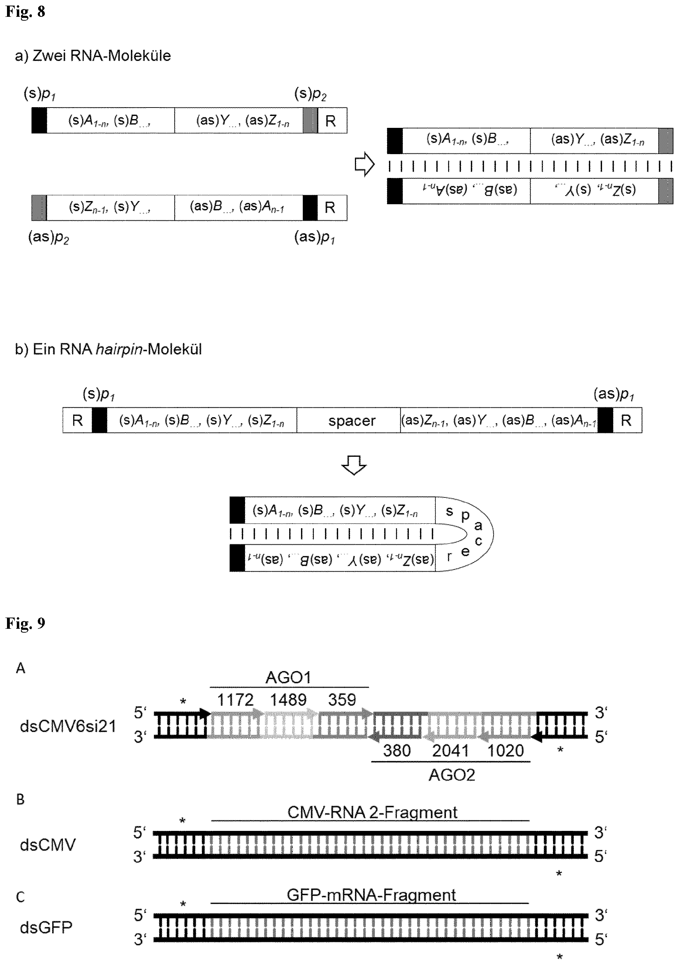

Das Grundprinzip des Aufbaus im Sinne der Erfindung konzipierter edsRNAs stellt sich somit folgendermaßen dar (siehe auch **Figuren 8** bis **11****):**
Die Sequenz enthält mindestens eine pseudo-siRNA-Sequenz, die, wie ausgeführt, ein getaktetes *processing* durch Dicer/DCLs ermöglicht. Zudem enthält die Sequenz der edsRNA eine Anzahl von 5' nach 3' "aufgereihter" Sequenzen von *e*siRNAs/*E*RNAs oder von *e*siRNAs/*E*RNAs abgeleiteter anderer sRNAs wie z.B. miRNAs. Diese Sequenzen können aus verschiedenen *screens* stammen, und entsprechend können die *e*siRNA/*E*RNAs bzw. davon abgeleiteten sRNAs in verschiedenen AGO/RISC aktiv sein. Die *e*siRNA/*E*RNAs bzw. davon abgeleiteten sRNAs, die eine edsRNA konstituieren, können somit gegen unterschiedliche *target-RNAs* gerichtet sein, die aus einem oder aus unterschiedlichen Organismen stammen.

Die Generierung einer edsRNA kann auf zweierlei Weise erfolgen: Aus zwei komplementären RNA-Molekülen oder aus einem RNA-Molekül, das zwei komplementäre Teilbereiche enthält (**Figur 8**). Im zweiten Fall werden die beiden komplementären Teilbereiche der transkribierten RNA durch einen *spacer* miteinander verbunden und bilden einen *hairpin.* Der *spacer* ist eine Sequenz beliebiger Komposition mit einer Minimallänge von 4 Nukleotiden (siehe Definition für *hairpins* oben), die für den spezifischen Zweck der edsRNA-Konstruktion aber funktionelle Bereiche wie beispielsweise Ribozyme (wie HH- oder HDV-Ribozyme), Transkriptionspromotoren oder Transkriptionsterminatoren, Transportsignale oder *Splice*-Stellen enthalten kann. *Splice*-Stellen sind Sequenzmotive in Bereichen von Vorläufer-RNA-Molekülen wie Introns, die von der *Splice*-Maschinerie einer Zelle erkannt werden. Die *Splice*-Maschinerie katalysiert die vollständige oder teilweise Entfernung der Intronsequenz. Durch die Präsenz dieser Elemente dient die *spacer*-Sequenz, zusätzlich zu der Funktion die beiden komplementären Einzelstrangkomponenten einer dsRNA zu verbinden, weiteren Zwecken: Enthält sie beispielsweise *Splice*-Stellen kann der *spacer* im Zuge der Expression der RNA *in vivo* durch die *Splice-*Maschinerie der Zelle, verkürzt werden. Da der *spacer* im Gegensatz zu den doppelsträngigen RNA-Regionen sensitiv ist gegenüber Ribonukleasen (wie z.B. die Einzelstrang-spezifischen RNasen T1 oder A), die in der Zelle präsent sind, kann diese Sequenz durch diese RNasen auch komplett entfernt werden (siehe auch **Figur 8**). Beispiele für *spacer* sind Sequenzen von Introns aus mRNA-Vorläufermolekülen ("prä-mRNAs"), die alle für ein *splicing* notwendigen Erkennungssequenzen enthalten. Diese Erkennungssequenzen sind dem Fachmann bekannt.

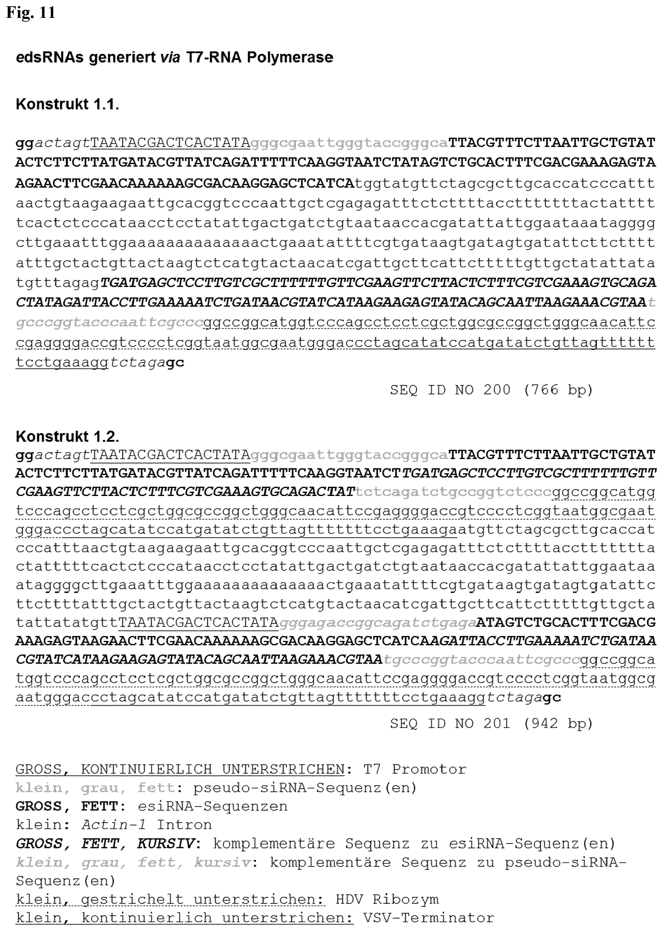

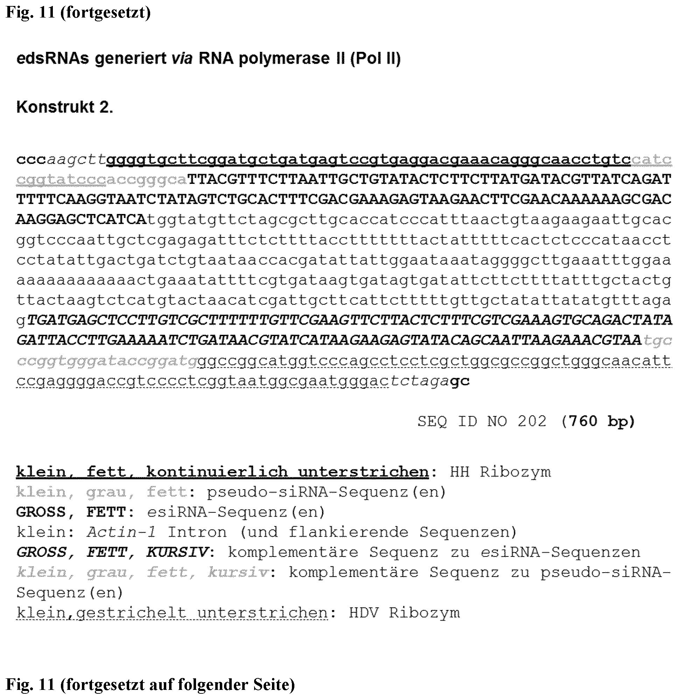

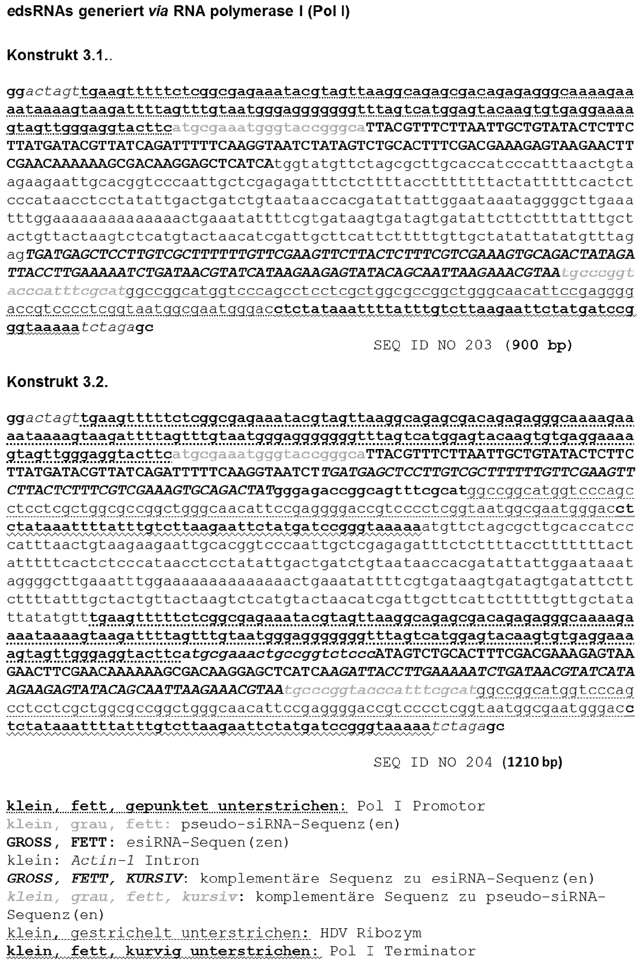

Die Transkription der RNAs kann *in vitro* oder *in vivo* erfolgen. Der Doppelstrang wird durch Hybridisierung der komplementären RNA-Stränge erhalten **(****Figur 8****).** Die Transkription kann durch verschiedenste Promotoren (siehe beispielhaft **Figur 11****)** erfolgen. Die Transkriptionstermination kann durch jedwede Art von Transkriptionsterminatoren (wie z.B. VSV Transkriptionsterminator siehe beispielhaft **Figur 11****)** erfolgen.

Je nach Art der Generierung sind die Termini der edsRNAs entweder stumpf (*blunt*)*,* oder sie enthalten einen Überhang (-Ü). Sie können auf unterschiedliche Weise, z.B. über *"run-off* Transkription" ("Herunterlaufen des RNA-Polymerasekomplexes vom DNA-template") bzw. Termination des jeweiligen RNA-Polymerasekomplexes über einen Transkriptionsterminator oder durch die Aktivität von Ribozymen über *self-splicing* generiert werden.

Die Sequenzen beider Stränge sind so konzipiert, dass bei der Prozessierung durch DCLs jeweils die authentischen sRNA *guide* und *passenger* strand-Sequenzen generiert werden. Das *processing,* das überwiegend zur Generierung der konstituierenden *e*siRNAs/*E*RNAs bzw. davon abgeleiteten sRNAs führt, wird durch die Präsenz der pseudo-siRNA-Sequenzen und das damit "getaktete *processing"* durch die DCLs/Dicer sichergestellt (siehe Anwendungsbeispiel 2 und **Figuren 8-14****).**

Die Doppelstrang-RNA der Erfindung kann in einer Ausführungsform somit stumpfe (blunt), in einer anderen Ausführungsform überhängende Enden aufweisen.

In einer weiteren Ausführungsform der Erfindung kann die Doppelstrang-RNA einen *spacer* enthalten.

In einer weiteren Ausführungsform der Erfindung enthalten die pseudo-siRNAs und/oder *spacer* in der Nukleinsäure gemäß der Erfindung Elemente, die ausgewählt sind aus Transkriptionspromotoren, Transkriptionsterminatoren, Transportsignalen, *Splice*-Stellen und Ribozymen.

In einer besonders bevorzugten Ausführungform der Erfindung ist die Doppelstrang-RNA der Erfindung ausgewählt aus der Gruppe bestehend aus den Nukleinsäuren mit der SEQ ID NO: SEQ ID NO: 181, 182, 185, 186 sowie 200 bis 204.

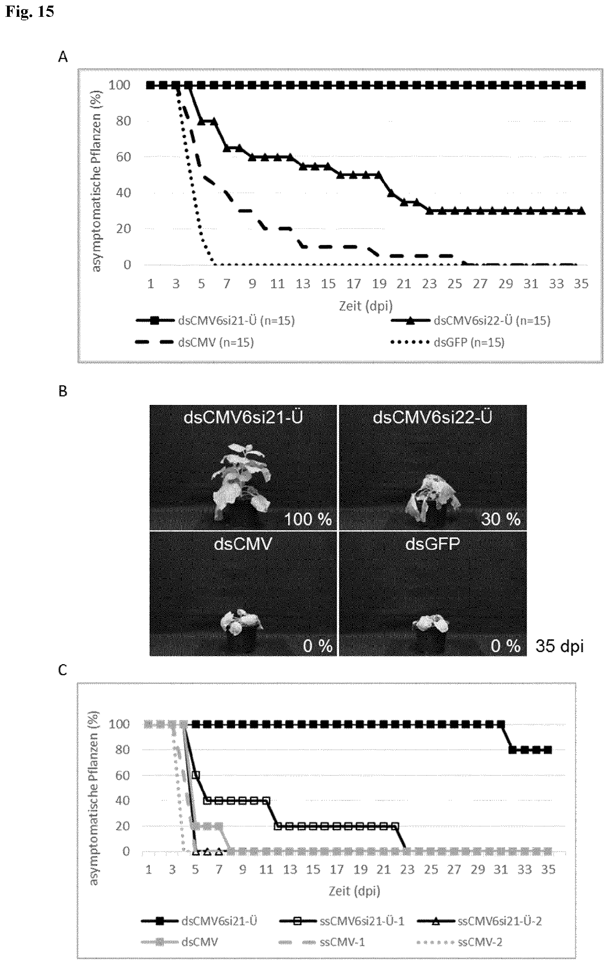

Die Funktionalität und gegenüber konventionellen dsRNAs deutlich verbesserte Protektivität derart neuartig aufgebauter edsRNAs gegen Pathogene wurde erfindungsgemäß demonstriert (siehe z.B. **Figuren 14** und **15****).**

Die Nukleinsäure gemäß der Erfindung weist in einer weiteren Ausführungsform eine oder mehrere chemische Modifikationen auf, wobei die chemischen Modifikationen ausgewählt sind aus z.B. Konjugaten wie GalNac, Basenmodifikationen wie 5-methylcytosin, 2'-Zuckermodifikationen wie 2'-O-methyl, 2'-fluoro, 2'-O-methoxyethyl (2'-MOE), cETBNA ((*S*)-gebundene ethylbicyclisch), andere Zuckermodifikationen wie *"locked"* (LNA) oder *"unlocked"* (UNA), "*Bockbone*"-Veränderungen wie Phosphorothioate (PS) oder *"peptide nucleic acids"* (PNA) sowie Zuckerphosphat-Modifikationen wie Morpholino/PMO *(phosphorodiamidate morpholino).*

In einem weiteren Aspekt betrifft die Erfindung eine Zusammensetzung enthaltend mindestens eine, ggf. mehrere Nukleinsäure(n) der Erfindung, wie hierin beschrieben.

Die Nukleinsäuren gemäß der vorliegenden Erfindung können in transgener Form, z.B. HIGS-Verfahren, zur Pathogenbekämpfung oder zur gezielten, transkriptionellen und post-transkriptionellen Regulation der Genexpression eingesetzt werden. Sie eignen sich besonders zur Verwendung in der Pathogenbekämpfung bei Pflanzen/Nutzpflanzen. In einer Ausführungsform betrifft die Erfindung daher die Verwendung einer Nukleinsäure oder Zusammensetzung gemäß der Erfindung bei Pflanzen zur Prophylaxe und/oder Behandlung gegen Befall und/oder Infektionen durch Pathogene, insbesondere zur Prophylaxe und/oder Behandlung gegen Befall und/oder Infektionen durch Pathogene, ausgewählt aus CMV, *Meloidogyne incognita* und *Botrytis cinerea.*

Transgen bedeutet, dass die genetische Information die für mindestens eine, ggf. mehrere der Nukleinsäure(n) der Erfindung kodiert, in das Genom eines Wirtsorganismus, vorzugsweise einer Pflanze, eines Mikroorganismus oder eines der genannten Pathogene, eingebracht wird und über entsprechende Promotoren induzierbar oder nicht induzierbar in diesem Organismus hergestellt (exprimiert) wird. Ist der Organismus eine Pflanze, kann diese so gegen Befall und/oder Infektionen eines oder mehrerer der entsprechenden Pathogene resistent gemacht werden. Ist dieser Organismus ein Mikroorganismus wie z.B. Bakterien oder Hefe kann die entsprechende Nukleinsäure in diesen Mikroorganismen produziert werden.

Die Nukleinsäuren gemäß der vorliegenden Erfindung können auch in transienter (non-transforming) Form, z.B. SIGS-Verfahren, zur Pathogenbekämpfung oder zur gezielten, transkriptionellen und post-transkriptionellen Regulation der Genexpression eingesetzt werden. Sie eignen sich besonders zur Verwendung in der Pathogenbekämpfung in Pflanzen/Nutzpflanzen. In einer Ausführungsform betrifft die Erfindung daher die Verwendung einer Nukleinsäure oder Zusammensetzung gemäß der Erfindung bei Pflanzen zur Prophylaxe und/oder Behandlung gegen Befall und/oder Infektionen durch Pathogene, insbesondere zur Prophylaxe und/oder Behandlung gegen Befall und/oder Infektionen durch Pathogene, ausgewählt aus CMV, *Meloidogyne incognita* und *Botrytis cinerea.*

Bei transienten Anwendungen sog. *topical*/*transient applications* werden eine oder mehrere Nukleinsäuren der Erfindung auf Zielorgane einer Pflanze wie z.B. Blätter, Stengel oder Wurzeln aufgebracht. Die Nukleinsäuren werden dann durch die Pflanze entweder aufgenommen, um z.B. die Replikation infizierender Viren zu inhibieren, oder aber attackierende (befallende) Pathogene wie Nematoden und Pilze, nehmen die Nukleinsäuren über die Oberfläche der zu schützenden Pflanze auf, und diese werden dann im RNA *silencing*/RNAi- oder *Antisense*-Mechanismus der jeweiligen Organismen gegen essenzielle *target-RNAs* der Pathogene aktiv.

In einer Ausführungsform betrifft die Erfindung entsprechend eine Zusammensetzung, umfassend mindestens eine Nukleinsäure der Erfindung und fakultativ ein oder mehrere Trägersubstanzen und /oder Hilfsstoffe, die zur Verabreichung in/auf Pflanzen geeignet ist.

Die Zusammensetzung ist vorzugsweise eine Lösung, die in direkter Form z.B. als Nährlösung oder als Aerosol/Spray, verabreicht werden kann. Damit lassen sich besonders einfach Erkrankungen an Pflanzen/Nutzpflanzen vorbeugen oder behandeln.

Vorzugsweise umfasst die Zusammensetzung wenigstens einen physiologisch verträglichen Träger, Verdünnungsmittel, und/oder Hilfsstoff. Die Nukleinsäuren gemäß der vorliegenden Erfindung können in einem pharmazeutisch verträglichen Träger, z.B. in einem herkömmlichen Medium, wie einem wässrigen Salzmedium oder einer Pufferlösung als Zusammensetzung für ein Aerosol/Spray enthalten sein. Ein solches Medium kann auch herkömmliche Hilfsstoffe enthalten, wie zum Beispiel Salze zur Einstellung des osmotischen Drucks, Puffer, Konservierungsmittel, Nanopartikel und dergleichen.

Weitere geeignete verträgliche Trägerstoffe sind dem Fachmann beispielsweise aus Remington's Practice of Pharmacy, 13. Ausgabe und J. of. Pharmaceutical Science & Technology, Vol. 52, Nr. 5, Sept-Okt., S. 238-311, bekannt.

In einer besonders bevorzugten Ausführungsform der Erfindung erfolgt die Verwendung der Nukleinsäuren der Erfindung in transgener oder transienter Form in RNA *silencing*/RNAi-Verfahren, wobei die Nukleinsäuren der Erfindung in Form von Doppelstrang-RNA-Molekülen gemäß Gruppe d. erfolgt, dadurch gekennzeichnet, dass diese entsprechend Ansprüchen 8 bis 12 aufgebaut sind und durch Dicer bzw. Dicer-like Enzyme zu den betreffenden sRNAs prozessiert werden. Eine derartige Verwendung ist in Anwendungsbeispiel 2 beschrieben.

**Die aufgeführten Aufgaben wurden durch die nachfolgend beschriebene Erfindung und die beschriebenen Anwendungsbeispiele gelöst.**

Es wurden Nukleinsäurewirkstoffe, *e*siRNAs/*E*RNAs und davon abgeleitete sRNAs und *e*ASO (*e*NAs), neu identifiziert, die in verschiedenen Anwendungen (RNA *silencing*/RNAi- bzw. *Antisense*-Verfahren) als Wirkstoffe im Pflanzenschutz gegen die Pathogene Cucumber Mosaic Virus bzw. *Meloidogyne incognita* bzw. *Botrytis cinerea* eingesetzt werden können.

Die Erfindung betrifft ferner die Konstruktion von doppelsträngigen Ribonukleinsäuren (edsRNAs; effektiv wirksame doppelsträngige RNAs), die aus identifizierten *e*siRNAs/*E*RNAs und/oder davon abgeleiteten sRNAs aufgebaut sind und die im RNA *silencing*/RNAi-Verfahren als Wirkstoffe gegen genannte Pathogene im Pflanzenschutz eingesetzt werden können.

**Die Erfindung wird nachfolgend anhand von 19** **Figuren, 8** **Tabellen und 4 Anwendungsbeispielen näher erläutert.**

Es zeigen:
**Figur 1****: *Slicer*-Assay mit esiRNAs/ERNAs die gegen CMV-RNA 2 identifiziert wurden.** AGO1- bzw. AGO2-mRNA wurde in Gegenwart der zu testenden siRNA in BYL (Gago et al., 2019) translatiert. Wichtig anzumerken ist, dass hier, wie auch in allen folgenden *Slicer*-Assays alle getesteten siRNAs gegen die (+)-orientierte Ziel-RNA gerichtet waren. Die entsprechend dem beschriebenen Verfahren gebildeten RISC wurden so mit der zu charakterisierenden siRNA programmiert und die endonukleolytische Hydrolyse radioaktiv markierter CMV-RNA 2 mittels Gelelektrophorese und Autoradiographie detektiert. Sternchen (^{∗}) markieren die durch *slicer*-Aktivität des AGO/RISC generierten Spaltprodukte. (A) SiRNA-Kandidaten aus *screening* mit einer doppelsträngigen Version von CMV-RNA 2, getestet mit AGO1/RISC. (B) SiRNA-Kandidaten aus *screening* mit AGO2/RISC. (C) *Slicer*-Assay unter kompetitiven Bedingungen: Dabei wurde die Translation der AGO-mRNA in Gegenwart von 10 nM der zu charakterisierenden siRNA sowie 0,1 µM (10 × Überschuss) einer Kompetitor-siRNA ("siR gf698") durchgeführt. Aus dem *screening* identifizierte siRNAs, die in Kombination mit dem jeweiligen AGO-Protein eine besonders effiziente Spaltung (*slicer*-Aktivität) der *target-RNA* bewirken, sind fett markiert. (D) Schematische Darstellung der Bindestellen der effizientesten siRNAs auf CMV-RNA 2. Proteinkodierende Regionen sind als graue Kästen dargestellt. Die Nummern der siRNA-Kandidaten entsprechen den im Folgenden in den Figuren, Tabellen und im Text gegebenen Bezeichnungen (mit Abkürzung siR oder siRCV2).
**Figur 2****: Charakterisierung der Protektionseffizienz CMV-RNA 2-spezifischer esiRNAs/ERNAs *in planta.*** Vier bis fünf Wochen alte *N*. *benthamiana-*Pflanzen wurden mit den genomischen CMV-RNAs 1, 2 und 3, die von infektiösen cDNAs generiert wurden, sowie individuellen, synthetischen siRNA-Kandidaten mechanisch inokuliert und über eine Dauer von 35 Tagen auf das Auftreten CMV-spezifischer Symptome überprüft (dpi = *days post inoculation*). (A) Die repräsentativen Bilder einzelner Individuen veranschaulichen die Unterschiede zwischen asymptomatischen und symptomatischen Pflanzen 35 Tage nach der Inokulation. Angegeben ist die Zahl asymptomatisch verbliebener Pflanzen. (B) Der Graph zeigt den prozentualen Anteil asymptomatischer Pflanzen über den Gesamtverlauf des Experiments: Die Verläufe mit AGO1-spezifischen siRNAs sind in schwarz, mit AGO2-spezifischen siRNAs sowie der siR gf698-Kontrolle in grau dargestellt. Die Ergebnisse stammen aus drei unabhängigen Pflanzenexperimenten. Die Anzahl der verwendeten Pflanzen ist angegeben (n = 12-15). Wie gezeigt, schützen einige *e*siRNA/*E*RNAs die Pflanzen sehr effizient vor einer CMV Infektion. (C) Dargestellt sind Agarose-Gele einer RT-PCR zum Nachweis der viralen Infektion auf RNA-Ebene. Blattscheiben asymptomatischer sowie symptomatischer Pflanzen wurden zum Zeitpunkt 35 dpi entnommen, die RNA extrahiert und cDNA mit Reverser Transkriptase synthetisiert. Anschließend erfolgte eine PCR zum Nachweis einer konservierten Sequenz, die in RNAs 1, 2 sowie 3 des *Cucumber mosaic virus* vorhanden ist. Die Amplifikation der CMV-spezifischen Sequenz erfolgte von Plasmiden, die die cDNA-Sequenz der jeweiligen viralen RNA enthalten (Positivkontrolle) sowie in Proben der symptomatischen Pflanzen. Wie ersichtlich ist, konnte aus cDNA asymptomatischer Pflanzen kein PCR-Produkt abgeleitet von CMV-RNA erhalten werden. Die RT-PCR bestätigt somit die auf visueller Basis getroffene Klassifizierung in symptomatische und asymptomatische Pflanzen. Die Ziffern entsprechen der Nummerierung der jeweiligen Pflanzen aus dem Infektionsexperiment. (-) RT-Assays (keine Zugabe der Reversen Transkriptase während der Reaktion) sowie die "Wasserkontrolle" (Zugabe von Wasser statt cDNA während der PCR-Reaktion) dienten als Negativkontrollen. (M = *GeneRuler* 100 bp DNA-Leiter, Thermo Scientific). Die Nummern der siRNA-Kandidaten entsprechen den zuvor und im Folgenden in den Figuren, Tabellen und im Text gegebenen Bezeichnungen (mit Abkürzung siR oder siRCV2).
   **Tabelle 1. esiRNA/ERNA Kandidaten gegen CMV-RNA 2 (SEQ ID NO: 189).** Aufgelistet sind im Rahmen des *screenings* aus CMV-RNA 2 identifizierte 21 nt lange siRNAs. Die siRNAs wurden als siRCV (CV für CMV) entsprechend der jeweiligen *target-RNA* (CMV-RNA 2) und der Position des 5'-Endes des identifizierten *guide strands* benannt. Darunter befinden sich siRNAs (*e*siRNAs/*E*RNAs), die eine sehr hohe oder hohe *slicer*-Aktivität in Kombination mit AGO1 bzw. AGO2 vermitteln (z.B. siRCV2 359, 380, 1020, 1172, 1489, 2041) sowie siRNAs, die keine Spaltung der *target*-RNA vermitteln konnten und zum Teil als Negativkontrollen für Infektionsexperimente in *N*. *benthamiana* dienten (z.B. siRCV2 1844 und 2634). Die Effizienz in Protektionsexperimenten mit *N*. *benthamiana* ist für *in planta* getestete siRNAs angegeben (n.a. - nicht angegeben): Angegeben ist der Anteil asymptomatischer Pflanzen 35 Tage nach Infektion. Fett markiert schwarz (AGO1 selektiert) bzw. fett markiert grau (AGO2 selektiert) sind solche RNAs, für die und Varianten hiervon (siehe **Tabelle 6)** entsprechend den Ansprüchen und eingesetzt in Form von *e*siRNAs/*E*RNAs oder davon abgeleiteter sRNAs oder *e*ASO (*e*NAs) gegen CMV, bevorzugt ein Patentschutz beantragt wird. Die jeweiligen SEQ ID NO sind als **"Zusatz zu Tabelle 1"** extra gelistet.

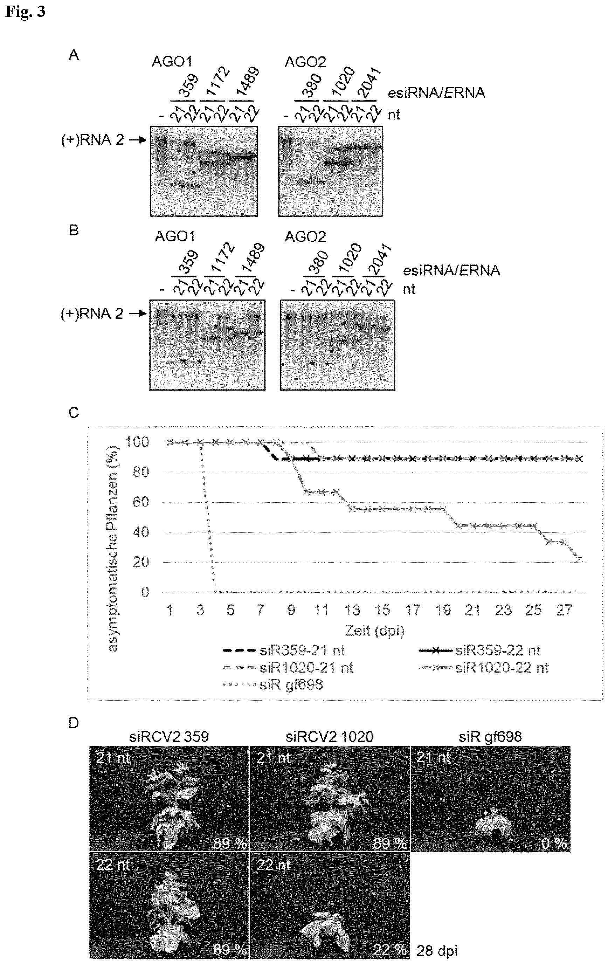

**Figur 3****. Wirksamkeit 21 nt und 22 nt langer esiRNAs/ERNAs gegen CMV-RNA 2 *in vitro* und *in planta.*** (A) *Slicer*-Assay unter Verwendung von Beispielen 21 nt langer *e*siRNA/*E*RNAs sowie davon abgeleiteter 22 nt langer Varianten. Wie gezeigt haben beide Varianten eine hohe *slicer*-Aktivität. (B) Vergleich der siRNAs anhand eines kompetitiven (siehe **Figur 1****)** *Slicer*-Assays in Gegenwart der unspezifischen siR gf698. Vor allem AGO1 aber auch AGO2 weisen in Kombination mit 21 nt langen *e*siRNAs/*E*RNAs eine leicht höhere *slicer*-Aktivität als mit 22 nt langen *e*siRNAs/*E*RNAs auf. Dagegen unterscheidet sich die *slicer*-Aktivität von AGO2 in Kombination mit 21 nt bzw. 22 nt langen *e*siRNAs/*E*RNAs nur minimal. Sternchen (^{∗}) markieren die durch *slicer*-Aktivität des AGO/RISC generierten Spaltprodukte. (C) Vergleich der protektiven Wirkung 21 nt und 22 nt langer siRNAs (Beispiele) *in planta.* Vier bis fünf Wochen alte *N*. *benthamiana-*Pflanzen wurden mit 21 nt bzw. 22 nt langen synthetischen *e*siRNAs/*E*RNAs sowie den genomischen CMV-RNAs mechanisch ko-inokuliert. Dargestellt ist der prozentuale Anteil asymptomatischer Pflanzen (dpi = *days post inoculation*). Beide *e*siRNA/ERNA Versionen haben einen deutlichen antiviralen Effekt. (D) Repräsentative Pflanzenbilder 28 Tage nach der mechanischen Ko-Inokulation. Der prozentuale Anteil asymptomatischer Pflanzen pro siRNA ist angegeben. Es wurden jeweils neun Pflanzen für die CMV-spezifischen siRNAs und drei Pflanzen für die siR gf698-Kontrolle eingesetzt. Alle weiteren Kontrollen wurden ähnlich durchgeführt wie in **Figur 2****.** Die Nummern der siRNA-Kandidaten entsprechen den zuvor und im Folgenden in den Figuren, Tabellen und im Text gegebenen Bezeichnungen (mit Abkürzung siR bzw. siRCV).
**Figur 4****. *Slicer*-Assay mit esiRNAs/ERNAs, die gegen CMV-RNA 3 identifiziert wurden.** AGO1- bzw. AGO2-mRNA wurde in Gegenwart der zu testenden siRNA im BYL translatiert. Die jeweiligen RISC wurden so mit der zu charakterisierenden siRNA programmiert und die endonukleolytische Hydrolyse der radioaktiv markierten CMV *target-RNA* 3 mittels Agarose-Gelelektrophorese und Autoradiographie detektiert. Sternchen (^{∗}) markieren die durch *slicer*-Aktivität des AGO/RISC generierten Spaltprodukte. (A) SiRNA-Kandidaten aus NGS-*screening* mit AGO1. (B) SiRNA-Kandidaten aus NGS-*screening* mit AGO2. (C) *Slicer*-Assay mit den indizierten siRNAs unter kompetitiven Bedingungen (siehe **Figur 1****).** SiRNAs (*e*siRNAs/*E*RNAs), die in Kombination mit dem jeweiligen AGO-Protein eine besonders effiziente Spaltung des *targets* bewirkten, sind fett markiert. (D) Schematische Darstellung der Bindestellen der effizientesten *e*siRNAs/*E*RNAs auf CMV-RNA 3. Proteinkodierende Regionen sind als graue Kästen dargestellt. Die Nummern der siRNA-Kandidaten entsprechen den zuvor und im Folgenden in den Figuren, Tabellen und im Text gegebenen Bezeichnungen (mit Abkürzung siR oder siRCV).
   **Tabelle 2. esiRNA/ERNA-Kandidaten gegen CMV-RNA 3 (SEQ ID NO: 190).** Aufgelistet sind im Rahmen des *screenings* aus CMV-RNA 3 identifizierte 21 nt lange siRNAs. Die siRNAs wurden als siRCV entsprechend der jeweiligen *target-RNA* (CMV-RNA 3) und der Position des 5'-Endes des identifizierten *guide strands* benannt Darunter befinden sich sowohl siRNAs, die eine sehr hohe oder hohe *slicer-*Aktivität in AGO1/RISC bzw. AGO2/RISC vermitteln (z.B. siRCV3 239, 507, 985, 593, 1019, 1569), sowie siRNAs, die keine Spaltung der *target-RNA* vermitteln und zum Teil als Negativkontrollen für Infektionsexperimente in *N*. *benthamiana* dienten (z.B. siRCV3 2061, 1132). Die Effizienz in Protektionsexperimenten mit *N*. *benthamiana* ist für die *in planta* getesteten siRNAs angegeben (n.a. - nicht angegeben). Angegeben ist der prozentuale Anteil asymptomatischer Pflanzen 28 Tage nach der Infektion. Fett markiert schwarz (AGO1 selektiert) bzw. fett markiert grau (AGO2 selektiert) sind solche RNAs, für die und Varianten hiervon (siehe **Tabelle 6)** entsprechend den Ansprüchen und eingesetzt in Form von *e*siRNAs/*E*RNAs oder davon abgeleiteter sRNAs oder *e*ASO (eNAs) gegen CMV, bevorzugt ein Patentschutz beantragt wird. Die jeweiligen SEQ ID NO sind als **"Zusatz zu Tabelle 2"** extra gelistet.

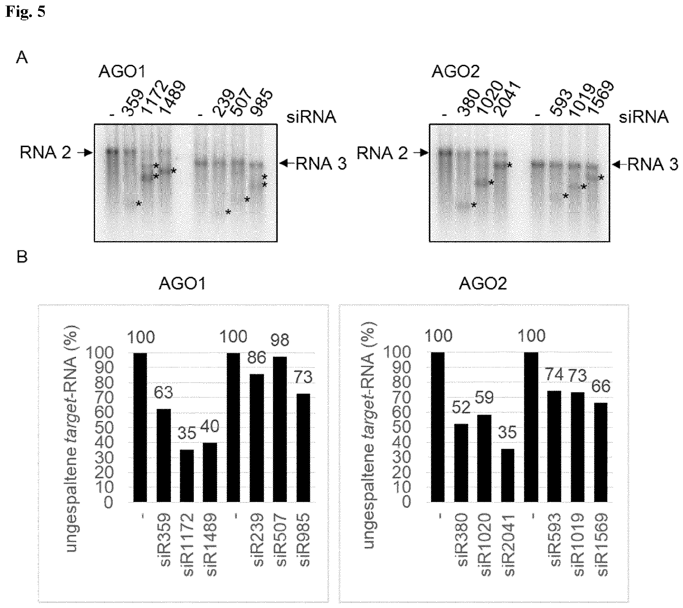

**Figur 5****. Vergleich der *slicer*-Aktivitäten von esiRNAs/ERNAs gegen CMV-RNAs 2 und 3.** (A) *Slicer-*Assay mit jeweils drei exemplarisch ausgewählten *e*siRNAs/*E*RNAs gegen CMV-RNA 2 und CMV-RNA 3. Sternchen (^{∗}) markieren die durch AGO/RISC generierten Spaltprodukte. (B) Quantifizierung der *Slicer-*Assays mittels ImageOuantTL. Die gegen CMV-RNA 2 gerichteten *e*siRNAs/*E*RNAs vermitteln eine tendenziell effizientere *slicer*-Aktivität der jeweiligen AGO/RISC; dies geht aus dem niedrigeren prozentualen Anteil ungespaltener *target-RNA* hervor. Die Nummern der siRNA-Kandidaten entsprechen den zuvor und im Folgenden in den Figuren, Tabellen und im Text gegebenen Bezeichnungen (mit Abkürzung siR bzw. siRCV).
**Figur 6****. Charakterisierung der Protektionseffizienz von esiRNAs/ERNAs** ***in planta**,* **die gegen CMV-RNA 3 gerichtet sind.** Vier bis fünf Wochen alte *N*. *benthamiana-*Pflanzen wurden mit den genomischen CMV-RNAs sowie individuellen, synthetischen *e*siRNAs/*E*RNAs mechanisch inokuliert (siehe **Figur 2****)** und über eine Dauer von 28 Tagen auf das Auftreten CMV-spezifischer Symptome überprüft (dpi = *days post inoculation*). (A) Der Graph zeigt den prozentualen Anteil asymptomatischer Pflanzen über die Dauer des Experiments. Verläufe mit AGO1-spezifischen *e*siRNAs/*E*RNAs sind in schwarz, mit AGO2-spezifischen *e*siRNAs/*E*RNAs sowie der siR gf698-Kontrolle in grau dargestellt. (B) Die repräsentativen Pflanzenbilder veranschaulichen die Unterschiede zwischen asymptomatischen und symptomatischen Pflanzen 28 Tage nach der Inokulation. Die Ergebnisse stammen aus drei unabhängigen Pflanzenexperimenten. Der prozentuale Anteil asymptomatischer Pflanzen sowie die Anzahl der verwendeten Pflanzen ist angegeben (n = 9-15). Alle weiteren Kontrollen wurden ähnlich durchgeführt wie in **Figur 2** angegeben. Die Nummern der siRNA-Kandidaten entsprechen den zuvor und im Folgenden in den Figuren, Tabellen und im Text gegebenen Bezeichnungen (mit Abkürzung siR bzw. siRCV).
**Figur 7****. Vergleich der *target-sites* CMV(Fny)-spezifischer esiRNAs/ERNAs in RNA 2 mit den entsprechenden potenziellen *target-sites* dieser esiRNAs/ERNAs in den RNA 2 Molekülen anderer CMV-Stämme.** (A) Das Schema ("*phylogenetic tree"*) fasst exemplarische CMV-Stämme und ihre Zugehörigkeit zu bestimmten Untergruppen (II, IB, IA) zusammen. Das zum *screening* verwendete Fny, Untergruppe IA, ist mit einem Pfeil markiert (modifiziert nach Roossinck, 1999). (B, C) Die Übersichten zeigen exemplarische AGO1- und AGO2-spezifische *e*siRNAs/*E*RNAs aus CMV-RNA 2 und deren Sequenzübereinstimmung mit verschiedenen CMV-Stämmen. Der *"expectation"*-Wert ist ein Maß für die *mismatches* zwischen siRNA *guide strand* und der komplementären *target-site.* Je höher der Wert, desto geringer ist die Komplementarität zwischen siRNA und *target-site* in der CMV-RNA 2 des jeweiligen CMV-Stammes (https://www.zhaolab.org/psRNATarget/help#maxexpectation).
   **Tabelle 3. CMV(Fny)-spezifische esiRNAs/ERNAs, die gegen RNAs 2 und 3 gerichtet sind und einen kompletten *match* (komplette Übereinstimmung der entsprechenden *target-sites*) auf den RNAs 2 und 3 anderer CMV-Stämme haben.** (A) Die Tabelle listet *e*siRNAs/*E*RNAs auf (schwarz aus AGO1 *screening*; grau aus AGO2 *screening*), die aus CMV(Fny) identifiziert wurden und deren *target- sites* vollständig mit potenziellen *target-sites* ausgewählter CMV-Stämme der Untergruppe IA übereinstimmen. Die Nummern entsprechen den jeweiligen siR bzw. siRCV in den vorangegangenen Figuren und Tabellen. (B) Die Tabelle listet *e*siRNAs/*E*RNAs auf (Markierung wie oben), die aus CMV(Fny) identifiziert wurden und deren *target-sites* vollständig mit potenziellen *target-sites* ausgewählter CMV-Stämme der Untergruppe IB übereinstimmen. Die aus CMV(Fny) identifizierten *e*siRNAs/*E*RNAs hatten keine vollständige Übereinstimmung (*match*) mit potenziellen *target-sites* in den RNAs 2 und 3 ausgewählter CMV-Stämme der Untergruppe II (siehe **Tabelle 6**). Die Nummern der siRNA-Kandidaten entsprechen den zuvor und im Folgenden in den Figuren, Tabellen und im Text gegebenen Bezeichnungen (mit Abkürzung siR bzw. siRCV). Die Angaben sind analog für andere sRNAs und *e*ASO, deren Sequenz von diesen *e*siRNAs/*E*RNAs abgeleitet ist.
   **Tabelle 4. Protektionspotenzial.** Die mit einem (+) markierten *e*siRNAs/*E*RNAs besitzen vollständig komplementäre *target-sites* in den genomischen RNA-Molekülen ausgewählter CMV-Stämme. Ein besonders breiter Schutz vor einer CMV-Infektion kann durch den Einsatz der grau hinterlegten und mit einem (+) markierten aus CMV-RNA 2 identifizierten *e*siRNAs/*E*RNAs erzielt werden, da diese vollständig komplementäre *target*-sites in jeweils fünf bis sechs der zum Vergleich ausgewählten CMV-Stämme besitzen. Mit einer Kombination verschiedener *e*siRNAs/*E*RNAs kann somit gegen alle hier betrachteten CMV-Stämme der Untergruppen IA sowie IB (und wahrscheinlich noch weitere Vertreter dieser Untergruppen) in Protektionsexperimenten geschützt werden. Durch entsprechende Variation der *e*siRNAs/ERNAs, d.h. Austausch einer oder mehrerer indizierter Nukleotide (siehe **Tabelle 6)** können die identifizierten *e*siRNAs/*E*RNAs ebenfalls gegen Stämme der Untergruppe II eingesetzt werden. Die Angaben sind analog für andere sRNAs und *e*ASO, deren Sequenz von diesen *e*siRNAs/*E*RNAs abgeleitet ist.
   **Tabelle 5. Protektionspotenzial.** Die mit einem (+) markierten siRNAs besitzen vollständig komplementäre *target*-sites in den ausgewählten CMV-Stämmen. Ein besonders breiter Schutz vor einer CMV-Infektion kann durch den Einsatz der grau hinterlegten und mit einem (+) markierten aus CMV-RNA 3 identifizierten *e*siRNAs/*E*RNAs erzielt werden, da diese vollständig komplementäre *target-sites* in jeweils fünf bis sechs der zum Vergleich ausgewählten CMV-Stämme besitzen. Mit einer Kombination verschiedener *e*siRNAs/*E*RNAs kann somit gegen alle hier betrachteten CMV-Stämme der Untergruppen IA sowie IB (und weitere Vertreter dieser Untergruppen) in Protektionsexperimenten geschützt werden. Durch entsprechende Variationen der *e*siRNAs/ERNAs, d.h. Austausch einer oder mehrerer indizierter Nukleotide (siehe **Tabelle 6),** können die identifizierten *e*siRNAs/*E*RNAs ebenfalls gegen Stämme der Untergruppe II eingesetzt werden. Die Angaben sind analog für andere sRNAs und *e*ASO, deren Sequenz von diesen *e*siRNAs/*E*RNAs abgeleitet ist.
   **Tabelle 6. Übereinstimmung der *target-sites* CMV(Fny)-spezifischer *e*siRNAs/*E*RNAs mit potenziellen *target-sites* dieser esiRNAs/ERNAs in den genomischen RNAs anderer CMV-Stämme.** (A) Die Tabelle zeigt *e*siRNAs/*E*RNAs (die Nummern entsprechen den jeweiligen siR bzw. siRCV in den vorangegangen Figuren und Tabellen), die aus CMV(Fny) identifiziert wurden und deren *target-sites* teilweise mit potenziellen *target-sites* ausgewählter CMV-Stämme der Untergruppe IA übereinstimmen. Die Anzahl jeweiliger *mismatches* ist in Klammern angegeben. *e*siRNAs/ERNAs, bei denen sich das *mismatch* am 5'-Nukleotid des *guide strand* befindet, sind kursiv geschrieben, *e*siRNAs/*E*RNAs mit mindestens einem *mismatch* innerhalb der sog. *seed*-Sequenz sind fett dargestellt. (B) Die Tabelle zeigt *e*siRNAs/ERNAs, die aus CMV(Fny) identifiziert wurden und deren *target-sites* teilweise mit potenziellen *target-sites* ausgewählter CMV-Stämme der Untergruppen IB und II übereinstimmen. Die Nummern der Kandidaten entsprechen den zuvor und im Folgenden in den Figuren, Tabellen und im Text gegebenen Bezeichnungen (mit Abkürzung siR bzw. siRCV). Die Angaben sind analog für andere sRNAs und ***e*ASO,** deren Sequenz von diesen *e*siRNAs/*E*RNAs abgeleitet ist.

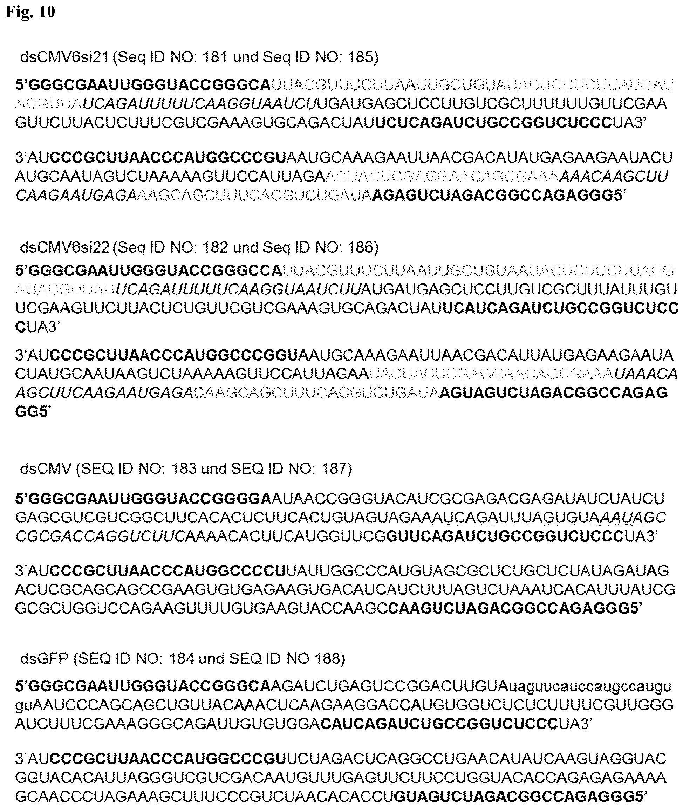

**Figur 8****: Schematische Darstellung der Organisationsformen von edsRNAs.** edsRNAs werden entweder durch Hybridisierung zweier separater komplementärer RNA-Moleküle (a) oder durch Hybridisierung komplementärer Bereiche eines RNA-Moleküls (b) erzeugt. Im Falle von (b) enthält das RNA-Molekül einen *spacer* (Definition siehe Text). Dieser *spacer* kann durch unterschiedliche Mechanismen (z.B. *splicing* oder Endonuklease-Aktivitäten) verkleinert oder komplett entfernt (prozessiert) werden, wodurch andere Formen von RNA *hairpin*-Molekülen oder ähnlich aufgebaute dsRNAs wie im Fall (a) entstehen können. edsRNAs, wie unter (a) werden im einfachsten Fall durch Hybrisierung unabhängig voneinander erzeugter komplementärer RNA-Moleküle gewonnen (siehe auch Anwendungsbeispiele und **Figuren 9** und **10****).** Schwarze und graue Kästen: pseudo (p)-siRNAs (Definition siehe Text) mit variabler Sequenz am 5' - bzw. 3'-Ende; (s) sense, (as) antisense. A, B....Y...Z stehen für 1 bis n (bzw. in umgekehrter Reihenfolge n-1) *e*siRNA/*E*RNA-Sequenzen, die aus einem screen mit einem AGO-Protein (A), oder anderen AGO-Proteinen (B..., Y, Z) identifiziert und in die edsRNA-Sequenz aufgenommen wurden; (s) sense, (as) antisense. R: Hammerhead- oder Hepatitis delta virus (HDV)-Ribozym. Die Ribozyme wurden zur Generierung der Transkriptenden über *"self-splicing"* verwendet. Sie sind nach der Generierung der jeweiligen edsRNA nicht mehr funktional vorhanden. Beispiele für edsRNAs, die entsprechend a) aufgebaut sind, sind in **Figuren 9** und **10** gegeben. Beispiele für die Komposition von cDNA-Konstrukten, mit denen nach a) und/oder b) konzipierte edsRNAs generiert werden können, sind in **Figur 11** gegeben.
**Figur 9****. Exemplarische edsRNA und Kontroll-dsRNAs.** Gezeigt ist der Aufbau einer edsRNA, die entsprechend des Schemas aus **Figur 8** a) aus zwei Transkripten generiert wurde. Die edsRNA enthält 21 nt lange *e*siRNA/ERNA Sequenzen, für die gezeigt wurde, dass sie gegen die CMV-RNA 2 im RNA-silencing-Prozess wirksam und *in planta* antiviral protektiv sind (Nummerierung entsprechend **Figuren 2****,** **3** und **Tabelle 1).** Zudem sind zwei dsRNAs gezeigt, die als Kontrollen eingesetzt wurden. (A) Exemplarische edsRNA ,dsCMV6si21'. Diese besteht aus je einer 21 nt langen pseudo-siRNA an beiden Enden (durch Sternchen (^{∗}) symbolisiert) sowie sechs 21 nt langen Sequenzen gegen die CMV-RNA 2 gerichteter ***e*siRNAs/ERNAs,** die in pflanzlichen AGO1/RISCs oder AGO2/RISCs aktiv sind. *Guide strands* (gs) sind jeweils als Pfeile dargestellt, die in sense-Richtung 5'-3' weisen. Die AGO1-spezifischen gs befinden sich auf dem einen, die AGO2-spezifischen gs auf dem anderen RNA-Strang. Die hier gezeigte Beispiel-RNA ist *blunt,* d.h. es stehen keine Nukleotide an den Enden über. Es wurden aber auch edsRNAs mit überstehenden Enden (Ü) generiert und getestet (siehe unten). Bezüglich ihrer Protektionseffizienz waren die erzielten Ergebnisse mit beiden Formen der edsRNAs identisch. Analog war die Protektionseffizienz auch mit edsRNAs, die nach dem Schema der **Figur 8** b) oder **Figur 11** aus einem Transkript generiert wurden (nicht gezeigt). (B) Kontroll-dsRNA 1. Die dsCMV besteht ebenfalls aus pseudo-siRNA-Sequenzen an den Enden sowie einem doppelsträngigen 126 nt langen Teilbereich (entsprechend einer Länge von sechs 21 nt langen siRNAs) aus CMV-RNA 2 und dem Teilbereich der CMV-RNA 2 entsprechender komplementärer Sequenz. In der dsCMV sind durch Zufall auch die Sequenzen von zwei siRNAs enthalten, die im *screening* gegen CMV-RNA 2 (s.o.) als *e*siRNAs/*E*RNAs identifiziert wurden (siehe **Figur 10****).** (C) Kontroll-dsRNA 2. Die dsGFP besteht ebenfalls aus pseudo-siRNA-Sequenzen an den Enden und einem 126 nt langen doppelsträngigen Teilbereich aus der *GFP-*mRNA (mRNA kodierend für das *green fluorescent protein)* und diesem Teilbereich der *GFP*-mRNA entsprechender komplementärer Sequenz. Die exakten Sequenzen der gezeigten dsRNAs sind in **Figur 10** gezeigt.
**Figur 10****. Aufbau/Sequenzen von exemplarischen edsRNAs und der verwendeten Kontroll-dsRNAs (schematisch gezeigt in** **Figur 9****).** Gezeigt sind die Sequenzen zweier edsRNAs sowie der in **Figur 9** gezeigten Kontroll-dsRNAs (jeweils gezeigt sind der sense und der antisense-Strang). Die edsRNAs enthalten 21 nt oder 22 nt lange *e*siRNA/*E*RNA-Sequenzen, für die gezeigt wurde, dass sie gegen die CMV-RNA 2 im RNA-*silencing*-Prozess wirksam und *in planta* antiviral protektiv sind. dsCMV6si21 besteht aus je einer 21 nt langen pseudo-siRNA an beiden Enden sowie sechs 21 nt langen CMV-spezifischen *e*siRNAs/*E*RNAs (je drei aktiv in AGO1/RISC bzw. AGO2/RISC). Die AGO1-spezifischen *guide strands (gs)* befinden sich auf dem einen, die AGO2-spezifischen gs auf dem anderen RNA-Strang. dsCMV6si22 besteht aus je einer 22 nt langen pseudo-siRNA an beiden Enden sowie sechs 22 nt langen CMV-spezifischen *e*siRNAs/*E*RNAs (je drei aktiv in AGO1/RISC bzw. in AGO2/RISC). Die AGO1-spezifischen gs befinden sich auf dem einen, die AGO2-spezifischen gs auf dem anderen RNA-Strang.

Die verschiedenen Teilbereiche der RNA sind folgendermaßen kenntlich gemacht.
dsCMV6si21 bzw. 22 (5'-3'): fett schwarz-pseudo-siRNA, dunkelgrau-siRCV2 1172 gs, hellgrau-siRCV2 1489 gs, kursiv schwarz-siRCV2 359 *gs. gs* - *guide strand*
dsCMV6si21 bzw. 22 (3'-5'): fett schwarz-pseudo-siRNA, hellgrau-siRCV2 380 gs, kursiv schwarz-siRCV2 2041 gs, dunkelgrau-siRCV2 1020 *gs*

Die dsCMV besteht ebenfalls aus pseudo-siRNA-Sequenzen an den Enden sowie einem 126 nt langen doppelsträngigen Teilbereich (entsprechend einer Länge von sechs 21 nt langen siRNAs) aus der CMV-RNA 2. In dem aus der CMV-RNA 2 ausgewählten Teilbereich waren durch Zufall zwei siRNAs enthalten, die im screening (s.o.) als *e*siRNAs/*E*RNAs identifiziert wurden (siRCV2 557 und siRCV2 540: unterstrichen bzw. kursiv).

Die dsGFP besteht ebenfalls aus pseudo-siRNA-Sequenzen an den Enden und einem 126 nt langen doppelsträngigen Teilbereich der GFP-mRNA. Diese dsRNA enthält die Sequenz der in früheren Experimenten verwendeten Kontroll-siRNA siR gf698 (Kleinbuchstaben).

Die jeweiligen SEQ ID NO sind angegeben.

**Figur 11****. Exemplarischer Aufbau von cDNA-Konstrukten/Templaten, mit denen auf verschiedene Weise *in vitro* oder *in vivo* edsRNAs mit unterschiedlichem Aufbau generiert werden können.** Die gezeigten cDNAs enthalten unterschiedliche, beispielhafte Promotoren (Phage T7 RNA Polymerase-, Pol II- und Pol I-Promotoren (Pol II und Pol I-Promotoren beispielhaft aus *saccharomyces cerevisiae*)), über die edsRNAs von diesen cDNAs trankribiert werden können. Die generierten edsRNAs enthalten mindestens eine pseudo-siRNA-Sequenz und esiRNA-Sequenzen, die hier beispielhaft den esiRNA-Sequenzen entsprechen, die gegen CMV gerichtet identifiziert wurden (identisch zu den esiRNA-Sequenzen in den edsRNA Konstrukten gezeigt in **Figuren 9** und **10****).** Wie erwähnt sind die verwendeten esiRNA-Sequenzen beispielhaft, d.h. in entsprechend anderen, sonst analog aufgebauten cDNA-Konstrukten können völlig unterschiedliche pseudo-siRNA-Sequenzen oder esiRNA-Sequenzen enthalten sein (siehe **Figur 8** und Text). Die cDNAs kodieren zusätzlich zu den komplementären pseudo-siRNA- bzw. esiRNA Sequenzen noch eine *spacer*-Sequenz, die hier, wiederum beispielhaft, für ein zelluläres Intron (*Actin 1*) kodiert und durch die zelluläre Speißmaschinerie gespleißt werden oder durch zelluläre RNAasen abgebaut werden kann. Zusätzlich kodieren die cDNAs für Ribozyme (HH Ribozym bzw. HDV Ribozym), die nach Transkription einen Terminus oder beide Termini der edsRNA durch *self-splicing* generieren. Zudem kodieren sie für Transkriptionsterminatoren (hier beispielhaft VSV- oder zelluläre Pol-Terminatoren) und Restriktionsstellen zu Klonierungszwecken. Die jeweiligen Sequenzen, die sich z.T. auch überlappen können (siehe Text oben) sind in unterschiedlicher Form kenntlich gemacht. Die jeweiligen SEQ ID NO sind angegeben.

### edsRNAs generiert via T7-RNA Polymerase

**Konstrukt 1.1.** *e*dsRNA wird als *hairpin*-Transkript generiert. Konstrukt enthält: Promotor der T7-RNA Polymerase (T7 Promotor); *e*siRNA-Sequenzen sowie eine pseudo-siRNA-Sequenz, die einseitig terminal der *e*siRNA-Sequenzen lokalisiert ist; das *Actin-1* Intron als *spacer,* das Hepatitis D Virus (HDV) Ribozym; den Vesicular stomatitis Virus (VSV) Transkriptionsterminator; Restriktionsstellen (*S*pe I / *Xba* I) für die Klonierung.

**Konstrukt 1.2.** edsRNA wird als *hairpin*-Transkript generiert, das weiter prozessiert wird. Konstrukt enthält: T7 Promotor; esiRNA-Sequenzen sowie zwei pseudo-siRNA-Sequenzen, die zweiseitig terminal der esiRNA-Sequenzen lokalisiert sind; das *Actin-1* Intron als *spacer;* das HDV Ribozym; den VSV Transkriptionsterminator; Restriktionsstellen (*Spe* I / *Xba* I) für die Klonierung.

### edsRNAs generiert via RNA polymerase II (Pol II)

**Konstrukt 2.** edsRNA wird als *hairpin*-Transkript generiert, das weiter prozessiert wird. cDNA wird hinter zellulären Pol II Promotor kloniert, der potenziell induzierbar ist (z.B. Gall Promotor von *saccharomyces cerevisiae*) und die Termination erfolgt durch einen 3' -seitigen Pol II-Termintor (z.B. CYC1 Terminator aus *saccharomyces cerevisiae*)*.* Konstrukt enthält: Hammerhead Ribozym (HH Ribozym); esiRNA-Sequenzen sowie eine pseudo-siRNA-Sequenz, die einseitig terminal der esiRNA-Sequenzen lokalisiert ist; das *Actin-1* Intron als *spacer;* das Hepatitis D Virus (HDV) Ribozym; Restriktionsstellen (*Hind* III / *Xba* I) für die Klonierung.

### edsRNAs generiert via RNA polymerase I (Pol I)

**Konstrukt 3.1.** edsRNA wird als *hairpin*-Transkript generiert. Konstrukt enthält: Einen zellulären Pol I Promotor; esiRNA-Sequenzen sowie eine pseudo-siRNA-Sequenz, die einseitig terminal der esiRNA-Sequenzen lokalisiert ist; das *Actin-1* Intron als *spacer;* das Hepatitis D Virus (HDV) Ribozym; einen minimalen Pol I Terminator; Restriktionsstellen (*Spel* / *Xba* I) für die Klonierung

**Konstrukt 3.2.** edsRNA wird als *hairpin*-Transkript generiert, das weiter prozessiert wird. Konstrukt enthält: Pol I Promotor; esiRNA-Sequenzen sowie zwei pseudo-siRNA-Sequenzen, die 5'- und 3'-seitig der esiRNA-Sequenzen lokalisiert sind; das *Actin-1* Intron als *spacer;* das HDV Ribozym; den Pol I-Terminator; Restriktionsstellen (*Spe* I / *Xba* I) für die Klonierung.

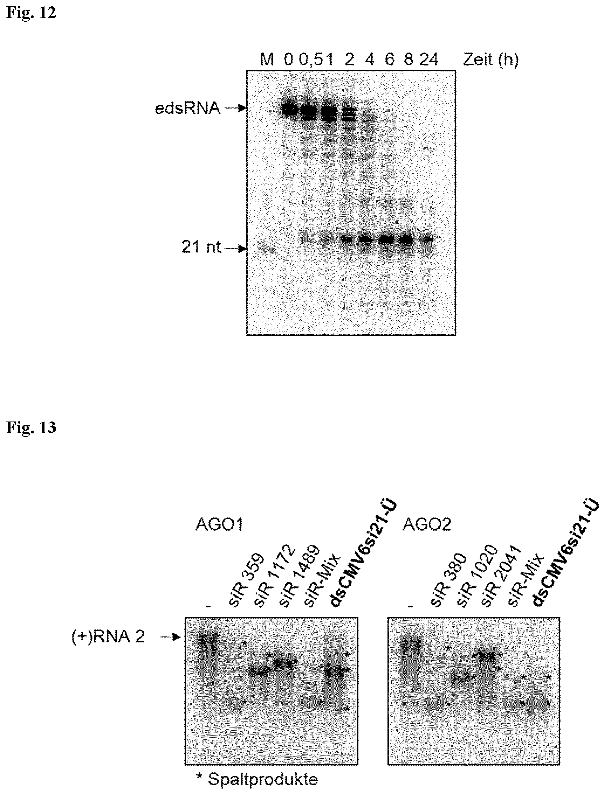

**Figur 12****. Prozessierung einer edsRNA durch DCLs *in vitro.*** Radioaktiv markierte dsCMV6si21 wurde BYL zugesetzt und die Prozessierung durch die im Extrakt endogen enthaltenen DCL4, DCL2 und DCL3 über einen Zeitraum von 24 h verfolgt. Proben, die nach den indizierten Zeitpunkten nach Zugabe der dsRNA dem BYL entnommen wurden, wurden mittels PAGE aufgetrennt und mittels Autoradiographie visualisiert (M = 21 nt siRNA als Marker). Das definierte Bandenmuster lässt auf eine definierte Prozessierung der edsRNA durch die DCL-Proteine schließen. Dabei entsteht ein signifikanter Anteil an 21 nt siRNAs.

**Figur 13****. *Slicer*-Assay mit einzelnen AGO1- und AGO2-spezifischen esiRNAs/ERNAs aus CMV-RNA 2 sowie mit den analogen esiRNAs/ERNAs die aus einer edsRNA in BYL generiert werden.** In BYL wurden AGO1- bzw. AGO2/RISC mit individuellen *e*siRNAs/ERNAs, mit einem entsprechenden Mix aus diesen *e*siRNAs/ERNAs, oder *e*siRNAs/*E*RNAs rekonstituiert, die im BYL durch die dort präsenten DCL aus der edsRNA ,dsCMV6si21' prozessiert wurden. Die endonukleolytische Hydrolyse der radioaktiv markierten *target-RNA* wurde mittels Agarose-Gelelektrophorese und Autoradiographie detektiert. Sternchen (^{∗}) markieren die durch *slicer*-Aktivität der AGO/RISC generierten Spaltprodukte. Sowohl die individuellen siRNAs als auch die aus der edsRNA prozessierten siRNAs führen zur effizienten Spaltung der *target-RNA* in die erwarteten Spaltprodukte.

**Figur 14****. NGS *RNA*-*seq*-Analyse von siRNAs, die in BYL aus einer edsRNA (die die Sequenzen von 21 nt *e*siRNAs/*E*RNAs enthält) generiert wurden. Angegeben sind die Anteile der *reads* von 21 nt *e*siRNAs/*E*RNAs an der Gesamtheit aller 21 nt *reads.*** (A) Anteil der *e*siRNA/ERNA *guide-* und *passenger* strand-*reads* basierend auf deren Position auf der hier eingesetzten edsRNA dsCMV6si21. (B) Anteil der *guide-* und *-passenger strand-reads* pro *e*siRNA/*E*RNA. Alle CMV-spezifischen *e*siRNAs/*E*RNAs konnten detektiert werden. Somit konnte gezeigt werden, dass diese durch das in BYL enthaltene DCL4 aus der entsprechenden edsRNA prozessiert werden. (C) Vergleich der prozentualen aus der edsRNA generierten und detektierbaren Anteile der *guide-* und *passenger strands* CMV-spezifischer siRNAs. Es wird deutlich, dass die eingesetzten *e*siRNAs/*E*RNAs zu einem hohen Anteil (zu ca. 60% der *reads*) gefunden werden und somit präferenziell durch DCL4 aus der edsRNA generiert werden.

**Figur 15****. Vergleich der protektiven Wirkung verschiedener dsRNAs *in planta.*** Vier bis fünf Wochen alte *N*. *benthamiana-*Pflanzen wurden mit verschiedenen dsRNAs sowie den genomischen CMV-RNAs mechanisch inokuliert. Der Einsatz der dsCMV6si21-Ü, bestehend aus sechs 21 nt langen CMV-spezifischen *e*siRNAs/ERNAs, vermittelt einen sehr effizienten (100%) Schutz vor einer CMV Infektion. Mit einem analogen Konstrukt, bestehend aus 22 nt langen Varianten derselben *e*siRNAs/ERNAs, wurde ein verminderter Schutz (30%) der Pflanzen vor einer CMV-Infektion erreicht. (A) Prozentualer Anteil asymptomatischer Pflanzen (dpi = *days post inoculation*). (B) Repräsentative Pflanzenbilder 35 Tage nach der mechanischen Ko-Inokulation. Der prozentuale Anteil asymptomatischer Pflanzen pro dsRNA ist angegeben. (C) Vergleich der protektiven Wirkung doppel- sowie einzelsträngiger RNA *in planta.* Dargestellt ist der prozentuale Anteil asymptomatischer *N. benthamiana-*Pflanzen über einen Zeitraum von 35 Tagen nach mechanischer Inokulation verschiedener dsRNAs bzw. der entsprechenden Einzelstränge (1 bzw. 2) in Gegenwart der genomischen CMV-RNAs. Lediglich die doppelsträngige RNA bestehend aus mehreren CMV-spezifischen *e*siRNAs/*E*RNAs (dsCMV6si21-Ü) schützt sehr effektiv vor einer CMV-Infektion. Dies lässt darauf schließen, dass der Schutz dieser dsRNA auf deren Prozessierung in *e*siRNAs/*E*RNAs durch pflanzliche DCLs beruht.

**Tabelle 7. Sequenzen und Aktivitäten der in den jeweiligen *screenings* gegen drei verschiedene mRNA-torgets identifizierten esiRNAs/ERNAs gegen *M*. *incognita.*** Die siRNAs wurden als siRMI (MI für M. *incognita*) und entsprechend der jeweiligen *target-RNA* (SPF - *Splicing factor* SEQ ID NO: 191; INT - *Integrase* SEQ ID NO: 192; ACT - *Actin* 4 SEQ ID NO: 193) und der Position des 5'-Endes des identifizierten *guide strands* benannt. Angegeben ist ob die *e*siRNAs/*E*RNAs eine nematizide Wirkung *in planta* haben (ja/n.t. - bisher nicht getestet). Angegeben sind ferner die *slicer*-Aktivitäten im *in vitro* Slicer-Assay: Hier ist der Prozentsatz der durch die jeweiligen *e*siRNAs/*E*RNAs gespaltenen *target-RNA* im Vergleich zu eingesetzter unspezifischer siRNA, siR gf698 (siR GFP) (0%) angegeben (siehe auch **Figur** 5). Schließlich ist auch die Aktivität der *e*siRNAs/*E*RNAs nach Behandlung der Tiere (*soaking*) *in vivo* angegeben: Hier ist die Normalisierte Expressionsrate (NER) der jeweiligen mRNAs, bestimmt durch qRT-PCR (siehe auch **Figur 14****)** angegeben (ermittelt über qRT-PCR). Die Werte (in Prozent) geben den Anteil an gespaltenem Produkt wieder: Z.B. im Falle der siRMISPF 441 erfolgt nach Behandlung bei 90% der mRNA ein *slicing in vivo* (im Vergleich zu 0% bei Behandlung mit siR gf698). Wie gezeigt und beschrieben war eine enge Korrelation zwischen RNA-Spaltungsaktivität *in vitro* und *in vivo* zu beobachten. Aufgelistet sind solche RNAs, für die und Varianten hiervon, entsprechend den Ansprüchen und eingesetzt gegen *M. incognita* in Form von *e*siRNAs/*E*RNAs oder davon abgeleiteter sRNAs oder *e*ASO (eNAs), ein Patentschutz beantragt wird. Die jeweiligen SEQ ID NO sind als **"Zusatz zu Tabelle 7"** extra gelistet.

**Figur 16****. *Silencing* der *Splicing factor* mRNA von M. *incognita* durch identifizierte esiRNAs/ERNAs *in vivo* und *in planta.*** A) *In vivo:* Normalisierte Expressionsrate (NER) der *Splicing factor* mRNA (SPF SEQ ID NO: 191), bestimmt durch qRT-PCR (gezeigt ist die noch messbare Quantität an mRNA) nach der Inkubation von Nematodenlarven im J2-Stadium (J2s) für 24 Stunden in siRNA-Lösung (siRMISPF 166, siRMISPF 220 und siRMISPF 441). Für die genspezifische reverse Transkription (RevertAid Reverse Transkriptase) zu cDNA nach einem Standardverfahren, wurden ca. 500 ng der Gesamt-RNA bzw. 1/5 verdünnte cDNA als PCR-template verwendet. Die MI 18S rRNA (HE667742) wurde in der quantitativen RT-PCR als Referenzgen verwendet. Die Berechnung der normalisierten Expressionsraten (NER) erfolgte nach der mathematischen Methode 2^{-ddCt} unter Verwendung des mittleren Ct-Wertes des Referenzgens. Zwei repräsentative Experimente mit je zwei Wiederholungen sind gezeigt (water - Wasserkontrolle ohne siRNA; siR GFP (bzw. siR gf698) - Negativkontrolle: gemessene mRNA Quantität 100%). B) *In planta*/Anzahl der Eier (eggs-J2 s) in Tomatenwurzeln. J2s wurden 24 Stunden lang in Gegenwart von Kontroll-siRNA (siR GFP bzw. siR gf698) oder Test-siRNA in Wasser inkubiert. Die Fähigkeit der Nematoden, eine Infektion in Wurzeln von Tomatenpflanzen (zwei Wochen alt n=15) zu etablieren und ihren Lebenszyklus abzuschließen, wurde durch Zählen der Eier (eggs J2) 56 Tage nach der Infektion analysiert. Fehlerbalken: Standardabweichung des Mittelwerts (SDM). Sternchen zeigen statistische Unterschiede zu den Kontrollen bei p≤ 0,05 (^{∗}), p≤ 0,01 (^{∗∗}) und p≤ 0,001 (^{∗∗∗}) an, die mit einem zweiseitigen Student's t-Test ermittelt wurden. Ein repräsentatives Experiment ist gezeigt.

**Figur 17****. *Silencing* der *Actin 4* und *Integrase* mRNAs *in vivo.*** Die hier gezeigten repräsentativen Experimente wurden analog zu **Figur 16** durchgeführt. A: Normalisierte Expressionsrate (NER) der *Actin* 4-mRNA (ACT SEQ ID NO: 193), bestimmt durch qRT-PCR (siehe **Figur 14****)** nach 24-stündiger Inkubation von Nematodenlarven im J2-Stadium (J2s) in siRNA-Lösungen (siR 154, siR 200, siR 303, siR 419, siR 433, siR 435 und siR 661). B: Normalisierte Expressionsrate (NER) der *Integrase*-mRNA (INT SEQ ID NO: 192), bestimmt durch qRT-PCR (siehe **Figur 14****)** nach 24-stündiger Inkubation von Nematodenlarven im J2-Stadium (J2s) in siRNA-Lösungen (siR 135, siR 273, siR 423 und siR 444). Fehlerbalken: Standardabweichung des Mittelwerts (SDM). Sternchen zeigen statistische Unterschiede zu den Kontrollen bei p≤ 0,05 (^{∗}), p≤ 0,01 (^{∗∗}) und p≤ 0,001 (^{∗∗∗}) an, die mit einem zweiseitigen Student's t-Test ermittelt wurden.

**Tabelle 8. Sequenzen der in den jeweiligen *screenings* gegen verschiedene mRNA-*targets* identifizierte esiRNAs/ERNAs gegen *B. cinerea.*** Die siRNAs wurden als siRBC (BC für *B. cinerea*) und entsprechend der jeweiligen *target-RNA* (VDS - *VDS51* SEQ ID NO: 194; DCTN - *DCTN1* SEQ ID NO: 195; SAC-*Sac1* SEQ ID NO: 196; ERG - *ERG27* SEQ ID NO: 197; EF - *EF2* SEQ ID NO: 198; CHS - *CHS1* SEQ ID NO: 199) und der Position des 5'-Endes des identifizierten *guide strands* benannt. Aufgelistet sind solche RNAs, für die und Varianten hiervon, entsprechend den Ansprüchen und eingesetzt gegen B. *cinerea* in Form von *e*siRNAs/*E*RNAs oder davon abgeleiteter sRNAs oder *e*ASO (eNAs), ein Patentschutz beantragt wird. Die jeweiligen SEQ ID NO sind als **"Zusatz zu Tabelle 8"** extra gelistet.

**Figur 18****. *Slicer*-Assay mit esiRNAs/ERNAs die gegen verschiedene *B. cinerea* mRNAs identifiziert wurden.** Die Slicer-Assays wurden wie oben beschrieben mit AGO1 aus *Colletotrichum graminicula* (siehe Text) durchgeführt. Die Translationsreaktion von C. *graminicula* AGO1 wurde in Gegenwart der zu testenden, synthetischen siRNA-Duplexe durchgeführt, sodass es zum Einbau der gewünschten siRNAs in den AGO/RISC kam. Anschließend wurden die radioaktiv markierten mRNAs als *target-RNA* zugegeben und inkubiert. Aus den Ansätzen wurde Gesamt-RNA isoliert und mittels denaturierender PAGE und Autoradiographie auf Spaltprodukte analysiert. Die verwendeten *target-RNAs* (VDS steht für *VDS51;* DCTN für *DCTN1;* SAC für *Sac1;* ERG für *ERG27;* EF für *EF2)* und die entstandenen Spaltprodukte sind jeweils markiert. Die siRNAs sind entsprechend **Tabelle 8** benannt. Zum Vergleich wurden jeweils siRNAs (257, 470, 653, 808) im Assay eingesetzt, die durch *in silico*-Prognose (https://www.zhaolab.org/pssRNAit/) ermittelt wurden. Als Kontrolle wurde der Slicer-Assay ohne siRNA (-) durchgeführt. Als weitere Kontrolle wurden ursprünglich verwendete dsRNAs (ds) bzw. *pools* von siRNAs ("pool") auf das Gel aufgetragen.

**Figur 19****. Inhibition des Wachstums von *B. cinerea* durch *topical application* von esiRNAs/ERNAs, die gegen definierte *target*-RNAs gerichtet sind.** (A) Balkendiagramme, die die Größe der von *B. cinerea* auf Blättern von *Arabidopsis thaliana* (Wildtyp-Form; Col-0) entwickelten Läsionen wiedergeben. Die Pflanzen wurden mit Suspensionen von *B. cinerea*-Sporen und verschiedenen Kombinationen von siRNAs inokuliert: i) einer Mischung aus sechs *e*siRNAs/ERNAs, die gegen die *Erg27* mRNA gerichtet sind (siRBCERG); ii) einer Mischung aus vier *e*siRNAs/ERNAs, die gegen die *Erg27* mRNA gerichtet sind, sowie zwei *e*siRNA/*E*RNAs, die gegen die *Sacl* mRNA gerichtet sind und eine *e*siRNA/*E*RNA, die gegen die *Ef2* mRNA gerichtet ist (siRBCMIX); und iii) eine siRNA, die gegen die *GFP*-mRNA gerichtet ist (siR gf698 bzw. siR GFP). Für jede Behandlung wurden zwölf Pflanzen verwendet, und pro Pflanze wurden drei Blätter (Blätter 8, 9 und 10) inokuliert. Auf jedes Blatt wurde ein Tropfen der Suspensionen mit den Sporen und 400 ng (siR gf698 bzw. siR GFP), 2400 ng (siRBCERG) oder 2800 ng (siRBCMIX bzw. siR GFP) der RNAs gegeben. Suspensionen ohne RNAs ("Water") wurden als Pilzwachstumskontrolle verwendet. Die Läsionsfläche auf den Blättern (siehe Beispiele unteres Feld) wurde 3 Tage nach der Inokulation (dpi) mit der Software ImageJ bestimmt, und die Größen wurden in fünf Kategorien eingeteilt: i) >50 mm² (+++), ii) 20-50 mm² (++), iii) 10-20 mm² (+), iv) 1-10 mm² (+/-), und v) 0 mm² (-). Die verschiedenen Kategorien sind in Prozent angegeben. Repräsentative Bilder von Blättern mit Läsionen der verschiedenen Kategorien (unteres Feld). (B) qRT-PCR-Bestimmung der *Erg27* mRNA-Level *in vivo* (während des pilzlichen Infektionsprozesses). 3 dpi wurde aus den Pilzläsionen der Blätter Gesamt-RNA extrahiert und cDNA mit einem Standardprotokoll generiert. Für die nachfolgende PCR-Amplifikation wurden zwei primer-Sätze verwendet, die die Regionen abdecken, auf die die verschiedenen *e*siRNA/*E*RNAs abzielen (oberer Bereich). Der Level an *Erg27* mRNA wurde auf den endogenen Level der *B. cinerea Actin* mRNA ("*house-keeping*") normiert. Angegeben ist die Normalisierte Expressionsrate (NER; siehe auch vorangegangene Figuren). Die Balken stellen den Mittelwert von vier biologischen Wiederholungen dar (die jeweils sechs Läsionen von unabhängigen Blättern enthielten), die Fehlerbalken und die SDM (Standardabweichung des Mittelwerts; unterer Bereich). Statistisch signifikante Unterschiede zur Wasserkontrolle ("Water") wurden mit einem zweiseitigen Student's t-Test ermittelt: ^{∗}p≤0,05, ^{∗}p≤0,01, ^{∗∗∗}p≤0,001.

### Anwendungsbeispiele

### Anwendungsbeispiel 1: Nukleinsäure-Wirkstoffe gegen Cucumber mosaic virus, CMV

Entsprechend dem beschriebenen Verfahrensprotokoll wurden *e*siRNAs/*E*RNAs gegen zwei RNA-Segmente des CMV-Genoms (Stamm Fny) als *target*-RNAs gescreent: RNA 2 (SEQ ID NO: 189) kodiert für das 2a-Protein und die subgenomische RNA 4A, die wiederum für den viralen Suppressor des RNA-*silencing* (VSR), 2b kodiert. RNA 3 (SEQ ID NO: 190) kodiert für das 3a Protein und die subgenomische RNA 4, die wiederum für das Kapsid-Protein (CP) kodiert **(****Figur 1D** und **Figur 4****).** Das *screening* wurde sowohl mit AGO1 (L-Version; Gursinsky et al., 2015) als auch mit AGO2 aus *Nicotiana benthamiana* (Nb) durchgeführt. Die *target-RNAs* wurden in doppelsträngiger Form in den screens eingesetzt.

Gegen CMV-RNA 2 wurden so mit NbAGO1L und NbAGO2 siRNAs identifiziert, von denen ein erheblicher Anteil im *Slicer*-Assay eine effiziente Hydrolyse der *target-RNA* (CMV-RNA 2) induziert **(****Figur 1** **A** und **B).** Die Spaltungsaktivität (slicer-Aktivität) wird indiziert durch den Anteil an endonukleolytisch generiertem Spaltungsprodukt im Vergleich zu nicht gespaltenen *target-RNA* (siehe **Figur 1****);** die slicer-Aktivität kann entsprechend auch quantifiziert werden (Beispiel in **Figur 5****).** Eine hohe Spaltaktivität dieser siRNAs wurde auch in Präsenz einer hohen Quantität unspezifischer (kompetitiv wirksamer) siRNA (siR GFP oder siR gf698, gerichtet gegen die mRNA des *green fluorescent protein* (GFP)) festgestellt **(****Figur 1C****).**

Auf diese Weise wurden siRNAs charakterisiert, die mit AGO1 auf der CMV-RNA 2 besonders spaltungsaktiv sind. Es waren dies siRCV2 359, siRCV2 1172 und siRCV2 1489 (Benennung der Kandidaten erfolgt mit "siR", "CV" für CMV, der jeweiligen RNA und der Position der viralen (+)RNA, zu der das 5'-Nukleotid des *siRNA-guide strands* komplementär ist). Eine hohe Spaltungsaktivität hatten auch siRCV2 149, siRCV2 186, siRCV2 1613, siRCV2 1982, siRCV2 2441, siRCV2 2562 und siRCV2 2727. Die funktionellen *in vitro* Daten mit diesen siRNAs sind in **Figur 1** gezeigt; die Sequenzen dieser siRNAs sind in **Tabelle 1** (im **Zusatz zu Tabelle 1** mit den jeweiligen SEQ ID NO) zusammengefasst.

Analog wurden siRCV2 380, siRCV2 1020 und siRCV2 2041 als besonders spaltungsaktive RNA Wirkstoffe im RISC-Komplex zusammen mit AGO2 identifiziert. Eine hohe Spaltungsrate hatten auch siRCV2 407, siRCV2 449, siRCV2 540, siRCV2 557, siRCV2 1054 und siRCV21248 **(****Figur 1****; Tabelle 1).** Die auf der RNA 2 lokalisierten Bindestellen der besonders spaltungsaktiven (effizientesten) siRNAs sind in **Figur 1D** schematisch aufgezeigt. Identifizierte siRNAs, die im *in vitro* Slicer-Assay eine besonders hohe Spaltungsaktivität auf der CMV-RNA 2 zeigen (siehe auch **Figur 5****),** wurden im Folgenden als *e*siRNAs/*E*RNAs gegen die CMV-RNA 2 klassifiziert.

Beispielhaft wurden die *in vitro* identifizierten *e*siRNAs/*E*RNAs siRCV2 1020, 1172, 359, 1489, 380 und 2041 in Protektionsexperimenten *in planta* eingesetzt. Dazu wurde eine statistisch repräsentative Anzahl an *N*. *benthamiana* Pflanzen (n=12-15) mit Carborundum über ein Standard-Protokoll ("*rub-in*") - mit 150 pmol (~1µg) der zu testenden siRNA (synthetisch bezogen von einer Firma) und je 20 fmol der genomischen CMV-RNAs (1-3; hergestellt durch *in vitro* Transkription ausgehend von "infektiösen cDNAs" (erhalten von Prof. Fernando García-Arenal Rodriguez (Polytechnische Universität Madrid) und Prof. John Carr (Universität Cambridge); Rizzo und Palukaitis 1990) pro Pflanze ko-inokuliert (RNAs gelöst in 15 mM KH₂PO₄, 25 mM Glycin Lösung). Die Infektion mit den CMV-RNAs wurde dabei so durchgeführt, dass die jeweilige Quantität an RNAs, wenn sie ohne Zusatz in *N*. *benthamiana* inokuliert wird, zu 100% zu einer Infektion mit signifikanter Symptomentwicklung führt (sog. "maximaler *challenge*")*.* Als Kontrollen wurden die unspezifische siR gf698 bzw. siRNAs eingesetzt, die im zuvor durchgeführten *screen* keine oder eine nur geringfügige Spaltungsaktivität auf der *target-RNA* aufwiesen (siRCV2 1844 und siRCV2 2634) eingesetzt. Die Pflanzen wurden über 35 Tage (dpi; *days post infection)* auf Symptomentwicklung untersucht. **Figur 2A** zeigt repräsentative Bilder von Pflanzen, die in der genannten Weise behandelt wurden, **Figur 2B** zeigt die Gesamtverläufe über mehrere Experimente (drei biologische Wiederholungen). Dabei wurde deutlich, dass mit siRCV2 359 und siRCV2 1489 jeweils zu 93% ein Schutz und mit siRCV2 1020 und siRCV2 1172 jeweils zu 100% ein Schutz gegen eine CMV-Infektion erreicht werden kann: D.h. alle bzw. die klare Mehrheit der so behandelten Pflanzen blieben bei dem angewandten maximalen *challenge* symptomlos. Mit siRCV2 380 und siRCV2 2041 wurde zu 60% ein Schutz gegen eine CMV-Infektion erreicht, d.h. 40% dieser Pflanzen entwickelten bei *challenge* Symptome. Die verschiedenen Negativkontroll-siRNAs vermittelten geringen oder keinen Schutz: Nur 0-7 % der so behandelten Pflanzen blieben während des *challenge* symptomlos. Diese Schutzwirkung konnte auch dadurch bestätigt werden, dass in Pflanzen, die nach Behandlung mit den jeweils protektiven *e*siRNAs/*E*RNAs "per Auge" nach 35 dpi als asymptomatisch identifiziert wurden, genomische CMV-RNA nicht mehr über RT-PCR (Standardprozedur) nachweisbar war **(****Figur 2C****).**

Mit 22 nt Versionen der jeweiligen *e*siRNAs/*E*RNAs ließ sich ebenfalls ein Pflanzenschutz gegen eine CMV-Infektion erreichen. Exemplarisch gezeigt ist dies in **Figur 3****:** 22 nt *e*siRNA/*E*RNA-Versionen zeigten in *in vitro* Slicer-Assays eine ähnliche, in manchen Fällen etwas geringere *slicer*-Aktivität in den jeweiligen AGO/RISC auf der *target-RNA.* In Pflanzenprotektionsexperimenten zeigte sich eine ähnliche Tendenz: Entweder war die Protektion mit 22 nt siRNAs vergleichbar gut, oder sie war leicht vermindert im Vergleich zur Situation mit eingesetzten 21 nt *e*siRNAs/*E*RNAs (in **Figur 3** exemplarisch gezeigt mit siRCV2 359 und siRCV2 1020). *Slicer*-Assays und Protektionsexperimente mit 24 nt langen Versionen der jeweiligen *e*siRNAs/*E*RNAs zeigten ähnliche Ergebnisse wie die mit den 22 nt *e*siRNAs/*E*RNAs durchgeführten Experimente (nicht gezeigt).

Mit dem gleichen Verfahren wurden gegen CM-RNA 3 *e*siRNAs/*E*RNAs gescreent; dabei wurden wieder NbAGO1L und NbAGO2 verwendet. Auch hier wurden wieder eine Reihe von siRNAs identifiziert, die mit AGO1 eine sehr hohe (siRCV3 239, siRCV3 507, siRCV3 985) bzw. eine hohe (siRCV3 151, siRCV3 988, siRCV3 1098) und mit AGO2 eine sehr hohe (siRCV3 593, siRCV3 1019, siRCV3 1569) bzw. eine hohe (siRCV3 358, siRCV3 478, siRCV3 496, siRCV3 592, siRCV3 733, siRCV3 1394) *slicer*-Aktivität gegen die CMV *target-RNA* 3 induzieren **(****Figur 4A-C).** Die Positionierung der im *Slicer*-Assay effizientesten siRNA-*guide strands* auf der RNA 3 ist in **Figur 4D** gezeigt. **Tabelle 2** fasst die Ergebnisse aus dem *screen* von *e*siRNAs/*E*RNAs (im **Zusatz zu Tabelle 2** mit den jeweiligen SEQ ID NO) gegen CMV-RNA 3 zusammen.

Im Vergleich zeigten die gegen CMV-RNA 2 charakterisierten *e*siRNAs/*E*RNAs eine tendenziell höhere *slicer*-Aktivität als die gegen RNA 3 identifizierten *e*siRNAs/*E*RNAs **(****Figur 5****).** Die Gründe hierfür könnten in einer leicht höheren Stabilität oder grundsätzlich kompakteren Strukturierung der CMV-RNA 3 (geringeren Zugänglichkeit von a-Sites) im Vergleich zu der Situation von CMV-RNA 2 liegen (Daten nicht gezeigt). **Figur 5** zeigt die *slicer*-Aktivität von drei exemplarisch ausgewählten, jeweils mit AGO1 bzw. AGO2 identifizierten *e*siRNAs/*E*RNAs im *Slicer-*Assay und in quantitativer Auswertung.

In Protektionsexperimenten in Pflanzen zeigten die gegen CMV-RNA 3 charakterisierten 21 nt und 22 nt *e*siRNA*s*/*E*RNAs einen wirksamen Schutz gegen das Virus. Wie aus den *in vitro* Experimenten zu erwarten, war dieser etwas geringer als dies bei den *e*siRNA/*E*RNA-Wirkstoffen gegen CMV-RNA 2 der Fall war (**Figur 6**). Slicer-Assays und Protektionsexperimente mit 24 nt langen Versionen der jeweiligen *e*siRNAs/*E*RNAs zeigten ähnliche Ergebnisse wie die mit den 22 nt *e*siRNAs/*E*RNAs durchgeführten Experimente (nicht gezeigt).

Durch Sequenzvergleiche konnte ermittelt werden, dass viele der hier aus dem CMV-Stamm Fny identifizierten *e*siRNA*s*/*E*RNAs auch auf den analogen RNA-Segmenten anderer CMV-Stämme wirksam sind, weil eine komplette Komplementarität zwischen den Sequenzen der jeweiligen *guide strands* dieser *e*siRNA*s*/*E*RNAs und den jeweiligen *target-sites* auf den viralen RNAs vorliegt. **Figur 7** zeigt für sechs *e*siRNA*s*/*E*RNAs exemplarisch die Übereinstimmungen der Sequenzen zu den komplementären *target-sites* auf den RNA 2-Genomsegmenten von CMV (modifiziert nach Roossinck, 1999). Ferner sind in der Figur berechnete *expectation rates* für RNA-RNA *mismatches* (fehlende Basenpaarung an einer oder mehrerer Positionen in der Nukleinsäure bei der Bindung des siRNA *guide strands* an die *targetsite*) aufgeführt. Basierend auf diesen Daten ist in **Tabelle 3** eine komplette Übersicht der identifizierten *e*siRNAs/*E*RNAs gegeben, deren *guide strands* vollständig komplementär, d.h. ohne *mismatches,* an die *target-sites* in RNAs 2 bzw. 3 der jeweiligen CMV-Stämme binden. Wie aus der Tabelle hervorgeht sind diese *e*siRNA*s*/*E*RNAs somit, entweder einzeln oder in Kombination eingesetzt, gegen praktisch alle CMV- Stämme der Gruppen IA sowie IB protektiv. Dies ist in den **Tabellen 4** und **5** verdeutlicht.

Wie aus den **Tabellen 3-5** hervorgeht bindet keine der identifizierten *e*siRNA*s*/*E*RNAs ohne *mismatch* an die entsprechenden *target-sites* in den RNAs 2 und 3 der CMV-Stämme der Untergruppe II. Wie oben ausgeführt und zuvor in einer Anmeldung (PCT/EP2022/057596) beschrieben, indizieren *e*siRNA*s*/*E*RNAs sog. a-Sites, d.h. Bereiche, die die *target-sites* dieser *e*siRNA*s*/*E*RNAs in komplex strukturierten *target*-RNAs enthalten, die trotz Strukturierung zugänglich für RISC oder andere Endonuklease-haltige zelluläre Komplexe sind (PCT/EP2022/057596). Dementsprechend kann, über eine jeweilige Anpassung der Sequenzen der hier identifizierten *e*siRNA*s*/*E*RNAs auf die *target-sites* in den *target*-RNA*s*, d.h. über die Vermeidung von *mismatches,* dennoch ein *silencing* der genomischen RNAs 2 und 3 der Stämme der Untergruppe II erfolgen. Dies wird in **Tabelle 6** zusammengefasst: Hier ist die Zahl an *mismatches* der *guide strands* der identifizierten *e*siRNA*s*/*E*RNAs bei Bindung an die jeweiligen *target-sites* in den RNAs 2 und 3 der verschiedenen CMV-Stammuntergruppen wiedergegeben. Die Tabelle zeigt auch *mismatches* in den sog. seed-Regionen der siRNAs an: Die *seed-*Region ist die wichtigste Interaktionsregion einer siRNA mit der *target-*RNA während des AGO/RISC vermittelten *silencing*-Prozesses (Jackson und Linsley, 2010). Zudem zeigt die Tabelle *mismatches* am 5'-Ende der RNA an, das nachweislich nicht an der Bindung der siRNA beteiligt ist. Fasst man diese Daten zusammen, so kann die hier identifizierte Population an *e*siRNA*s*/*E*RNAs entweder in der unveränderten Form (wie bei den meisten Mitgliedern der 1a und 1b Untergruppen) oder variiert an 1 bis zu 10 Nukleotiden (**Tabelle 6**) zu Protektionszwecken gegen alle CMV-Stämme eingesetzt werden. Die *e*siRNAs/*E*RNAs können dabei entweder einzeln oder, nochmals deutlich wirksamer, als Kombination (Mix) in RNAi-Verfahren eingesetzt werden. Durch letzteren Ansatz kann besonders wirksam ein *escape* über *antigenic drifts* oder *shifts* verhindert werden. Die analoge Feststellung gilt für andere sRNAs und *e*ASO (gesamt als *e*NAs bezeichnet), deren Sequenzen von den entsprechenden *e*siRNAs/*E*RNAs abgeleitet werden konnte.

**Zusammenfassung zu Anwendungsbeispiel 1: Die gestellten Aufgaben wurden gelöst. Erfindungsgemäß wurden *e*siRNA/*E*RNA-Wirkstoffe bzw. davon abgeleitete *e*NA-Wirkstoffe identifiziert, die gegen CMV eine antivirale Wirkung haben und im Pflanzenschutz gegen CMV eingesetzt werden können. Die identifizierten *e*NAs können entweder in der unveränderten Form oder verändert an 1 bis 10 Nukleotiden zu Protektionszwecken gegen alle bekannten CMV Varianten eingesetzt werden. Die *e*NAs können entweder einzeln oder, nochmals deutlich wirksamer, als Kombination (Mix) in *RNA-silencing* Ansätzen gegen CMV im Pflanzenschutz eingesetzt werden.**

### Anwendungsbeispiel 2: Doppelsträngige-RNA-Wirkstoffe, die esiRNA/ERNA-Sequenzen oder Sequenzen anderer, verwandter sRNAs enthalten

Aufgabe war es, für die praktische Anwendung von *e*siRNAs/*E*RNAs oder verwandter *s*RNAs im RNA-*silencing*-Prozess optimierte doppelsträngige RNAs, sog. *e*dsRNAs, zu konstruieren. *e*dsRNAs sollten entsprechend die Sequenzen mehrerer *esiRNAs*/*ERNA*s und/oder davon abgeleiteter sRNAs enthalten. Die *e*dsRNAs sollen bei Einsatz im Zielorganismus durch die dort präsenten DCLs/Dicer zu einem hohen Anteil in die ursprünglich konstituierenden *e*siRNAs/*E*RNAs bzw. *s*RNAs prozessiert werden, und diese sollen dann in entsprechenden RISC gegen anvisierte *target*-RNAs aktiv werden. Um durch diese *e*dsRNAs eine maximal wirksame *silencing* Antwort gegen eine oder sogar mehrere *target*-RNAs zu erzeugen sollen diese *e*dsRNAs als weitere Eigenschaft aus Sequenzen von *e*siRNAs/*E*RNAs oder verwandter sRNAs bestehen, die in verschiedenen AGO-Proteinen und damit verschiedenen RISC im RNA-*silencing* aktiv sein können.

Erfindungsgemäß wurde bei der Konstruktion von *e*dsRNAs eine bis dahin unvollständig belegte Hypothese zugrundgelegt. Diese geht davon aus, dass DCLs/Dicer an beiden Enden (Termini) einer dsRNA aktiv sein können. Die Hypothese beinhaltet ferner, dass DCLs/Dicer durch die Präsenz sog. "pseudo-siRNA-Sequenzen" an den Enden der dsRNA in ein "getaktetes *processing"* gezwungen werden können. Eine pseudo-siRNA-Sequenz im Sinne der Erfindung ist demnach eine doppelsträngige Ribonukleotidsequenz beliebiger Komposition, die, entsprechend der geplanten Prozessierung der jeweiligen edsRNA durch DCLs/Dicer 21, 22, 23 oder 24 nt lang ist. Pseudo-siRNA-Sequenzen sollten sich entsprechend an den Termini der Doppelstrang-RNA befinden und durch ihre Präsenz die auf dieser dsRNA aktiven DCLs/Dicer in ein getaktetes *processing* zwingen. Getaktetes *processing* bedeutet, dass entsprechend Position und Länge der pseudo-siRNA-Sequenzen und entsprechend der Aktivität der beteiligten DCLs/Dicer die endonukleolytischen Spaltungen der dsRNA so erfolgen, dass die dabei präferenziell generierten sRNAs die gleiche Länge wie die pseudo-siRNAs aufweisen. Ein Beispiel: Sind die pseudo-siRNA-Sequenzen 21 nt lang und ist DCL4 das prozessierende Enzym (DCL4 generiert präferenziell 21 nt siRNAs aus einer dsRNA), dann werden aus einer so konstruierten *e*dsRNA präferiert 21 nt sRNAs generiert und zwar in der Reihenfolge, wie sie in der Sequenzabfolge nach den pseudo-siRNAs in der *e*dsRNA gereiht sind. Dabei agiert DCL4 prozessiv: Ausgehend von den pseudo-siRNA-Sequenzen an den Termini der edsRNA erfolgen die endonukleolytischen Schnitte des Enzyms nacheinander. Wird eine edsRNA entsprechend so aufgebaut, dass z.B. auf 21 nt lange pseudo-siRNA-Sequenzen unmittelbar 21 nt lange *e*siRNA/*E*RNA-Sequenzen folgen (siehe **Figuren 8-11****),** sollten also präferenziell 21 nt lange *e*siRNAs/*E*RNAs entstehen.

Pseudo-siRNA-Sequenzen können darüber hinaus Elemente enthalten, die für eine erfolgreiche Transkription und Prozessierung der jeweiligen edsRNAs wichtig sind: Dies können Bereiche des jeweiligen Transkriptionspromotors oder -terminators sein; es können aber auch Teile eines Ribozyms (*ribozyme*) sein, welches das korrekte 5'- bzw. 3'-Ende der jeweiligen RNAs generiert (siehe **Figuren 8** und **11****).** Letzteres trifft beispielsweise zu auf HH-Ribozyme; ein Beispiel wurde in diesem Anwendungsbeispiel verwendet: Hier enthält die pseudo-siRNA-Sequenz eine definierte Anzahl an Nukleotiden, die komplementär zum 5'-Ende des Ribozyms sind und durch Hybridisierung dessen Helix I und damit dessen funktionelle Struktur mit ausbilden können **(****Figur 11****).**

Das Grundprinzip des Aufbaus so konzipierter edsRNAs stellt sich somit folgendermaßen dar **(****Figur 8** und folgende):
- Die Sequenz enthält mindestens eine pseudo-siRNA-Sequenz, die, wie ausgeführt, ein getaktetes *processing* durch Dicer/DCLs ermöglicht. Zudem enhält die Sequenz der *e*dsRNA eine Anzahl von 5' nach 3' "aufgereihter" Sequenzen von *e*siRNAs/*E*RNAs oder von *e*siRNAs/*E*RNAs abgeleiteter anderer sRNAs wie z.B. miRNAs, in **Figur 8** als 1-n in sense (s) Richtung und mit n-1 in antisense (as) Richtung beschrieben. Diese Sequenzen können aus verschiedenen *screens* stammen, und entsprechend können die *e*siRNA/*E*RNAs bzw. davon abgeleiteten *s*RNAs in verschiedenen AGO/RISC (in **Figur 8** als A-Z beschrieben) aktiv sein. Im optimalen Fall können die *e*siRNA/*E*RNAs bzw. davon abgeleiteten sRNAs, die eine edsRNA konstituieren, auch gegen unterschiedliche *target-RNAs* aus einem oder aus unterschiedlichen Organismen gerichtet sein. n in **Figur 8** ist dabei eine Zahl zwischen 2 und unendlich, vorzugsweise zwischen 2 und 100 oder wie für Anspruch 1 d. oben beschrieben.
- Die Generierung einer edsRNA kann auf zweierlei Weise erfolgen: Aus zwei komplementären RNA-Molekülen oder aus einem RNA-Molekül, das zwei komplementäre Teilbereiche enthält **(****Figur 8****).** Im zweiten Fall werden die beiden komplementären Teilbereiche der transkribierten RNA durch einen *spacer* miteinander verbunden und bilden einen *hairpin.* Der *spacer* ist eine Sequenz beliebiger Komposition mit einer Minimallänge von 4 Nukleotiden (Definition wie für *hairpins* ist oben gegeben), die für den spezifischen Zweck der *e*dsRNA-Konstruktion aber funktionelle Bereiche wie beispielsweise Ribozyme, Transkriptionspromotoren oder Transkriptionsterminatoren, Transportsignale oder *Splice*-Stellen enthalten kann. Auf diese Weise erfüllt die *spacer*-Sequenz zusätzlich zu der Funktion die beiden komplementären Einzelstrangkomponenten einer dsRNA zu verbinden noch andere Zwecke: Enthält sie beispielsweise *Splice*-Signale kann der *spacer* im Zuge der Expression der RNA *in vivo* durch die *splice*-Maschinerie der Zelle verkürzt werden. Da der *spacer* im Gegensatz zu den doppelsträngigen RNA-Regionen sensitiv ist gegenüber Ribonukleasen (wie z.B. die Einzelstrang-spezifischen RNasen T1 oder A) kann diese Sequenz durch diese RNasen auch komplett entfernt werden (siehe auch **Figur 8****).**
- Die Transkription der RNAs kann *in vitro* oder *in vivo* erfolgen. Der Doppelstrang wird durch Hybridisierung der komplementären RNA-Stränge erhalten **(****Figur 8****).** Die Transkription kann durch verschiedenste Promotoren (siehe beispielhaft **Figur** 1**1**) erfolgen. Die Transkriptionstermination kann durch jedwede Art von Transkriptionsterminatoren (siehe beispielhaft **Figur 11****)** erfolgen.
- Je nach Art der Generierung sind die Termini der *e*dsRNAs entweder stumpf (*blunt*), oder sie enthalten einen Überhang (-Ü). Sie können auf unterschiedliche Weise, z.B. über ,run-off Transkription' bzw. Termination der jeweiligen Polymerasen oder durch die Aktivität von Ribozymen (z.B. HH oder HDV-Ribozyme, wie in diesem Anwendungsbeispiel verwendet) über *self-splicing* generiert werden.
- Die Sequenzen beider Stränge sind so konzipiert, dass bei der Prozessierung durch DCLs jeweils die authentischen *s*RNA *guide* und *passenger strand*-Sequenzen generiert werden. Das *processing,* das überwiegend zur Generierung der konstituierenden *e*siRNAs/*E*RNAs bzw. davon abgeleiteten *s*RNAs führt, wird durch die Präsenz der pseudo-siRNA-Sequenzen und das damit getaktete *processing* durch die DCLs/Dicer sichergestellt (siehe oben und **Figuren 8-14****).**

Die Hypothese des ,getakteten *processing'* und damit die Funktionalität verschiedener *e*dsRNA-Konstrukte, die nach diesen Prinzipien aufgebaut waren, wurde erfindungsgemäß, d.h. empirisch über iteratives *trial and error*-Prinzip, überprüft und bestätigt. Beispiele der Komposition einfach konzipierter *e*dsRNAs, die über die Hybridisierung zweier komplementärer RNA-Moleküle generiert werden können, sind in **Figuren 9** und **10** (SEQ ID NO: 181 und 185 sowie 182 und 186) gegeben. Derartige *e*dsRNAs können über *in vitro* Transkription von klassisch aufgebauten cDNAs, bestehend aus einem Promotor, der kodierenden Sequenz und einem Terminator/*run-off*, generiert werden. Erfindungsgemäße Beispiele für die Komposition von *e*dsRNAs, die sowohl *in vitro* als auch *in vivo* generiert werden können, sind über die Darstellung der zugrundeliegenden cDNA-Konstrukte (SEQ ID NO: 200, 201, 201, 203 und 204) in **Figur 11** gegeben. Letztere edsRNAs enthalten alle einen *spacer,* der für den Einsatz bestehen bleiben kann oder, wie oben beschrieben, verkürzt oder abgebaut werden kann.

In **Figuren 12-15** ist die Funktionalität derart konzipierter edsRNAs demonstriert. Beispielhaft gezeigt ist dies am Beispiel der edsRNA ,dsCMV6si21' **(****Figuren 9** und **10****;** SEQ ID NO 181 und 185). Diese *e*dsRNA enthält sechs verschiedene, 21 nt lange *e*siRNAs/*E*RNA-Sequenzen, die gegen die CMV-RNA 2 gerichtet sind. Für drei dieser *e*siRNAs/*E*RNAs wurde in Anwendungsbeispiel 1 gezeigt, dass sie im pflanzlichen AGO1/RISC gegen CMV-RNA 2 aktiv sind; für weitere drei dieser *e*siRNAs/*E*RNAs wurde zuvor gezeigt, dass sie im pflanzlichen AGO2/RISC gegen CMV-RNA 2 aktiv sind. Die Funktionalität der *e*dsRNA war dabei unabhängig davon ob sie *in vitro* oder *in vivo* generiert wurde **(****Figur 11****,** nicht gezeigt) und ob diese RNA stumpfe (*blunt*) oder überhängende Enden (-Ü) hatte. Die Funktionalität der *e*dsRNA war dabei ebenfalls unabhängig davon, ob sie aus 21, 22 oder 24 nt langen pseudo-siRNAs und *e*siRNAs/*E*RNAs aufgebaut war.

Zunächst wurde demonstriert, dass die edsRNA in BYL, das erwiesenermaßen die DCLs 4, 2 und 3 in aktiver Form enthält (Schuck et al., 2013), zu 21 nt, 22 nt bzw. 24 nt langen siRNAs prozessiert wird.

Somit wurde die grundsätzliche Prozessierbarkeit der konzipierten *e*dsRNA-Konstrukte durch Dicer/DCL demonstriert **(****Figur 12****).**

Durch den Einsatz der *e*dsRNA in *in vitro Slicer*-Assays mit der *target-RNA* konnte gezeigt werden, dass aus der *target*-RNA (exemplarisch CMV-RNA 2), die durch die Aktivität der enthaltenen *e*siRNAs/*E*RNAs zu erwartenden Spaltprodukte generiert werden **(****Figur 13****).** Dies wurde auch in einem Folgeexperiment bestätigt. Dort wurden die aus einer *e*dsRNA, dsCMV6si21, in BYL (durch die dort aktiv vorliegenden DCLs) prozessierten siRNAs durch NGS (*RNA-seq*) bestimmt: So ist deutlich zu erkennen, dass sowohl die pseudo-siRNAs als auch die *e*siRNAs/*E*RNAs zu hohen Anteilen (erkennbar an den detektierbaren *guide* oder *passenger strands*) aus der edsRNA prozessiert werden **(****Figur 14** A+B; gezeigt sind jeweils 21 nt reads). Die erfindungsgemäß gestellte Hypothese, die zu dem ermittelten Aufbau von *e*dsRNAs führte, erwies sich damit als korrekt: Aus so aufgebauten dsRNAs werden durch DCL4 bevorzugt die 21 nt pseudo-siRNAs und *e*siRNAs/*E*RNAs generiert: In der Summe sind dies ca. 60% aller generierten 21 nt siRNAs **(****Figur 14** C). Erfindungsgemäß wurde gezeigt, dass die Prozessierung durch die DCLs von beiden Enden der dsRNA aus in Form eines "getakteten *processing"* erfolgt.

In Pflanzenprotektionsexperimenten wurde schließlich die hohe Wirksamkeit (Protektivität) des Einsatzes von *e*dsRNAs gegen einen CMV-*challenge* (Infektion mit einer letalen Konzentration an CMV) und damit final die Funktionalität der *e*dsRNAs demonstriert **(****Figur 15****).**

Aus den exemplarisch gezeigten Experimenten wurden mehrere, wichtige Aspekte deutlich:
- Zum einen wurde gezeigt, dass im Vergleich zu einer unspezifischen dsRNA (Kontroll-dsRNA 2, dsGFP; bestehend aus der analog langen Sequenz aus der *GFP*-mRNA und der dazu komplementären RNA) eine aus 21 nt *e*siRNA/*E*RNAs aufgebaute *e*dsRNA (dsCMV(6si21)) zu 100% protektiv wirksam ist. D.h. alle der mit der *e*dsRNA behandelten Pflanzen blieben im *challenge*-Experiment mit CMV im Vergleich zur unspezifisch aufgebauten dsRNA ohne Symptome **(****Figur 15****).**
- Im Vergleich zu einer konventionell^{∗} aufgebauten dsRNA, die eine zu dsCMV6si21 analog lange ds Sequenz aus der CMV-RNA 2 enthielt (Kontroll-dsRNA 1, dsCMV; bestehend aus einem ähnlich langen Bereich der CMV RNA2 und der dazu komplementären RNA; siehe **Figuren 9** und **10****),** hatten edsRNAs eine deutlich effizientere antivirale Wirksamkeit:
   ^{∗} konventionell heißt: nach diesem Prinzip aufgebaute dsRNAs werden derzeit im RNAi-mediierten Pflanzenschutz eingesetzt
   So zeigte die konventionell aufgebaute dsCMV (Kontroll-dsRNA 1) zwar einen gewissen Schutz im Vergleich zu dsGFP (Kontroll-dsRNA 2), dieser hielt aber nicht über den kompletten Testzeitraum von 35 dpi an. Im Vergleich dazu hatte, wie erwähnt, die aus *e*siRNA/*E*RNAs aufgebaute *e*dsRNA einen über den gesamten Testzeitraum anhaltenden Schutz von 100% **(****Figur 15** A und B). Wichtig ist hier anzumerken, dass die als Kontrolle angewendete dsCMV auch zwei der hier zuvor als *e*siRNAs/*E*RNAs charakterisierten Sequenzen enthielt (gezeigt in **Figur 9**). Somit spiegelte diese Kontrolle die Situation einer Behandlung mit einer konventionellen dsRNA sehr gut wieder, in der, wenn überhaupt, nur wenige *e*siRNA/*E*RNA-Sequenzen enthalten sind.
- *e*dsRNAs, die 21 nt *e*siRNAs/*E*RNAs generierten, waren am effizientesten protektiv gegen CMV; edsRNAs, die die gleichen *e*siRNAs/*E*RNAs, aber als 22 nt Versionen produzierten, waren weniger protektiv: Im Gegensatz zu der 21 nt edsRNA zeigte die 22 nt edsRNA keinen 100% Schutz über den gesamten Testzeitraum. Im Vergleich zur konventionell aufgebauten dsCMV war der Schutz durch die 22 nt *e*siRNAs/*E*RNAs generierende edsRNA dennoch deutlich ausgeprägter **(****Figur 15** A und B). Das gleiche gilt für eine 24 nt *e*siRNA*s*/*E*RNAs generierende edsRNA (nicht gezeigt).
- Einzelsträngige (ss)-RNA-Komponenten von edsRNAs zeigen keinen vergleichbaren Schutz **(****Figur 15** C). Dieses als weitere Kontrolle durchgeführte Experiment demonstrierte, dass eingesetzte edsRNAs tatsächlich ausschließlich in der doppelsträngigen Form aktiv sind und die Schutzwirkung demzufolge auf deren Prozessierung in *e*siRNA/*E*RNAs durch die pflanzlichen DCLs *in vivo* beruht.

**Zusammenfassung zu Anwendungsbeispiel 2: Erfindungsgemäß wurden völlig neuartig aufgebaute edsRNA-Wirkstoffe entwickelt, die einfach über Transkription *in vitro* oder *in vivo* hergestellt werden können und aus denen, über die Prozessierung durch Dicer/DCLs, signifikante Quantitäten der verschiedenen konstituierenden *s*RNAs generiert werden. So aufgebaute *e*dsRNAs können als effiziente antipathogene Wirkstoffe eingesetzt werden.**

### Anwendungsbeispiel 3: Nukleinsäure-Wirkstoffe gegen Meloidogyne incognita

*e*siRNAs/*E*RNAs wurden in BYL mit dem beschriebenen Verfahren mit dem AGO2 Protein von *Nicotiana benthamiana* (Nb) gegen drei mRNAs von *Meloidogyne incognita target-*Genen (SEQ ID NO: 191 bis 193) identifiziert: Diese *target*-Gene kodieren für die Proteine "Splicing factor", "Actin-4" sowie "Integrase". Neben anderen Faktoren war für diese Proteine eine jeweils essenzielle Funktion im Lebenszyklus von *Meloidogyne incognita* anzunehmen (siehe auch oben).

Die jeweiligen Gen- bzw. *target*-RNA-Sequenzen (siehe Anhang) wurden folgendermaßen erstellt. Im Fall von *Actin-4*: Mit der Accession No. von *Actin-4* für *C*. *elegans* wurde in https://wormbase.org//#012-34-5 die Sequenz ermittelt. Damit wurde in der *database* https://meloidogyne.inrae.fr/ ein Sequenzvergleich (Blast N) durchgeführt. So wurde die Sequenz der vorhergesagten cDNA in *M. incognita* ermittelt. Diese wurde wiederum durch einen Sequenzvergleich der abgeleiteten Proteinsequenz (Blast P) in der NCBI-Datenbank (National Center for Biotechnology Information) bestätigt und kloniert. Im Fall von *Splicing factor* und *Integrase*: Hier wurden EST (expressed sequence tag) - Sequenzen von *M. incognita* (AW828516 bzw. AW871671) aus der NCBI-Datenbank verwendet. Diese wurden für einen Blast N in der *database* https://meloidogyne.inrae.fr/ verwendet. Nachdem die vorhergesagten cDNA-Sequenzen gefunden waren, wurde ein Blast P mit den abgeleiteten Proteinsequenzen durchgeführt, mit dem bestätigt werden konnte, dass diese homolog zu den Sequenzen von Splicing factor und Integrase von *C. elegans* sind. Entsprechend wurden diese cDNAs kloniert.

Die in **Tabelle 7** zusammengefassten *e*siRNAs/*E*RNAs (SEQ ID NO in **Zusatz zu Tabelle 7)** wurden identifiziert. Die Benennung der Kandidaten erfolgt mit "siR", "MI" für *M*. *incognita,* der jeweiligen mRNA (SPF, *Splicing factor*; INT, *Integrase*; ACT, *Actin 4*) und der Position der RNA, zu der das 5'-Nukleotid des siRNA-*guide strands* komplementär ist). Sie wurden auf verschiedene Weise validiert (siehe **Figuren 16** und **17****).**
- *Slicer*-Assays. Die *e*siRNAs/*E*RNAs und davon abgeleitete *e*dsRNAs **(****Figuren 8** und **11****)** wurden einerseits in *Slicer*-Assays in BYL mit NbAGO2 bezüglich ihrer Spaltungs (*slicer*)-Aktivität der jeweiligen *target-*RNA *in vitro* validiert.
- *In vivo.* Zum anderen wurde *in vivo* (d.h. im Tier) auf die Effizienz der Spaltung der jeweiligen mRNA nach Aufnahme der siRNAs getestet. Dazu wurden *M. incognita* J2 Tiere gewonnen, und 10.000 der Tiere wurden in 40 µl Wasser inkl. 50-200 ng/µl RNA für 24 Stunden inkubiert (*soaked* -,durchtränkt'), sodass sie die RNAs aufnahmen. Als Negativkontrolle wurde die siRNA siR gf698 (siR GFP) verwendet. Anschließend wurden die Nematoden mit einem Standardverfahren aufgeschlossen, die Gesamt-RNA extrahiert und die Spaltung der jeweiligen *target*-mRNA mit entsprechenden *primern* und quantitativer Echtzeit-RT-PCR (qRT-PCR) untersucht. Für die genspezifische reverse Transkription (RevertAid Reverse Transkriptase, ThermoFisher) zu cDNA, ebenfalls nach einem Standardverfahren, wurden ca. 500 ng der Gesamt-RNA bzw. 1/5 verdünnte cDNA als PCR-template verwendet. Als Referenzgen wurde das *M*. *incognita GAPDH-*Gen (Minc3s07075g40689) verwendet. Die Berechnung der normalisierten Expressionsraten (NER), die die nach der Spaltung verbleibende messbare Quantität an *target*-mRNA wiedergibt (siehe auch **Figur 14****),** folgte nach der mathematischen Methode 2^{-ddCt} unter Verwendung des mittleren Ct-Wertes des Referenzgens.
- *In vivo*/*in planta.* Schließlich wurde die Fähigkeit der *e*siRNAs/*E*RNAs getestet, den kompletten Lebenszyklus der Nematoden *in planta* zu unterdrücken. In einer ersten Form der Experimente wurden J2 Tiere, wie oben beschrieben, 24 Stunden in wassergelösten RNAs oder Kontroll-RNA eingelegt ("*gesoakt*"). Anschließend wurde eine Infektion zwei Wochen alter Tomatenpflanzen mit den so behandelten Nematoden nach einem Standardprotokoll (500 Tiere per Pflanze) durchgeführt und untersucht, ob diese Behandlung zu einer Verminderung der Eiablage nach der Infektion bzw. zu einer verringerten Pathogenese (insbesondere Gallenbildung) der infizierten Pflanzen führt.

In einer zweiten Form dieser Experimente wurden Infektionsstudien an frischen Keimlingen durchgeführt. Dazu wurden Tomatensamen durch Einweichen in 70% Ethanol oberflächensterilisiert. Nach Entfernen des Ethanols wurden die Samen zur Weiterbehandlung mit einer Lösung behandelt, die 30% (v/v) NaOCl und 0,02% Triton X-100 enthält und 20-30 Minuten lang inkubiert. Anschließend wurden sie mit sterilem, destilliertem Wasser gewaschen und in Petrischalen gegeben, die etwa 6 mm 0,6% Phytagel pH 6,4 in ¼ MS (Murashige and Skoog Medium) inklusive 0,5% Sucrose enthielten. Zehn Samen wurden nach dem Zufallsprinzip in jeder Petrischale verteilt, diese dann mit Nescofilm versiegelt und bei 28°C mit einem 16-Stunden-Licht/8-Stunden-Dunkel-Zyklus gehalten. Sieben bis zehn Tage nach der Aussaat wurde jeder Keimling mit 100-200 sterilen und *gesoakten* J2 beimpft. Die J2 Tiere wurden dazu 5 Minuten lang in einer Lösung mit 0,004% Quecksilberchlorid, 0,004% Natriumazid und 0,002% Triton X-100 oberflächensterilisiert, mit sterilem Wasser gewaschen und in Agarose (0,1 %) suspendiert. Das *soaking* wurde wie oben beschrieben ausgeführt. Die inokulierten Sämlinge wurden unter den oben beschriebenen Bedingungen aufgezogen. Der Infektionsprozess wurde überwacht, indem die Gallenbildung drei Wochen nach der Inokulation untersucht wurde.

Die in **Tabelle 7** aufgeführten *e*siRNAs/*E*RNAs hatten in den genannten Tests einen signifikanten *silencing* bzw. nematiziden Effekt (siehe **Figuren 16** und **17****).**

**Zusammenfassung zu Anwendungsbeispiel 3: Die gestellten Aufgaben wurden gelöst. Erfindungsgemäß wurden *e*siRNA/*E*RNA-Wirkstoffe bzw. davon abgeleitete *e*NA-Wirkstoffe identifiziert, die gegen *M*. *incognita* eine nematizide Wirkung haben und im Pflanzenschutz gegen *M. incognita* eingesetzt werden können. Die identifizierten *e*NAs können entweder in der unveränderten Form oder verändert an 1 bis zu 10 Nukleotiden zu Protektionszwecken gegen alle bekannten *M*. *incognita* Varianten eingesetzt werden. Die *e*NAs können entweder einzeln oder, nochmals deutlich wirksamer, als Kombination (Mix) in *RNA-silencing* Ansätzen im Pflanzenschutz gegen *M. incognita* eingesetzt werden. Zudem können *e*dsRNAs, die die Sequenzen von identifizierten *e*siRNAs/ERNAs bzw. davon abgeleiteten anderen *s*RNAs enthalten, im Pflanzenschutz gegen *M*. *incognita* eingesetzt werden.**

### Anwendungsbeispiel 4: Nukleinsäurewirkstoffe gegen Botrytis cinerea

*e*siRNAs/*E*RNAs wurden in BYL über das beschriebene Verfahren mit dem AGO1 Protein von *Colletotrichum graminicula* (*C. graminicula* ist, wie *B. cinerea,* ein pflanzenpathogener Pilz) gegen verschiedene mRNAs von *B. cinerea target-*Genen gescreent. Drei der *target-RNAs* (SEQ ID NO: 197, 199, 198); wurden dabei aufgrund der Tatsache ausgewählt, dass die jeweils kodierten Proteine, "Cytochrome P450-Monooxygenase" (Erg27), "Chitin-Synthase 1" (CHS1) und "Elongationsfaktor 2" (EF2), bereits als Zielmoleküle verschiedener Fungizide bekannt sind (siehe Einleitung zu *B. cinerea*). Drei weitere *target*-RNAs (SEQ ID NO: 194, 195, 196) wurden ausgewählt, weil die jeweils kodierten Proteine, "Vacuolar protein sorting 51" (VPS51), "Dynactin" (DCTN1) und "Suppressor of actin" (SAC1), in den vesikularen Transportweg im Pilz involviert sind und essenzielle Virulenzfaktoren von *B. cinerea* während der Interaktion mit Pflanzenwirten sind.

Die cDNAs für diese Gene wurden aus einer mRNA-Präparation generiert und kloniert. Die mRNA-Präparation wurde nach einem Standardverfahren aus *B*. *cinerea*-infizierten *Arabidopsis thaliana* Pflanzen präpariert.

Die in **Tabelle 8** zusammengefassten *e*siRNAs/*E*RNAs wurden identifiziert (SEQ ID NO in "**Zusatz zu Tabelle 8").** Die Benennung der Kandidaten erfolgt mit "siR", "BC" für *B. cinerea,* der jeweiligen RNA (ERG - *Erg 27*; CHS - *Chitin-Synthase* 1; EF - *Elongation factor 2*; VPS - *Vacuolar protein sorting 51*; DCTN
- *Dynactin1*; SAC - *Suppressor of actin*) und der Position der RNA, zu der das 5'-Nukleotid des siRNA-*guide strands* komplementär ist. Sie wurden folgendermaßen validiert.
- *Slicer*-Assays. Dazu wurden die *e*siRNAs/*E*RNAs mit *C*. *graminicula* AGO1 hinsichtlich der Spaltungs (*slicer*)-Aktivität der jeweiligen *target-*RNA *in vitro* getestet: Im Vergleich zu *in silico*-prognostizierten siRNAs (https://www.zhaolab.org/pssRNAit/) zeigen praktisch alle identifizierten *e*siRNAs/*E*RNAs eine deutlich bessere slicer-Aktivität **(****Figur 18****).**
- *In vitro* Pilzwachstumsexperimente. *B. cinerea* B05.10 wurde auf Früchten kultiviert. Nachdem der Pilz fünf Tage lang sporulieren konnte, wurden die Sporen gesammelt und in entsprechendem Medium auf eine Endkonzentration von 1×10⁵ Sporen/ml verdünnt (Standardverfahren). Anschließend wurde die Keimung der Sporen durch Zugabe von Phosphat initiiert. Um dies gleichmäßig zu ermöglichen, wurden 10 µl 1M Phosphatpuffer, pH 6.4, zu 990 µl Sporenlösung hinzugefügt. Die Auswirkungen der *e*siRNAs/*E*RNAs auf das Wachstum von *B. cinerea* wurden *in vitro* auf Kartoffel-Dextrose-Agar-Platten (PDA) getestet. Von einer Sporenlösung mit einer Endkonzentration von 1×10⁵/ml wurden 10µl in die Mitte einer Platte gegeben. Für die Behandlung wurden 400-8000 ng RNA in die Sporenlösung gegeben und dann kontinuierlich alle 12 h zugeführt. Neben den *B*. *cinerea*-spezifischen *e*siRNAs/*E*RNAs und edsRNAs wurden den Sporen als Kontrollen siR gf698 (siR GFP) und Wasser zugesetzt. Die Wachstumsdynamik unter Einfluss der RNAs im Vergleich zu den Kontrollen wurde durch Messung des Pilzkoloniedurchmessers (ImageJ software) an Tag 1, 2, 3 bzw. 5 nach der Inokulation bewertet.
- *In vivo*/*in planta* Pilzwachstumsexperimenten. Hier wurde die Fähigkeit der *e*siRNAs/*E*RNAs getestet, den kompletten Lebenszyklus von *B. cinerea in planta* zu unterdrücken. Dazu wurden RNAs mit einer Endkonzentration von 400-8000 ng in die Lösung induzierter Sporen (1×10⁵/ml, siehe oben) gegeben und die Lösung für anderthalb Stunden bei Raumtemperatur inkubiert. Für eine Infektion wurden die Blätter von *A*. *thaliana* mit einem 10µl Tropfen der Inkubationslösung benetzt. Anschließend wurden die Pflanzen zwei Tage lang in Kammern mit hoher Luftfeuchtigkeit aufbewahrt; danach wurde die Größe der durch den Pilz verursachten Läsionen mit denen von Kontrollpflanzen verglichen (ImageJ software), die mit Sporen infiziert wurden, die die siR gf698 Kontrolle bzw. Wasser enthielten (siehe auch **Figur 19****).** Zur weiteren Bestimmung des Pilzwachstums wurde das Gewebe der Blattabschnitte entnommen und die *B. cinerea*-DNA mittels qPCR unter Verwendung der Standardkurvenmethode quantifiziert. Nachgewiesen wurden dazu das *B. cinerea Actin-*Gen und ein Kontrollgen aus einem Plasmidpräparat, das in die Pflanzenproben *gespiked* wurde. Das Plasmid-Kontrollgen wurde verwendet, um die Effizienz des DNA-Extraktionsprozesses abzuschätzen und die Amplifikation von *cut A* zu normalisieren. Schließlich wurde die Menge der *B. cinerea*-DNA durch Interpolation der normalisierten *cut A*-Werte auf der Standardkurve bestimmt.
- Durch Transkriptanalyse. Um das *silencing* der *target-RNAs* in *B. cinerea* zu bewerten, wurde eine mRNA-Expressionsanalyse mittels quantitativer Echtzeit-RT-PCR (qRT-PCR) durchgeführt. Dazu wurden 1 × 10⁵ Sporen in 2 mL flüssigem Medium aufgenommen und RNA zugegeben (siehe oben). Die Proben wurden anschließend bei Raumtemperatur unter Schütteln 24, 48 und 72 Stunden lang gezüchtet. Die RNA-Extraktion aus der Pilzprobe erfolgte mit TRIzol gemäß den Anweisungen des Herstellers. Die qRT-PCR erfolgte wie in **Anwendungsbeispiel 3** beschrieben (siehe auch **Figur 19****).**

Die in **Tabelle 8** aufgeführten *e*siRNAs/*E*RNAs hatten einen signifikanten *silencing* Effekt *in vitro* **(****Figur 18****)** bzw. fungiziden Effekt **(****Figur 19****).** Wie auch aus **Figur 19** hervorgeht, hatte die Behandlung *in planta,* auch mit unspezifischen NAs, einen leichten fungiziden Effekt, die Behandlung mit spezifisch gegen target-RNAs gerichteten *e*NAs hatte jedoch eine signifikant stärkere fungizide Wirkung.

**Zusammenfassung zu Anwendungsbeispiel 4: Die gestellten Aufgaben wurden gelöst. Erfindungsgemäß wurden *e*siRNA/*E*RNA-Wirkstoffe bzw. davon abgeleitete *e*NA-Wirkstoffe identifiziert, die gegen *B. cinerea* eine nematizide Wirkung haben und im Pflanzenschutz gegen *B*. *cinerea* eingesetzt werden können. Die identifizierten *e*NAs können entweder in der unveränderten Form oder verändert an 1 bis zu 10 Nukleotiden zu Protektionszwecken gegen alle bekannten *B*. *cinerea* Varianten eingesetzt werden. Die *e*NAs können entweder einzeln oder, nochmals deutlich wirksamer, als Kombination (Mix) in *RNA-silencing* Ansätzen im Pflanzenschutz gegen *B. cinerea* eingesetzt werden. Zudem können *e*dsRNAs, die die Sequenzen von identifizierten *e*siRNAs/*E*RNAs bzw. davon abgeleiteten anderen *s*RNAs enthalten, im Pflanzenschutz gegen *B. cinerea* eingesetzt werden.**

### Referenzen

1. Shabalina, S.A. and Koonin, E.V. (2008) Origins and evolution of eukaryotic RNA interference, Trends Ecol Evol, 23, 578-587.
2. Carthew, R.W. and Sontheimer, E.J. (2009) Origins and Mechanisms of miRNAs and siRNAs, Cell, 136, 642-655.
3. Ding, S.-W. (2010) RNA-based antiviral immunity, Nat Rev Immunol, 10, 632-644.
4. Zvereva, A.S. and Pooggin, M.M. (2012) Silencing and innate immunity in plant defense against viral and non-viral pathogens, Viruses, 4, 2578-2597.
5. Gammon, D.B. and Mello, C.C. (2015) RNA interference-mediated antiviral defense in insects, Curr Opin Insect Sci, 8, 111-120.
6. Guo, Z., Li, Y. and Ding, S.-W. (2019) Small RNA-based antimicrobial immunity, Nat Rev Immunol, 19, 31-44.
7. Fukudome, A. and Fukuhara, T. (2017) Plant dicer-like proteins: double-stranded RNA-cleaving enzymes for small RNA biogenesis, J Plant Res, 130, 33-44.
8. Song, M.-S. and Rossi, J.J. (2017) Molecular mechanisms of Dicer: Endonuclease and enzymatic activity, Biochem J, 474, 1603-1618.
9. Deleris, A., Gallego-Bartolome, J., Bao, J., Kasschau, K.D., Carrington, J.C. and Voinnet, O. (2006) Hierarchical action and inhibition of plant Dicer-like proteins in antiviral defense, Science, 313, 68-71.
10. Parent, J.-S., Bouteiller, N., Elmayan, T. and Vaucheret, H. (2015) Respective contributions of Arabidopsis DCL2 and DCL4 to RNA silencing, Plant J, 81, 223-232.
11. Elbashir, S.M., Harborth, J., Lendeckel, W., Yalcin, A., Weber, K. and Tuschl, T. (2001a) Duplexes of 21-nucleotide RNAs mediate RNA interference in cultured mammalian cells, Nature, 411, 494-498.
12. Elbashir, S.M., Martinez, J., Patkaniowska, A., Lendeckel, W. and Tuschl, T. (2001b) Functional anatomy of siRNAs for mediating efficient RNAi in Drosophila melanogaster embryo lysate, EMBO J, 20, 6877-6888.
13. Meister, G. (2013) Argonaute proteins: functional insights and emerging roles, Nat Rev Genet, 14, 447-459.
14. Kobayashi, H. and Tomari, Y. (2016) RISC assembly: Coordination between small RNAs and Argonaute proteins, Biochim Biophys Acta, 1859, 71-81.
15. Carbonell, A. and Carrington, J.C. (2015) Antiviral roles of plant ARGONAUTES, Curr Opin Plant Biol, 27, 111-117.
16. Mi, S., Cai, T., Hu, Y., Chen, Y., Hodges, E., Ni, F., Wu, L., Li, S., Zhou, H., Long, C., Chen, S., Hannon, G.J. and Qi, Y. (2008) Sorting of small RNAs into Arabidopsis argonaute complexes is directed by the 5' terminal nucleotide, Cell, 133, 116-127.
17. Takeda, A., Iwasaki, S., Watanabe, T., Utsumi, M. and Watanabe, Y. (2008) The mechanism selecting the guide strand from small RNA duplexes is different among argonaute proteins, Plant Cell Physiol, 49, 493-500.
18. Schuck, J., Gursinsky, T., Pantaleo, V., Burgyán, J. and Behrens, S.-E. (2013) AGO/RISC-mediated antiviral RNA silencing in a plant in vitro system, Nucleic Acids Res, 41, 5090-5103.
19. Liu, Q., Wang, F. and Axtell, M.J. (2014) Analysis of complementarity requirements for plant microRNA targeting using a Nicotiana benthamiana quantitative transient assay, Plant Cell, 26, 741-753.
20. Elbashir, S.M., Lendeckel, W. and Tuschl, T. (2001c) RNA interference is mediated by 21- and 22-nucleotide RNAs, Genes Dev, 15, 188-200.
21. Wang, Y., Sheng, G., Juranek, S., Tuschl, T. and Patel, D.J. (2008) Structure of the guide-strandcontaining argonaute silencing complex, Nature, 456, 209-213.
22. Brodersen, P., Sakvarelidze-Achard, L., Bruun-Rasmussen, M., Dunoyer, P., Yamamoto, Y.Y., Sieburth, L. and Voinnet, O. (2008) Widespread translational inhibition by plant miRNAs and siRNAs, Science, 320, 1185-1190.
23. Iwakawa, H.-O. and Tomari, Y. (2013) Molecular insights into microRNA-mediated translational repression in plants, Mol Cell, 52, 591-601.
24. Khalid, A., Zhang, Q., Yasir, M. and Li, F. (2017) Small RNA Based Genetic Engineering for Plant Viral Resistance: Application in Crop Protection, Front Microbiol, 8, 43.
25. Pooggin, M.M. (2017) RNAi-mediated resistance to viruses: a critical assessment of methodologies, Curr Opin Virol, 26, 28-35.
26. Tomilov, A.A., Tomilova, N.B., Wroblewski, T., Michelmore, R. and Yoder, J.I. (2008) Trans-specific gene silencing between host and parasitic plants, Plant J, 56, 389-397.
27. Banerjee, S., Banerjee, A., Gill, S.S., Gupta, O.P., Dahuja, A., Jain, P.K. and Sirohi, A. (2017) RNA Interference: A Novel Source of Resistance to Combat Plant Parasitic Nematodes, Front Plant Sci, 8, 834.
28. Majumdar, R., Rajasekaran, K. and Cary, J.W. (2017) RNA Interference (RNAi) as a Potential Tool for Control of Mycotoxin Contamination in Crop Plants: Concepts and Considerations, Front Plant Sci, 8, 200.
29. Price, D.R.G. and Gatehouse, J.A. (2008) RNAi-mediated crop protection against insects, Trends Biotechnol, 26, 393-400.
32. Setten, R.L., Rossi, J.J. and Han, S.-P. (2019) The current state and future directions of RNAi-based therapeutics, Nat Rev Drug Discov, 18, 421-446.
33. Qureshi, A., Tantray, V.G., Kirmani, A.R. and Ahangar, A.G. (2018) A review on current status of antiviral siRNA, Rev Med Virol, 28, e1976.
34. Herrera-Estrella, L., Simpson, J. and Martínez-Trujillo, M. (2005) Transgenic plants: an historical perspective, Methods Mol Biol, 286, 3-32.
35. Dong, O.X. and Ronald, P.C. (2019) Genetic Engineering for Disease Resistance in Plants: Recent Progress and Future Perspectives, Plant Physiol, 180, 26-38.
36. Koch, A. and Wassenegger, M. (2021) Host-induced gene silencing - mechanisms and applications, New Phytol, 231, 54-59.
37. Jan, F.-J., Pang, S.-Z., Tricoli, D.M. and Gonsalves, D. (2000) Evidence that resistance in squash mosaic comovirus coat protein-transgenic plants is affected by plant developmental stage and enhanced by combination of transgenes from different lines, J Gen Virol, 81, 2299-2306.
38. Savenkov, E.I. and Valkonen, J.P.T. (2001) Coat protein gene-mediated resistance to Potato virus A in transgenic plants is suppressed following infection with another potyvirus, J Gen Virol, 82, 2275-2278.
39. Simón-Mateo, C. and García, J.A. (2006) MicroRNA-guided processing impairs Plum pox virus replication, but the virus readily evolves to escape this silencing mechanism, J Virol, 80, 2429-2436.
40. Tabashnik, B.E., Brévault, T. and Carriere, Y. (2013) Insect resistance to Bt crops: lessons from the first billion acres, Nat Biotechnol, 31, 510-521.
41. Kung, Y.-J., You, B.-J., Raja, J.A.J., Chen, K.-C., Huang, C.-H., Bau, H.-J., Yang, C.-F., Huang, C.-H., Chang, C.-P. and Yeh, S.-D. (2015) Nucleotide sequence-homology-independent breakdown of transgenic resistance by more virulent virus strains and a potential solution, Sci Rep, 5, 9804.
42. Lucht, J.M. (2015) Public Acceptance of Plant Biotechnology and GM Crops, Viruses, 7, 4254-4281.
43. Dalakouras, A., Wassenegger, M., Dadami, E., Ganopoulos, I., Pappas, M.L. and Papadopoulou, K. (2020) Genetically Modified Organism-Free RNA Interference: Exogenous Application of RNA Molecules in Plants, Plant Physiol, 182, 38-50.
44. Robinson, K.E., Worrall, E.A. and Mitter, N. (2014) Double stranded RNA expression and its topical application for non-transgenic resistance to plant viruses, J Plant Biochem Biotechnol, 23, 231-237.
45. Kaur, G., Cheung, H.-C., Xu, W., Wong, J.V., Chan, F.F., Li, Y., McReynolds, L. and Huang, L. (2018) Milligram scale production of potent recombinant small interfering RNAs in Escherichia coli, Biotechnol Bioeng, 115, 2280-2291.
46. Le Page, M. (2017) Gene-silencing spray lets us modify plants without changing DNA, New Scientist, 3108**.**
49. Dowdy, S.F. (2017) Overcoming cellular barriers for RNA therapeutics, Nat Biotechnol, 35, 222-229.
50. Jackson, A.L. and Linsley, P.S. (2004) Noise amidst the silence: off-target effects of siRNAs?, Trends Genet, 20, 521-524.
51. Jackson, A.L., Burchard, J., Schelter, J., Chau, B.N., Cleary, M., Lim, L. and Linsley, P.S. (2006) Widespread siRNA "off-target" transcript silencing mediated by seed region sequence complementarity, RNA, 12, 1179-1187.
52. Senthil-Kumar, M. and Mysore, K.S. (2011) Caveat of RNAi in plants: the off-target effect, Methods Mol Biol, 744, 13-25.
53. Casacuberta, J.M., Devos, Y., Du Jardin, P., Ramon, M., Vaucheret, H. and Nogué, F. (2015) Biotechnological uses of RNAi in plants: risk assessment considerations, Trends Biotechnol, 33, 145-147.
54. Kamola, P.J., Nakano, Y., Takahashi, T., Wilson, P.A. and Ui-Tei, K. (2015) The siRNA Non-seed Region and Its Target Sequences Are Auxiliary Determinants of Off-Target Effects, PLoS Comput Biol, 11, e1004656.
55. Qu, J., Ye, J. and Fang, R. (2012) Artificial microRNAs for plant virus resistance, Methods Mol Biol, 894, 209-222.
56. Dalakouras, A., Wassenegger, M., McMillan, J.N., Cardoza, V., Maegele, I., Dadami, E., Runne, M., Krczal, G. and Wassenegger, M. (2016) Induction of Silencing in Plants by High-Pressure Spraying of In vitro-Synthesized Small RNAs, Front Plant Sci, 7, 1327.
57. Birmingham, A., Anderson, E., Sullivan, K., Reynolds, A., Boese, Q., Leake, D., Karpilow, J. and Khvorova, A. (2007) A protocol for designing siRNAs with high functionality and specificity, Nat Protoc, 2, 2068-2078.
58. Cerritelli, S.M. and Crouch, R.J. (2009) Ribonuclease H: the enzymes in eukaryotes, FEBS J, 276, 1494-1505.
59. Miozzi, L., Gambino, G., Burgyan, J. and Pantaleo, V. (2013) Genome-wide identification of viral and host transcripts targeted by viral siRNAs in Vitis vinifera, Mol Plant Pathol, 14, 30-43.
60. Fakhr, E., Zare, F. and Teimoori-Toolabi, L. (2016) Precise and efficient siRNA design: a key point in competent gene silencing, Cancer Gene Ther, 23, 73-82.
61. Carbonell, A., Lopez, C. and Daròs, J.-A. (2018) Fast-forward Identification of Highly Effective Artificial Small RNAs Against Different Tomato spotted wilt virus Isolates, Mol Plant Microbe Interact, 32, 142-156.
62. Eastman, P., Shi, J., Ramsundar, B. and Pande, V.S. (2018) Solving the RNA design problem with reinforcement learning, PLoS Comput Biol, 14, e1006176.
63. Han, Y., He, F., Chen, Y., Liu, Y. and Yu, H. (2018) SiRNA silencing efficacy prediction based on a deep architecture, BMC Genomics, 19, 669.
64. Gago-Zachert, S., Schuck, J., Weinholdt, C., Knoblich, M., Große, I., Pantaleo, V., Gursinsky, T. and Behrens, S.-E. (2019) Highly efficacious antiviral protection of plants by small interfering RNAs identified in vitro, Nucleic Acids Res, 47, 9343-9357.
65. Shen, X. and Corey, D.R. (2018) Chemistry, mechanism and clinical status of antisense oligonucleotides and duplex RNAs, Nucleic Acids Res, 46, 1584-1600.
66. Bennett, C.F. (2019) Therapeutic Antisense Oligonucleotides Are Coming of Age, Annu Rev Med, 70, 307-321
67. Wdowikowska, A. and Janicka, M. (2022) Antisense oligonucleotide technology as a research tool in plant biology, Funct Plant Biol, 49, 1-12.
68. Crooke, S.T., Liang, X-H, Bakre B. F., Crooke, R.M. (2021) Antisense technology: A review, J Biol Chem 296, 100416
70. Bachman, P., Fischer, J., Song, Z., Urbanczyk-Wochniak, E. and Watson, G. (2020) Environmental Fate and Dissipation of Applied dsRNA in Soil, Aquatic Systems, and Plants, Front Plant Sci, 11, 21.
71. Scholthof, K.-B.G., Adkins, S., Czosnek, H., Palukaitis, P., Jacquot, E., Hohn, T., Hohn, B., Saunders, K., Candresse, T., Ahlquist, P., Hemenway, C. and Foster, G.D. (2011) Top 10 plant viruses in molecular plant pathology, Mol Plant Pathol, 12, 938-954.
72. Rybicki, E.P. (2015) A Top Ten list for economically important plant viruses, Arch Virol, 160, 17-20.
73. Gallitelli, D. (2000) The ecology of Cucumber mosaic virus and sustainable agriculture, Virus Res, 71, 9-21.
74. Garcia-Arenal, F. and Palukaitis, P. (2008) Cucumber Mosaic Virus, In: Mahy, B.W.J. and van Regenmortel, M.H.V. (eds.), Encyclopedia of Virology, 3rd Ed., Academic Press, Oxford, 614-619.
75. Jacquemond, M. (2012) Cucumber mosaic virus, Adv Virus Res, 84, 439-504.
76. Roossinck, M.J. (2001) Cucumber mosaic virus, a model for RNA virus evolution, Mol Plant Pathol, 2, 59-63.
77. Roossinck, M.J. (2002) Evolutionary history of Cucumber mosaic virus deduced by phylogenetic analyses, J Virol, 76, 3382-3387.
78. Palukaitis, P. (2016) Satellite RNAs and Satellite Viruses, Mol Plant Microbe Interact, 29, 181-186.
79. Nouri, S., Arevalo R., Falk B.W. and Russell, L.G. (2014) Genetic Structure and Molecular Variability of Cucumber mosaic virus Isolates in the United States, PLoS One, 9, e96582.
80. Mochizuki, T. and Ohki, S.T. (2012) Cucumber mosaic virus: viral genes as virulence determinants, Mol Plant Pathol, 13, 217-225.
81. Holeva, M.C., Sklavounos, A., Rajeswaran, R., Pooggin, M.M. and Voloudakis, A.E. (2021) Topical Application of Double-Stranded RNA Targeting 2b and CP Genes of Cucumber mosaic virus Protects Plants against Local and Systemic Viral Infection, Plants (Basel), 10, 963.
82. Ali, A. and Kobayashi, M. (2010) Seed transmission of Cucumber mosaic virus in pepper, J Virol Methods, 163, 234-237.
83. O'Keefe, D.C., Berryman, D.I., Coutts, B.A. and Jones, R.A.C. (2007) Lack of Seed Coat Contamination with Cucumber mosaic virus in Lupin Permits Reliable, Large-Scale Detection of Seed Transmission in Seed Samples, Plant Dis, 91, 504-508.
84. Nicaise, V. (2014) Crop immunity against viruses: outcomes and future challenges, Front Plant Sci, 5, 660.
85. Fadenwürmer - Wikipedia
86. Bird, A.F. and Bird, J. (1991a) The Exoskeleton, In: The structure of nematodes, 2nd Ed., Academic Press Inc, San Diego, 44-74.
87. Perry, R.N. and Moens, M. (2011) Introduction to plant-parasitic nematodes: modes of parasitism, In: Jones, J., Fenoll, C. and Gheysen, G. (eds.), Genomics and Molecular Genetics of Plant-Nematode Interactions, Springer Netherlands, Dordrecht, 3-20.
88. Bird, A.F. and Bird, J. (1991b) The Nervous System, In: The structure of nematodes, 2nd Ed., Academic Press Inc, San Diego, 129-156.
89. Bird, A.F. and Bird, J. (1991c) Digestive System, In: The structure of nematodes, 2nd Ed., Academic Press Inc, San Diego, 183-229.
90. Blok, V.C., Jones, J.T., Phillips, M.S. and Trudgill, D.L. (2008) Parasitism genes and host range disparities in biotrophic nematodes: the conundrum of polyphagy versus specialization, BioEssays, 30, 249-259.
91. Perry, R.N. (1996) Chemoreception in plant parasitic nematodes, Annu Rev Phytopathol, 34, 181-199.
92. Semblat, J.P., Rosso, M.N., Hussey, R.S., Abad, P. and Castagnone-Sereno, P. (2001) Molecular cloning of a cDNA encoding an amphid-secreted putative avirulence protein from the root-knot nematode Meloidogyne incognita, Mol Plant Microbe Interact, 14, 72-79.
93. Curtis, R.H.C. (2007) Plant parasitic nematode proteins and the host-parasite interaction, Brief Funct Genomic Proteomic, 6, 50-58.
94. Trudgill, D.L. and Blok, V.C. (2001) Apomictic, polyphagous root-knot nematodes: exceptionally successful and damaging biotrophic root pathogens, Annu Rev Phytopathol, 39, 53-77.
95. Abad, P. and Williamson, V.M. (2010) Plant nematode interaction: a sophisticated dialogue, Adv Bot Res, 53, 147-192.
96. Jones, M.G.K. and Payne, H.L. (1978) Early stages of nematode-induced giant-cell formation in roots of Impatiens balsamina, J Nematol, 10, 70-84.
97. Caillaud, M.-C., Dubreuil, G., Quentin, M., Perfus-Barbeoch, L., Lecomte, P., de Almeida Engler, J., Abad, P., Rosso, M.-N. and Favery, B. (2008) Root-knot nematodes manipulate plant cell functions during a compatible interaction, J Plant Physiol, 165, 104-113.
98. Castagnone-Sereno, P., Danchin, E.G.J., Perfus-Barbeoch, L. and Abad, P. (2013) Diversity and evolution of root-knot nematodes, genus Meloidogyne: new insights from the genomic era, Annu Rev Phytopathol, 51, 203-220.
99. Papadopoulou, J. and Traintaphyllou, A.C. (1982) Sex differentiation in Meloidogyne incognita and anatomical evidence of sex reversal, J Nematol, 14, 549-566.
100. Hussey, R.S. and Mims, C.W. (1991) Ultrastructure of feeding tubes formed in giant-cells induced in plants by the root-knot nematode Meloidogyne incognita, Protoplasma, 162, 99-107.
101. Chitwood, D.J. and Perry, R.N. (2009) Reproduction, physiology and biochemistry, In: Perry, R.N., Moens, M. and Starr, J.L. (eds.), Root-knot nematodes, CABI, Wallingford, 182-200.
102. Curtis, R.H., Robinson, A.F. and Perry, R.N. (2009). Hatch and host location, In: Perry, R.N., Moens, M. and Starr, J.L. (eds.) Root-knot nematodes, CABI, Wallingford, 139-162.
103. Evans, K., Trudgill, D.L. and Webster, J.M. (eds.) (1993) Plant parasitic nematodes in temperate agriculture, CABI, Wallingford.
104. Chen, S.Y. and Dickson, D.W. (2004) Biological control of nematodes by fungal antagonists, In: Chen, Z.X., Chen, S.Y. and Dickson, D.W. (eds.), Nematology: advances and perspectives. Volume 2: Nematode management and utilization, CABI, Wallingford, 979-1039.
105. Stirling, G.R. (2014) Biological control of plant-parasitic nematodes: soil ecosystem management in sustainable agriculture. CABI, Wallingford.
106. Arguel, M.-J., Jaouannet, M., Magliano, M., Abad, P. and Rosso, M.-N. (2012) siRNAs trigger efficient silencing of a parasitism gene in plant parasitic root-knot nematodes, Genes, 3, 391-408.
107. Bakhetia, M., Charlton, W., Atkinson, H.J. and McPherson, M.J. (2005) RNA interference of dual oxidase in the plant nematode Meloidogyne incognita, Mol Plant Microbe Interact, 18, 1099-1106.
108. Banakar, P., Hada, A., Papolu, P.K. and Rao, U. (2020) Simultaneous RNAi Knockdown of Three FMRFamide-Like Peptide Genes, Mi-flp1, Mi-flp12, and Mi-flp18 Provides Resistance to Root-Knot Nematode, Meloidogyne incognita, Front Microbiol, 11, 573916.
109. Chaudhary, S., Dutta, T.K., Shivakumara, T.N. and Rao, U. (2019) RNAi of esophageal gland-specific gene Mi-msp-1 alters early stage infection behaviour of root-knot nematode, Meloidogyne incognita, J Gen Plant Pathol, 85, 232-242.
110. Dalzell, J.J., McMaster, S., Fleming, C.C. and Maule, A.G. (2010a) Short interfering RNA-mediated gene silencing in Globodera pallida and Meloidogyne incognita infective stage juveniles, Int J Parasitol, 40, 91-100.
111. Dalzell, J.J., Warnock, N.D., Stevenson, M.A., Mousley, A., Fleming, C.C. and Maule, A.G. (2010b). Short interfering RNA-mediated knockdown of drosha and pasha in undifferentiated Meloidogyne incognita eggs leads to irregular growth and embryonic lethality, Int J Parasitol, 40, 1303-1310.
112. Danchin, E.G., Arguel, M.J., Campan-Fournier, A., Perfus-Barbeoch, L., Magliano, M., Rosso, M.-N., Da Rocha, M., Da Silva, C., Nottet, N., Labadie, K., Guy, J., Artiguenave, F. and Abad, P. (2013) Identification of novel target genes for safer and more specific control of root-knot nematodes from a pan-genome mining, PLoS Pathog, 9, e1003745.
113. Dong, L., Li, X., Huang, L., Gao, Y., Zhong, L., Zheng, Y. and Zuo, Y. (2014) Lauric acid in crown daisy root exudate potently regulates root-knot nematode chemotaxis and disrupts Mi-flp-18 expression to block infection, J Exp Bot, 65, 131-141.
114. Dong, L., Xu, J., Chen, S., Li, X. and Zuo, Y. (2016) Mi-flp-18 and Mi-mpk-1 genes are potential targets for Meloidogyne incognita control, J Parasitol, 102, 208-213.
115. Dutta, T.K., Papolu, P.K., Banakar, P., Choudhary, D., Sirohi, A. and Rao, U. (2015) Tomato transgenic plants expressing hairpin construct of a nematode protease gene conferred enhanced resistance to root-knot nematodes, Front Microbiol, 6, 260.
116. Huang, G., Allen, R., Davis, E.L., Baum, T.J. and Hussey, R.S. (2006) Engineering broad root-knot resistance in transgenic plants by RNAi silencing of a conserved and essential root-knot nematode parasitism gene, Proc Natl Acad Sci USA, 103, 14302-14306.
117. Iqbal, S., Fosu-Nyarko, J. and Jones, M.G.K. (2020) Attempt to silence genes of the RNAi pathways of the root-knot nematode, Meloidogyne incognita results in diverse responses including increase and no change in expression of some genes, Front Plant Sci, 11, 328.
118. Niu, J., Jian, H., Xu, J., Chen, C., Guo, Q., Liu, Q. and Guo, Y. (2012) RNAi silencing of the Meloidogyne incognita Rpn7 gene reduces nematode parasitic success, Eur J Plant Pathol, 134, 131-144.
119. Papolu, P.K., Gantasala, N.P., Kamaraju, D., Banakar, P., Sreevathsa, R. and Rao, U. (2013) Utility of host delivered RNAi of two FMRF amide like peptides, flp-14 and flp-18, for the management of root knot nematode, Meloidogyne incognita, PLoS One, 8, e80603.
120. Shivakumara, T.N., Papolu, P.K., Dutta, T.K., Kamaraju, D., Chaudhary, S. and Rao, U. (2016) RNAiinduced silencing of an effector confers transcriptional oscillation in another group of effectors in the root-knot nematode, Meloidogyne incognita, Nematology, 18, 857-870.
121. Elad, Y. (1997) Responses of plants to infection by Botrytis cinerea and novel means involved in reducing their susceptibility to infection, Biol Rev, 72, 381-422.
122. Choquer, M., Fournier, E., Kunz, C., Levis, C., Pradier, J.-M., Simon, A. and Viaud, M. (2007) Botrytis cinerea virulence factors: new insights into a necrotrophic and polyphageous pathogen, FEMS Microbiol Lett, 277, 1-10.
123. van Kan, J.A.L. (2006) Licensed to kill: the lifestyle of a necrotrophic plant pathogen, Trends Plant Sci, 11, 247-253.
124. Williamson, B., Tudzynski, B., Tudzynski, P. and van Kan, J.A.L. (2007) Botrytis cinerea: the cause of grey mould disease, Mol Plant Pathol, 8, 561-580.
125. Nakajima, M. and Akutsu, K. (2014) Virulence factors of Botrytis cinerea, J Gen Plant Pathol, 80, 15-23.
126. Pinedo, C., Wang, C.-M., Pradier, J.-M., Dalmais, B., Choquer, M., Le Pêcheur, P., Morgant, G., Collado, I.G., Cane, D.E. and Viaud, M. (2008) Sesquiterpene synthase from the botrydial biosynthetic gene cluster of the phytopathogen Botrytis cinerea, ACS Chem Biol, 3, 791-801.
127. Dean, R., van Kan, J.A.L., Pretorius, Z.A., Hammond-Kosack, K.E., Di Pietro, A., Spanu, P.D., Rudd, J.J., Dickman, M., Kahmann, R., Ellis, J. and Foster, G.D. (2012) The Top 10 fungal pathogens in molecular plant pathology, Mol Plant Pathol, 13, 414-430.
128. Pedras, M.S.C., Hossain, S. and Snitynsky, R.B. (2011) Detoxification of cruciferous phytoalexins in Botrytis cinerea: Spontaneous dimerization of a camalexin metabolite, Phytochemistry, 72, 199-206.
129. Villa-Rojas, R., Sosa-Morales, M.E., López-Malo, A. and Tang, J. (2012) Thermal inactivation of Botrytis cinerea conidia in synthetic medium and strawberry puree, Int J Food Microbiol, 155, 269-272.
130. Malhat, F.M., Haggag, M.N., Loutfy, N.M., Osman, M.A.M. and Ahmed, M.T. (2015) Residues of organochlorine and synthetic pyrethroid pesticides in honey, an indicator of ambient environment, a pilot study, Chemosphere, 120, 457-461.
131. Oliveira, B.R., Penetra, A., Cardoso, V.V., Benoliel, M.J., Barreto Crespo, M.T., Samson, R.A. and Pereira, V.J. (2015) Biodegradation of pesticides using fungi species found in the aquatic environment, Environ Sci Pollut Res Int, 22, 11781-11791.
132. Tomenson, J.A. and Matthews, G.A. (2009) Causes and types of health effects during the use of crop protection chemicals: data from a survey of over 6,300 smallholder applicators in 24 different countries, Int Arch Occup Environ Health, 82, 935-949.
133. Leroux, P. (2007) Chemical Control of Botrytis and its Resistance to Chemical Fungicides, In: Elad, Y., Williamson, B., Tudzynski, P. and Delen, N. (eds.) Botrytis: Biology, Pathology and Control, Springer Netherlands, Dordrecht, 195-222.
134. Garfinkel, A.R. (2021) The history of Botrytis taxonomy, the rise of phylogenetics, and implications for species recognition, Phytopathology, 111, 437-454.
135. Fournier, E., Levis, C., Fortini, D., Leroux, P., Giraud, T. and Brygoo, Y. (2003) Characterization of Bc-hch, the Botrytis cinerea homolog of the Neurospora crassa het-c vegetative incompatibility locus, and its use as a population marker, Mycologia, 95, 251-261.
136. Fournier, E., Giraud, T., Albertini, C. and Brygoo, Y. (2005) Partition of the Botrytis cinerea complex in France using multiple gene genealogies, Mycologia, 97, 1251-1267**.**
137. Nair, N.G., Guilbaud-Oulton, S., Barchia, I. and Emmett, R. (1995) Significance of carry over inoculum, flower infection and latency on the incidence of Botrytis cinerea in berries of grapevines at harvest in New South Wales, AustJ Exp Agric, 35, 1177-1180.
138. ten Have, A., Mulder, W., Visser, J. and van Kan, J.A. (1998) The endopolygalacturonase gene Bcpg1 is required for full virulence of Botrytis cinerea, Mol Plant Microbe Interact, 11, 1009-1016.
139. Li, H., Tian, S. and Qin, G. (2019) NADPH oxidase is crucial for the cellular redox homeostasis in fungal pathogen Botrytis cinerea, Mol Plant Microbe Interact, 32, 1508-1516.
140. Liu, J.-K., Chang, H.-W., Liu, Y., Qin, Y.H., Ding, Y.-H., Wang, L., Zhao, Y., Zhang, M.-Z., Cao, S.-N., Li, L.-T., Liu, W., Li, G.-H. and Qin, Q.-M. (2018) The key gluconeogenic gene PCK1 is crucial for virulence of Botrytis cinerea via initiating its conidial germination and host penetration, Environ Microbiol, 20, 1794-1814.
141. Nerva, L., Sandrini, M., Gambino, G. and Chitarra, W. (2020) Double-stranded RNAs (dsRNAs) as a sustainable tool against gray mold (Botrytis cinerea) in grapevine: Effectiveness of different application methods in an open-air environment, Biomolecules, 10, 200.
142. Qiao, L., Lan, C., Capriotti, L., Ah-Fong, A., Nino Sanchez, J., Hamby, R., Heller, J., Zhao, H., Glass, N.L., Judelson, H.S., Mezzetti, B., Niu, D. and Jin, H. (2021) Spray-induced gene silencing for disease control is dependent on the efficiency of pathogen RNA uptake, Plant Biotechnol J, 19, 1756-1768.
143. Schumacher, J., Viaud, M., Simon, A. and Tudzynski, B. (2008) The Gα subunit BCG1, the phospholipase C (BcPLC1) and the calcineurin phosphatase co-ordinately regulate gene expression in the grey mould fungus Botrytis cinerea, Mol Microbiol, 67, 1027-1050.
144. Segmüller, N., Kokkelink, L., Giesbert, S., Odinius, D., van Kan, J. and Tudzynski, P. (2008) NADPH oxidases are involved in differentiation and pathogenicity in Botrytis cinerea, Mol Plant Microbe Interact, 21, 808-819.
145. Soulié, M.C., Perino, C., Piffeteau, A., Choquer, M., Malfatti, P., Cimerman, A., Kunz, C., Boccara, M. and Vidal-Cros, A. (2006) Botrytis cinerea virulence is drastically reduced after disruption of chitin synthase class III gene (Bcchs3a), Cell Microbiol, 8, 1310-1321.
146. Ren, W., Sang, C., Shi, D., Song, X., Zhou, M. and Chen, C. (2018) Ubiquitin-like activating enzymes BcAtg3 and BcAtg7 participate in development and pathogenesis of Botrytis cinerea, Curr Genet, 64, 919-930.
147. Yang, Q., Chen, Y. and Ma, Z. (2013) Involvement of BcVeA and BcVelB in regulating conidiation, pigmentation and virulence in Botrytis cinerea, Fungal Genet Biol, 50, 63-71.
148. Zheng, L., Campbell, M., Murphy, J., Lam, S. and Xu, J.R. (2000) The BMP1 gene is essential for pathogenicity in the gray mold fungus Botrytis cinerea, Mol Plant Microbe Interact, 13, 724-732.
149. Wang, M., Weiberg, A., Lin, F.-M., Thomma, B.P.H.J., Huang, H.-D. and Jin, H. (2016) Bidirectional cross-kingdom RNAi and fungal uptake of external RNAs confer plant protection, Nat Plants, 2, 16151.
150. Weiberg, A., Wang, M., Lin, F.-M., Zhao, H., Zhang, Z., Kaloshian, I., Huang, H.-D. and Jin, H. (2013) Fungal small RNAs suppress plant immunity by hijacking host RNA interference pathways, Science, 342, 118-123.
151. McLoughlin, A.G., Wytinck, N., Walker, P.L., Girard, I.J., Rashid, K.Y., de Kievit, T., Fernando, W.G.D., Whyard, S. and Belmonte, M.F. (2018) Identification and application of exogenous dsRNA confers plant protection against Sclerotinia sclerotiorum and Botrytis cinerea, Sci Rep, 8, 7320.
152. Gebremichael, D.E., Haile, Z.M., Negrini, F., Sabbadini, S., Capriotti, L., Mezzetti, B. and Baraldi, E. (2021) RNA interference strategies for future management of plant pathogenic fungi: prospects and challenges, Plants (Basel), 10, 650.
153. Gursinsky, T., Pirovano, W., Gambino, G., Friedrich, S., Behrens, S.-E. and Pantaleo, V. (2015) Homeologs of the Nicotiana benthamiana Antiviral ARGONAUTE1 Show Different Susceptibilities to microRNA168-Mediated Control, Plant Physiol, 168, 938-952.
154. Meyer M. and Masquida B. (2014) cis-acting 5' hammerhead ribozyme optimization for in vitro transcription of highly structured RNAs, Methods Mol Biol, 1086, 21-40.
155. Avis J.M., Conn G.L. and Walker S.C. (2012). Cis-acting ribozymes for the production of RNA in vitro transcripts with defined 5' and 3' ends, Methods Mol Biol, 941, 83-98.
156. Rizzo, T.M. and Palukaitis, P. (1990) Construction of full-length cDNA clones of cucumber mosaic virus RNAs 1, 2 and 3: Generation of infectious RNA transcripts, Mol Gen Genet, 222, 249-256.
157. Roossinck, M.J., Zhang, L. and Hellwald, K.H. (1999) Rearrangements in the 5' nontranslated region and phylogenetic analyses of cucumber mosaic virus RNA 3 indicate radial evolution of three subgroups, J Virol, 73, 6752-6758.
158. Jackson A.L. and Linsley, P.S. (2010) Recognizing and avoiding siRNA off-target effects for target identification and therapeutic application, Nat Rev Drug Discov, 9, 57-67.

## Patentansprüche

1. Nukleinsäure zum Schutz von Pflanzen vor Pflanzenpathogenen, **dadurch gekennzeichnet, dass**
a. die Nukleinsäure eine Einzelstrang-RNA ist und aus 21, 22, 23 oder 24 Nukleotiden besteht und eine Nukleinsäure enthält, die aus der Gruppe bestehend aus den Nukleinsäuren mit den SEQ ID NO: 1 bis 180 ausgewählt ist; oder
b. die Nukleinsäure eine Einzelstrang-RNA gemäß Gruppe a. ist und Veränderungen an 1 bis 10 Positionen der Nukleotidsequenz aufweist; oder
c. die Nukleinsäure eine *small* RNA (*s*RNA, wie z.B. eine *small interfering* RNA, siRNA, oder wie z.B. eine micro RNA, miRNA) ist, deren RNA-Doppelstrang aus komplett komplementären oder teilweise komplementären Nukleinsäuren der Gruppe a. und/oder Gruppe b. besteht; oder
d. die Nukleinsäure eine Doppelstrang-RNA ist, die Nukleotidsequenzen von mindestens zwei sRNAs der Gruppe c. enthält; oder
e. die Nukleinsäure eine Einzelstrang-DNA ist, die eine Sequenz von 12 oder mehr Nukleotiden enthält, die homolog ist (Deoxyribonukleotide statt Ribonukleotide) zu einer der Nukleotidsequenzen der Einzelstrang-RNAs der Gruppen a. oder b.

2. Nukleinsäure gemäß Anspruch 1, **dadurch gekennzeichnet, dass** das Pathogen Cucumber mosaic virus (CMV) ist und die Ribonukleotid- oder Deoxyribonukleotidsequenz von mindestens einer Nukleinsäure enthält, die gegen eine *target-*RNA des CMV gerichtet ist, wobei die *target-*RNA des CMV ausgewählt ist aus den *target*-RNAs mit den SEQ ID NO: 189 und 190.

3. Nukleinsäure gemäß Anspruch 1 oder 2, **dadurch gekennzeichnet, dass** das Pathogen Cucumber mosaic virus (CMV) ist und die Ribonukleotid- oder Deoxyribonukleotidsequenz von mindestens einer Nukleinsäure enthält, die aus der Gruppe bestehend aus den Nukleinsäuren mit den SEQ ID NO: 1 bis 92 ausgewählt ist.

4. Nukleinsäure gemäß Anspruch 1, **dadurch gekennzeichnet, dass** das Pathogen *Meloidogyne incognita* ist und die Ribonukleotid- oder Deoxyribonukleotidsequenz von mindestens einer Nukleinsäure enthält, die gegen eine *target-*RNA von *Meloidogyne incognita* gerichtet ist, wobei die *target-*RNA von *Meloidogyne incognita* ausgewählt ist aus den *target-*RNAs mit den SEQ ID NO: 191, 192 und 193.

5. Nukleinsäure gemäß Anspruch 1 oder 4, **dadurch gekennzeichnet, dass** das Pathogen *Meloidogyne incognita* ist und die Ribonukleotid- oder Deoxyribonukleotidsequenz von mindestens einer Nukleinsäure enthält, die aus der Gruppe bestehend aus den Nukleinsäuren mit den SEQ ID NO: 93 bis 120 ausgewählt ist.

6. Nukleinsäure gemäß Anspruch 1, **dadurch gekennzeichnet, dass** das Pathogen *Botrytis cinerea* ist und die Ribonukleotid- oder Deoxyribonukleotidsequenz von mindestens einer Nukleinsäure enthält, die gegen eine *target-*RNA von *Botrytis cinerea* gerichtet ist, wobei die *target-*RNA von *Botrytis cinerea* ausgewählt ist aus den *target*-RNAs mit den SEQ ID NO: 194, 195, 196, 197, 198 und 199.

7. Nukleinsäure gemäß Anspruch 1 oder 6, **dadurch gekennzeichnet, dass** das Pathogen *Botrytis cinerea* ist und die Ribonukleotid- oder Deoxyribonukleotidsequenz von mindestens einer Nukleinsäure enthält, die aus der Gruppe bestehend aus den Nukleinsäuren mit den SEQ ID NO: 121 bis 180 ausgewählt ist.

8. Nukleinsäure gemäß Anspruch 1, **dadurch gekennzeichnet, dass** die Nukleinsäure eine Doppelstrang-RNA ist und diese Doppelstrang-RNA Nukleotidsequenzen enthält, die aus pseudo-siRNA-Sequenzen und Sequenzen von mindestens zwei sRNAs gemäß Anspruch 1 c. bestehen.

9. Nukleinsäure gemäß Anspruch 8, **dadurch gekennzeichnet, dass** die Nukleinsäure stumpfe (blunt) oder überhängende Enden aufweisen.

10. Nukleinsäure gemäß Anspruch 8 oder 9 **dadurch gekennzeichnet, dass** die Nukleinsäure einen *spacer* enthält.

11. Nukleinsäure gemäß einem der Ansprüche 8 bis 10, **dadurch gekennzeichnet, dass** die pseudo-siRNA-Sequenzen und *spacer* in der Nukleinsäure Elemente enthalten, die ausgewählt sind aus Transkriptionspromotoren, Transkriptionsterminatoren, Transportsignalen, *Splice*-Stellen und Ribozymen.

12. Nukleinsäure gemäß einem der Ansprüche 8-11, **dadurch gekennzeichnet, dass** die Nukleinsäure ausgewählt ist aus der Gruppe bestehend aus den Nukleinsäuren mit der SEQ ID NO: 181, 182, 185, 186 sowie 200 bis 204.

13. Nukleinsäure nach einem der vorherigen Ansprüche, wobei die Nukleinsäure eine oder mehrere chemische Modifikationen aufweist, **dadurch gekennzeichnet, dass** die chemischen Modifikationen ausgewählt sind aus z.B. Konjugaten wie GalNac, Basenmodifikationen wie 5-methylcytosin, 2'-Zuckermodifikationen wie 2'-O-methyl, 2'-fluoro, 2'-O-methoxyethyl (2'-MOE), cETBNA ((S)-gebundene ethylbicyclisch), andere Zuckermodifikationen wie *"locked"* (LNA) oder *"unlocked"* (UNA), *"Backbone"*-Veränderungen wie Phosphorothioate (PS) oder *"peptide nucleic acids"* (PNA) sowie Zuckerphosphat-Modifikationen wie Morpholino/PMO (*phosphorodiamidate morpholino*)*.*

14. Zusammensetzung zur transgenen oder transienten Anwendung in der Schädlingsbekämpfung bei Pflanzen enthaltend eine oder mehrere Nukleinsäuren nach einem der Ansprüche 1 bis 13 und fakultativ ein oder mehrere Trägersubstanzen und/oder Hilfsstoffe.

15. Verwendung einer Nukleinsäure oder Zusammensetzung nach einem der Ansprüche 1 bis 14 bei Pflanzen zur Prophylaxe und/oder Behandlung gegen Befall und/oder Infektionen durch Pathogene.
